(19) Europäisches Patentamt
European Patent Office
Office européen des brevets

(11) **EP 3 910 066 A1**

(12) **EUROPEAN PATENT APPLICATION**
published in accordance with Art. 153(4) EPC

(43) Date of publication:
**17.11.2021 Bulletin 2021/46**

(51) Int Cl.:
*C12Q 1/68* *(2018.01)*    *C12N 15/00* *(2006.01)*

(21) Application number: **19887924.9**

(22) Date of filing: **20.11.2019**

(86) International application number:
**PCT/CN2019/119684**

(87) International publication number:
**WO 2020/103862 (28.05.2020 Gazette 2020/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.11.2018  CN 201811402300**

(71) Applicant: **GUANGZHOU IGENE BIOTECHNOLOGY CO., LTD.**
**Guangdong 510663 (CN)**

(72) Inventors:
• **YANG, Shuwei**
  **Guangdong 510663 (CN)**
• **HUANG, Liancheng**
  **Guangdong 510663 (CN)**
• **LIANG, Chen**
  **Guangdong 510663 (CN)**
• **CHEN, Yunyi**
  **Guangdong 510663 (CN)**
• **CHEN, Haiying**
  **Guangdong 510663 (CN)**

(74) Representative: **Nederlandsch Octrooibureau**
**P.O. Box 29720**
**2502 LS The Hague (NL)**

(54) **DNA REFERENCE STANDARD AND USE THEREOF**

(57)    The present invention discloses a reference DNA and the use thereof, wherein the reference DNA is selected from the group consisting of:
(i) DNA fragment 1: characterized in that it carries a defined gene mutation and at least one another artificially altered base X2, wherein, as compared to a wild type of the gene, at least one defined base X1 in the defined gene mutation undergoes a mutation associated with the occurrence, diagnosis, and/or treatment(such as a target targeted by a medicament) of a disease (such as a tumor), wherein the mutation is a substitution mutation, a deletion mutation, and/or an insertion mutation, and the artificially altered base X2 is different from the mutant base X1 which is contained in the DNA of a sample to be detected and defined to be associated with the occurrence, diagnosis and/or treatment of a disease,
(ii) DNA fragment 2: characterized in that it differs from the DNA fragment 1 only in that it does not comprise the defined base X1 mutation, or
(iii) a mixture of the DNA fragment 1 and the DNA fragment 2.

DNA FRAGMENTS WITH CHANGED BASES AT PARTICUALR POSITIONS AND USE

Fig. 38

**Description**

**FIELD OF THE INVENTION**

The present invention relates to the field of cell biology technology, in particular to a DNA fragment carrying a marker able to be spiked into a sample to be detected and a defined gene mutation as well as the use thereof.

**BACKGROUND OF THE INVENTION**

"Precision medicine" has become a globally popular subject. New technologies such as Next generation sequencing (NGS) and liquid biopsy are important components of methods and technologies for disease occurrence, diagnosis, treatment, classification and evaluation in the field of precision medicine. The selection and determination of targeted therapy for tumors is one of the most important challenges in biomedicine today. In view of this, NGS technologies and reagents for various uses emerge constantly.

Although NGS technology has been developed rapidly, becoming the most common research tool in the fields of drug discovery and translational medicine, and having been used for determining individual genome sequences and identifying genetic disease-associated mutations and tumor cell somatic mutations, it is still limited by various factors such as systematic errors and operational errors in multiple steps of PCR and sequencing library construction in terms of sensitivity and accuracy of mutation detection.

In terms of experimental operation, each step of target sequence enrichment and library preparation as well as a sequencing instrument all inevitably introduce a variation into the final sequencing data, such as the step for amplifying a target sequence by the DNA polymerase in the multiplex PCR processes of the library construction causes an uneven amplification, an inefficient Barcode linkage process, an occurrence of the highly reproducible sequence readouts, and PCR amplification biases.

In terms of instrument, the principles, performance, and parameters of the instruments used in PCR and NGS processes vary greatly depending on the experimental design. The same sample used by instruments from different manufacturers, or the same sample used by the same instrument, the same kit, but in different laboratories will have a larger difference in detection results.

In terms of errors between experimenters, different experimenters often lead to different results due to differences in their own experience and operating habits.

In terms of kit, there are no strict uniform quality standards for gene mutation detection kits produced by different manufacturers in the market. Therefore, there are also differences in the detection results using kits from different manufacturers or different batches of kits from the same manufacturer, and such detection results limit the application of the kit for clinical diagnosis and treatment.

In terms of laboratory environmental condition, laboratories that do not comply with operation procedures of national GMP standards and specifications will be inevitably subjected to interference from non-sample inclusions.

All of the above biases inevitably affect the accuracy, sensitivity and repeatability of detection results, as a result, the obtained data cannot accurately reflect the quality and quantity of the original sample DNA and RNA fragment sequence.

To address these problems, parallel experimental reference (standard) for NGS sequencing have been used to evaluate and correct sequencing errors from different instruments, different reagents, different operators and different laboratories, for evaluating the sensitivity and reproducibility of each instrument and kit to the detection of a mutation at each specific site.

However, these standards cannot be directly spiked into the sample to be detected, and cannot be used to evaluate and correct the number of molecule comprising mutations such as substitution, deletion, or insertion contained in the sample to be detected, let alone excluding an error resulted from a certain experimental step (such as library construction, Barcode linkage efficiency, or a lost of partial samples of a certain sample reaction system due to operational errors)or instrument inhomogeneity (such as abnormality in a certain well in a 96-well PCR instrument) for detecting of a large number of samples.

In view of this, Ira W. Deveson et al. (Nature Methods, 2016, 13 (9): 784-791) proposed the use of a series of synthetic sequencing spike-in standards (abbreviated as sequins). Sequins are DNA molecules with a length not more than 10 kb prepared by using E. coli, carrying the true genetic characteristics of interest to a researcher, such as containing genetic mutation sites, while also containing a sequence not homologous to the native genomic sequence to be detected. The Sequins standard, like the sample DNA, undergoes each step of the sequencing process and the same reactions. However, 1) this artificially designed standard contains the "recognition" sequence not homologous to the sequence of the sample to be detected, which is greatly different from the sequence of the sample to be detected; 2) they are inconsistent in the efficiencies for obtaining the target DNA sequences from the spiked-in standard and from the sample to be detected, obtained by the method of capturing the target sequence with probe hybridization or directional amplification of the target sequence with PCR amplification method; 3) the DNA spiked-in standards prepared by E.coli and the DNA extracted from human tissues and blood, contain different contents of contaminating inhibitors for DNA polymerase enzymatic reactions; 4) there is great difference in the degrees of modifications (such as methylations) of bases in DNA standards prepared by E. coli and the DNA of the sample to be detected. These differences will lead to inconsistencies in the amplification efficiency of the corresponding DNA fragments in the standard and the sample to be detected during the library preparation process, so that the quantitative change of the mutant fragments in the sample to be detected cannot be accurately evaluated and corrected. As a result, the sequins standard can only be used for whole genome sequencing, which is adequate for neither a targeted sequencing which currently has a large market and clinical needs, nor the needs of the sequencing of long fragments more than 10 kb using sequencing methods such as PacBio and Nanopore.

## SUMMARY OF THE INVENTION

The object of the present invention is to provide a reference DNA and a preparation method thereof, which can play quantitative and qualitative roles in a further detection such as NGS and PCR amplification, thereby improving the accuracy of PCR amplification, NGS and subsequent data analysis.

Specifically, the present disclosure relates to the following content:

1. A reference DNA, selected from the group consisting of:

(i) DNA fragment 1: characterized in that it carries a defined gene mutation and at least one another artificially altered base X2, wherein, as compared to a wild type of the gene, at least one defined base X1 in the defined gene mutation undergoes a mutation associated with the occurrence, diagnosis, and/or treatment (such as a target targeted by a medicament) of a disease (such as a tumor), wherein the mutation is a substitution mutation, a deletion mutation, and/or an insertion mutation, and the artificial altered base X2 is different from the mutant base X1 which is contained in the sample to be detected and defined to be associated with the occurrence, diagnosis and/or treatment of a disease,

(ii) DNA fragment 2: characterized in that the DNA fragment 2 comprises the artificial altered base X2 in (i), and it differs from the DNA fragment 1 only in that it does not comprise the defined base X1 mutation, or

(iii) a mixture of the DNA fragment 1 and the DNA fragment 2.

2. The reference DNA according to item 1, wherein the base X2 is located at any position upstream, downstream or both of the defined base X1.

3. The reference DNA according to item 1, wherein the DNA fragment 1 and the DNA fragment 2 are double stranded DNAs.

4. The reference DNA according to item 1, characterized in that when the mutation in (i) is a substitution mutation, the interval between the defined base X1 and the base X2 is 1 bp, 2 bp, 3 bp, 4 bp, 5 bp, 10 bp or less, 100 bp or less, 500 bp or less, 1 kb or less, 2 kb or less, 10 kb or less, or 100 kb or less, preferably,

(a) when the position of the third base in the codon comprising the defined base X1 mutation is set as 0, and the base X2 is located upstream of the defined base X1, the position of the base X2 is represented by 3n, wherein the n is a positive integer, preferably, the altering of the base X2 does not cause any change to the original amino acid coded, or

(b) when the position of the third base in the codon comprising the defined base X1 mutation is set as 0, and the base X2 is located downstream of the defined base X1, the position of the base X2 is represented by -3n, wherein the n is a positive integer, preferably, the altering of the base X2 does not cause any change to the original amino acid coded; or

(c) when the position of the third base in the codon comprising the defined base X1 mutation is set as 0, and the base X2 is located upstream and downstream of the defined base X1, respectively, the position of the base X2 located upstream of the defined base X1 is represented by 3n, and the position of the base X2 located downstream of the defined base X1 is represented by -3n, wherein both of the n is a positive integer, preferably, the altering of the base X2 does not cause any change to the original amino acid coded.

5. The reference DNA according to item 4, characterized in that the mutation in (i) is a consecutive substitution or a discrete substitution, preferably substitution mutations in the first and the second consecutive bases of the same codon.

6. The reference DNA according to item 1, characterized in that when the mutation in (i) is a deletion mutation, the interval between the defined base X1 and the base X2 is 1 bp, 2 bp, 3 bp, 4 bp, 5 bp, 10 bp or less, 100 bp or less, 500 bp or less, 1 kb or less, 2 kb or less, 10 kb or less, or 100 kb or less,
preferably,

(a) when as compared to a wild type of the gene, one of the defined base X1 is deleted at one base position, or multiple defined base X1s are deleted consecutively at multiple base positions, and the base X2 is located upstream of the deleted defined base X1, the position of the third base of a codon immediately adjacent to the upstream of the defined base X1 and corresponding to the first codon of the wide type of the gene is set as 0, the position of the base X2 is represented by 3n, wherein the n is a positive integer, preferably, the altering of the base X2 does not cause any change to the original amino acid coded, or

(b) when as compared to a wild type of the gene, one of the defined base X1 is deleted at one base position, or multiple defined base X1s are deleted consecutively at multiple base positions, and the base X2 is located downstream of the defined base X1, the base X2 is located at any position downstream of the defined base X1;

(c) when as compared to a wild type of the gene, one of the defined base X1 is deleted at one base position, or multiple defined base X1s are deleted consecutively at multiple base positions, and the base X2s are located upstream and downstream of the deleted defined base X1, when the base X2 is located upstream of the base X1, the definition of the base X2 is described in (a), and when the base X2 is located downstream of the base X1, the definition of the base X2 is described in (b), preferably, the altering of the base X2 does not cause any change to the original amino acid coded.

7. The reference DNA of item 6, characterized in that in the conditions of (a)-(c), the deletion is a consecutive deletion or a discrete deletion.

8. The reference DNA according to item 1, characterized in that when the mutation in (i) is an insertion mutation, the interval between the defined base X1 and the base X2 is 1 bp, 2 bp, 3 bp, 4 bp, 5 bp, 10 bp or less, 100 bp or less, 500 bp or less, 1 kb or less, 2 kb or less, 10 kb or less, or 100 kb or less,
preferably,

(a) when as compared to a wild type of the gene, one of the defined base X1 is inserted between two bases, or multiple defined base X1s are consecutively inserted between two bases, and the base X2 is located upstream of the inserted defined base X1, the position of the third base of a codon immediately adjacent to the upstream of the defined base X1 and corresponding to the first codon of the wide type of the gene is set as 0, the position of the base X2 is represented by 3n, wherein the n is a positive integer, preferably, the altering of the base X2 does not cause any change to the original amino acid coded,

(b) when as compared to a wild type of the gene, one of the defined base X1 is inserted between two bases, or multiple defined base X1s are consecutively inserted between two bases, and the base X2 is located downstream of the defined base X1, the base X2 is located at any position downstream of the defined base X1; or

(c) when as compared to a wild type of the gene, one of the defined base X1 is inserted between two bases, or multiple defined base X1s are consecutively inserted between two bases, and the base X2s are located upstream and downstream of the inserted defined base X1, respectively, when the base X2 is located upstream of the base X1, the definition of the base X2 is described in (a), and when the base X2 is located downstream of the base X1, the definition of the base X2 is described in (b), preferably, the altering of the base X2 does not cause any change to the original amino acid coded.

9. The reference DNA of item 8, characterized in that in the conditions of (a)-(c), the insertion is a consecutive insertion or a discrete insertion.

10. The reference DNA according to item 5, characterized in that, the substitution mutation in (i) is m discrete substitution mutations, wherein the m is a integer of 2 or more, and when the distance between each two mutations is 10 bp, 10-20 bp, 10-30 bp, 10-40 bp, 10-50 bp, 10-60 bp, 10-70 bp or 10-80 bp, the artificial altered base X2 in (ii) is formed simultaneously upstream and downstream of the base X1.

11. The reference DNA according to item 7, characterized in that, the deletion mutation in (i) is m discrete deletion mutations, wherein the m is a integer of 2 or more, and when the distance between each two mutations is 10 bp, 10-20 bp, 10-30 bp, 10-40 bp, 10-50 bp, 10-60 bp, 10-70 bp or 10-80 bp, the artificial altered base X2 in (ii) is formed simultaneously upstream and downstream of the base X1.

12. The reference DNA according to item 9, characterized in that, the insertion mutation in (i) is m discrete insertion mutations, wherein the m is an integer of 2 or more, and when the distance between each two mutations is 10 bp, 10-20 bp, 10-30 bp, 10-40 bp, 10-50 bp, 10-60 bp, 10-70 bp or 10-80 bp, the artificial altered base X2 in (ii) is simultaneously formed upstream and downstream of the base X1.

13. Reference DNA according to item 1, characterized in that the gene comprising a defined mutant base X1 associated with the occurrence, diagnosis, and/or treatment (such as a target targeted by a medicament) of a disease (such as a tumor) includes, but not limited to, EGFR, KRAS, BRAF, P53, Met, PTEN, ROS1, NRAS, PIK3CA, RET, HER2, CMET, FGFR1 and/or DDR2.

14. Reference DNA according to item 13, characterized in that the position of the amino acid encoded by the codon comprising the defined base X1 mutation includes, but not limited to, amino acid position 858, 790, 768, 746, 747, 748, 749, 750, 719 and/or 797 of EGFR, amino acid position 12 and/or 13 of KRAS, amino acid position 12, 13 and/or 600 of BRAF, amino acid position 12, 59 and/or 61 of NRAS, amino acid position 880 and/or 837 of HER2, amino acid position 816 of cKIT, and amino acid position 545 and/or 1047 of PIK3CA, wherein the position is calculated by taking the position of the amino acid encoded by the start codon as 1.

15. Reference DNA according to item 14, characterized in that there are deletion mutations in EGFR amino acid positions 746, 747, 748, 749, 750; a mutation of substituting arginine R for leucine L at amino acid position 858 of EGFR; a mutation of substituting serine S for cysteine C at amino acid position 797 of EGFR; a mutation of substituting serine S for glycine G at amino acid position 719 of EGFR; a mutation of substituting methionine M for threonine T at amino acid position 790 of EGFR; a mutation of substituting isoleucine I for serine S at amino acid position 768 of EGFR; a mutation of substituting glutamic acid E for valine V at amino acid position 600 of BRAF; a mutation of substituting cysteine C for glycine G at amino acid position 12 of BRAF; a mutation of substituting cysteine C for glycine G at amino acid position 13 of BARF; a mutation of substituting aspartic acid D for glycine G at amino acid position 13 of KRAS; a mutation of substituting aspartic acid D for glycine G at amino acid position 12 of KRAS; a mutation of substituting alanine A for glycine G at amino acid position 12 of KRAS; a mutation of substituting valine V for glycine G at amino acid position 12 of KRAS; a mutation of substituting serine S for glycine G at amino acid position 12 of KRAS; a mutation of substituting arginine R for glutamine Q at amino acid position 61 of NRAS; a mutation of substituting lysine K for glutamine Q at amino acid position 61 of NRAS; a mutation of substituting aspartic acid D for glycine G at amino acid position 12 of NRAS; a mutation of substituting threonine T for alanine A at the amino acid position 59 of NRAS; a mutation of substituting lysine K for alanine A at the amino acid position 59 of NRAS; a mutation of substituting asparagine N for aspartic acid D at amino acid position 880 of HER2; a mutation of substituting tyrosine Y for glutamic acid E at amino acid position 837 of HER2; a mutation of substituting valine V for aspartic acid D at amino acid position 816 of KIT; a mutation of substituting arginine R for histidine H at amino acid position 1047 of PIK3CA; a mutation of substituting lysine K for glutamic acid E at amino acid position 545 of PIK3CA.

16. The reference DNA according to any one of items 1-15, characterized in that the reference DNA is synthesized by chemical methods.

17. The reference DNA according to any one of items 1-16, which is used as a reference standard DNA.

18. A reference cell, characterized in that it contains the reference DNA of any one of items 1-17.

19. The reference cell according to item 18, characterized in that the gene contained in the reference DNA exists in homozygous or heterozygous state, preferably the cell is a prokaryotic cell or an eukaryotic cell.

20. The reference cell according to item 18, characterized in that the cell is derived from a mammal.

21. The reference cell according to item 18, characterized in that the cell is derived from a human.

22. The reference cell according to item 18, characterized in that the cell is derived from a tumor tissue cell.

23. The reference cell according to item 18, characterized in that the cell is constructed (engineered) by a method including, but not limited to, gene editing technology, such as the CRISPER-Cas9, TALEN or ZFN, preferably, the cell is used as a reference standard cell.

24. A vector, characterized in that it comprises the reference DNA of any one of items 1-17, preferably, the vector is a plasmid vector or a viral vector, preferably a prokaryotic cell vector or an eukaryotic cell vector, more preferably, the prokaryotic vector includes, but is not limited to, a pUC19 plasmid, and the eukaryotic viral vector includes, but is not limited to, an adenovirus (AV), an adeno-associated virus (AAV).

25. A host cell, characterized in that it comprises the vector of item 24, preferably the host cell is a prokaryotic cell or a eukaryotic cell, more preferably an *E. coli* cell, or a yeast cell.

26. A method of detecting whether the sample of a subject carries a defined gene mutation (preferably the method is a whole genome sequencing method or a next-generation sequencing method, more preferably a targeted sequencing of a next-generation sequencing method), characterized in that one or more (preferably 1-1000, 10-900, 100-800, 200-700, 300-600 or 400-500) of the reference DNA according to any one of items 1-17, the reference cell according to any one of items 18 to 23, the vector according to item 24 or the host cell according to item 25 are spiked into the sample to be detected.

27. The method according to item 26, characterized in that the sample to be detected is from the subject, including, but not limited to, a cell derived from blood, saliva, urine, tissue, cerebrospinal fluid, or alveolar lavage fluid, or an DNA extract from the above sample(s).

28. The method according to item 26 or 27, characterized in that, the cell contained in the sample of the subject includes, but not limited to, a tissue cell and/or a circulating tumor cell derived from a colon cancer patient, preferably, the cell comprises a protein encoded by a gene having the codon with the defined base X1 mutation, and the position of the amino acid encoded by the codon in the protein includes, but not limited to, amino acid positions 12 and/or 13 of KRAS, amino acid positions 12, 13 and/or 600 of BRAF, amino acid positions 12, 59 and/or 61 of NRAS, and/or amino acid positions 545 and/or 1047 of PIK3CA, wherein the amino acid positions are calculated taking the amino acid encoded by the start codon of the wild type of the gene as 1.

29. The method according to item 26 or 27, characterized in that, the cell contained in the sample of the subject includes, but is not limited to, a tissue cell and/or a circulating tumor cell derived from a lung cancer patient, the protein encoded by the gene comprising the codon having the defined base X1 mutation in the reference DNA fragment comprised in the reference cell and the position of the amino acid encoded by the codon in the protein includes, but not limited to, amino acid positions 858, 790, 768, 746, 747, 748, 749, 750, 719 and/or 797 of EGRF, amino acid position 12 and/or 13 of KRAS, amino acid positions 12, 13 and/or 600 of BRAF, wherein the amino acid positions are calculated taking the amino acid encoded by the start codon of the wild type of the gene as 1.

30. The method according to item 26 or 27, characterized in that, the cell contained in the sample of the subject includes, but is not limited to, a tissue cell and/or a circulating tumor cell derived from a breast cancer patient, the protein encoded by the gene comprising the codon having the defined base X1 mutation in the reference DNA fragment comprised in the reference cell and the position of the amino acid encoded by the codon in the protein includes, but not limited to, amino acid positions 858, 790, 797, 719 and/or 768 of EGRF, amino acid position 12 and/or 13 of KRAS, amino acid positions 12, 13 and/or 600 of BRAF, and/or amino acid positions 880 and/or 837 of HER2, wherein the amino acid positions are calculated taking the amino acid encoded by the start codon of the wild type of the gene as 1.

31. The method according to item 26, characterized in that the reference DNA is from the genomic DNA in the reference cell of item 18.

32. The method according to item 31, characterized in that, the DNA of the sample to be detected includes, but not limited to, a DNA from a tissue or a cell derived from a colon cancer patient, wherein a protein is encoded by the gene comprising the codon having the defined base X1 mutation in the reference DNA and the position of the amino acid encoded by the codon in the protein includes, but not limited to, amino acid positions 12 and/or 13 of KRAS, amino acid positions 12, 13 and/or 600 of BRAF, amino acid positions 12, 59 and/or 61 of NRAS, and/or amino acid positions 545 and/or 1047 of PIK3CA, wherein the amino acid positions are calculated by taking the position of the amino acid encoded by the start codon of the wild type of the gene as 1.

33. The method according to item 31, characterized in that, the DNA of the sample to be detected includes, but not limited to, a DNA from a tissue or a cell derived from a lung cancer patient, wherein a protein is encoded by the gene comprising the codon having the defined base X1 mutation in the reference DNA and the position of the amino acid encoded by the codon in the protein includes, but not limited to, amino acid positions 858, 790, 768, 746, 747, 748, 749, 750, 719 and/or 797 of EGRF, amino acid position 12 and/or 13 of KRAS, and/or amino acid positions 12, 13 and/or 600 of BRAF, wherein the amino acid positions are calculated by taking the position of the amino acid encoded by the start codon of the wild type of the gene as 1.

34. The method according to item 31, characterized in that, the DNA of the sample to be detected includes, but not limited to, a DNA from a tissue or a cell derived from a breast cancer patient, wherein a protein is encoded by the gene comprising the codon having the defined base X1 mutation in the reference DNA and the position of the amino acid encoded by the codon in the protein includes, but not limited to, amino acid positions 858, 790, 797, 719 and/or 768 of EGRF, amino acid position 12 and/or 13 of KRAS, amino acid positions 12, 13 and/or 600 of BRAF, and/or amino acid positions 880 and/or 837 of HER2, wherein the amino acid positions are calculated by taking the position of the amino acid encoded by the wild type of the start codon of the gene as 1.

35. The method according to item 26, characterized in that the DNA of the sample to be detected is fragmented, and the DNA of the sample to be detected is circulating cell-free DNA in cells, tissues, saliva and blood, and the spiked-in reference DNA has a length of 20 bp to 500 bp, wherein about 60-90% of the reference DNAs are 140-170 bp in length.

36. The method according to item 35, wherein when the reference DNA is a mixture of the DNA fragment 1 and the DNA fragment 2, the content percentage of the DNA fragment 1 and the DNA fragment 2 is 0.01% to 99.9%; preferably 10%, 25% or 50%; more preferably 1.0%, 2.5% or 5%; further preferably 0.01%, 0.025% or 0.05%.

37. A kit, characterized in that the kit comprises one or more (preferably 1-1000, 10-900, 100-800, 200-700, 300-600, 400-500) of the reference DNAs of any one of items 1-17, preferably the number of the reference DNAs is from 1 to $10^9$.

38. The kit according to item 37, characterized in that the DNA fragment 1 is or is not mixed with the DNA fragment 2.

39. The kit according to item 38, wherein when the DNA fragment 1 is mixed with the DNA fragment 2, the content percentage of the DNA fragment 1 and the DNA fragment 2 is 0.01% to 99.9%; preferably 10%, 25% or 50%; more preferably 1.0%, 2.5% or 5%; further preferably 0.01%, 0.025% or 0.05%.

40. A kit, characterized in that the kit comprises one or more (preferably 1-1000, 10-900, 100-800, 200-700, 300-600, 400-500) of the reference cells of any one of items 18-23, preferably the number of the reference cells is from 1 to $10^9$.

41. The kit according to item 40, characterized in that the DNA fragment 1 and the DNA fragment 2 are present in different cells or in the same cell, alternatively, when the DNA fragment 1 and the DNA fragment 2 are present in different cells, the different cells are present in a mixed form or in a separated form.

42. The kit according to item 41, characterized in that when the DNA fragment 1 and the DNA fragment 2 are present in different cells, the content percentage of a cell containing the DNA fragment 1 and a cell containing the DNA fragment 2 is 0.01% to 99.9%; preferably 10%, 25% or 50%; more preferably 1.0%, 2.5% or 5%; further preferably 0.01%, 0.025% or 0.05%.

43. A method of ensuring sensitivity and accuracy of detection of a gene mutation associated with the occurrence, diagnosis, and/or treatment (such as a target targeted by a medicament) of a disease (such as a tumor), characterized in that using the reference DNA according to any one of items 1-17 or the reference cell according to any one of items 18-23 as a reference standard for parallel experiments of the sequencing process of the sample to be detected and a reference standard to be spiked into the sample to be detected.

44. Use of the reference DNA according to any one of items 1-17, or the reference cell of any one of items 18-23 in the manufacture of a reagent for detecting whether a defined gene mutation is present in a sample of a subject, preferably for quality analysis and/or quality control, preferably, the defined gene mutation is associated with the occurrence, diagnosis, and/or treatment (such as a target targeted by a medicament) of a disease (such as a tumor).

45. Use of the reference DNA according to any one of items 1-17 or the reference cell according to any one of items 18-23 as a reference standard for parallel experiments of the sequencing process of the sample to be detected and a reference standard to be spiked into the sample to be detected.

The beneficial effects achieved by the invention are as follows: the reference DNA constructed in the invention can be directly added to the sample to be detected, and plays quantitative and qualitative roles inside the sample, thereby providing reliable assurance for the quality controlling and analyzing of each part of the PCR amplification and the NGS experiments to ensure the accuracy of the data. For example, the reference DNA of the present invention can be used for a parallel experiment, or can be spiked into a clinical sample, thereby calculating the DNA molecule number of the mutation sites to be detected in the sample to be detected, and accurately calculating the number of cells carrying genetic variation in the sample to be detected (a certain weight of tissue or a certain volume of blood) and providing reliable method for the quality controlling and analyzing of each part of the experiments to ensure the accuracy of the data.

**DEFINITIONS**

In order to facilitate understanding of the invention, explanations for the terms are given below:
As used herein, the term "defined gene mutation" refers to a gene mutation which is selected for a particular need, with its gene having a structural change in the base composition or base sequence, for example, a gene mutation associated with the occurrence, diagnosis, and/or treatment (such as a target targeted by a medicament) of a disease (such as a tumor), including but not limited to the mutations in Table 1:

Table 1. Common mutation sites in tumor cell DNA sequences

| (The following table involves 31 genes, with a total of 79 mutation sites, wherein the confirmation of the amino acid positions is calculated by taking the position of the amino acid encoded by the start codon ATG as 1) | |
| --- | --- |
| **Gene** | **Mutation Site** |
| ABL1 | T315I |
| AKT1 | E17K |
| ALK | F1174L |
| BRAF | G469A |
| | D594G |
| | V600E |
| | V600G |
| | V600K |
| | V600M |
| | V600R |

(continued)

| (The following table involves 31 genes, with a total of 79 mutation sites, wherein the confirmation of the amino acid positions is calculated by taking the position of the amino acid encoded by the start codon ATG as 1) | |
|---|---|
| Gene | Mutation Site |
| BRCA1 | E515** |
|  | D1739V |
| BRCA2 | Q2354* |
|  | G2837V |
| cKIT | D816V |
| DDR2 | S768R |
| EGFR | dE746-A750 |
|  | L858R |
|  | L858M |
|  | L861Q |
|  | T790M |
|  | C797S |
|  | T790M & C797S |
|  | G719S |
|  | G719A |
|  | L861Q |
|  | V769 - D770insASV |
|  | S768I |
|  | S492R |
| EML4-ALK | EML4-ALK translocation variant 1 |
| FGFR1 | P150L |
| FGFR2 | S252W |
| FLT3 | D835Y |
|  | ΔI836 |
| GNA11 | Q209L |
| GNAQ | Q209L |
| GNAS | R201C |
| HER2 | D880N |
|  | E837Y |
| IDH1 | R132C |
|  | R132H |
| IDH2 | R140Q |
|  | R172K |
| JAK2 | V617F |
| cKIT | D816V |

(continued)

| (The following table involves 31 genes, with a total of 79 mutation sites, wherein the confirmation of the amino acid positions is calculated by taking the position of the amino acid encoded by the start codon ATG as 1) | |
| --- | --- |
| Gene | Mutation Site |
| KRAS | A146T |
| | A59T |
| | G12A |
| | G12C |
| | G13C |
| | G12D |
| | G13D |
| | G12A |
| | G12R |
| | G12S |
| | G12V |
| | Q61H |
| | Q61L |
| MEK1 | P124L |
| MET | Y1253D |
| NOTCH | L1601P |
| NOTCH1 | L1600P |
| NRAS | A59T |
| | A146T |
| | G12D |
| | G13D |
| | G12V |
| | K117N |
| | Q61H |
| | Q61K |
| | Q61L |
| | Q61R |
| | D842V |
| PDGFRA | G426D |
| PI3KCA | H1047R |
| | E542K |
| | E545K |
| ROS1 | SLC34A2/ROS1 fusion |
| RET | CCDC6/RET fusion |

The term "marker able to be spiked into a sample to be detected" refers to a unique sequence code that is attached to a DNA molecule to be detected and can be used to qualify and quantify a sample to be detected after being added to the sample. In the present disclosure, a unique spiked-in marker is formed by constructing at least one base mutation (e.g., substitution, wherein the mutation keeps its activity unchanged) upstream and/or downstream of a defined gene mutation site of the DNA sequence, i.e., base X2.

The term "reference DNA" refers to a DNA having at least one defined base X1 which undergoes a mutation associated with the occurrence, diagnosis, and/or treatment (such as a target targeted by a medicament) of a disease (such as a tumor), and having at least another artificially altered base X2, or, also refers to a DNA comprising not the defined base X1, but the base X2. The reference DNA can be used to prepare a standard, reference DNA fragment. As used herein, the term "reference DNA" may refer to a single DNA fragment, as well as a mixture of DNA fragments.

The term "DNA fragment" can be a fragment in a length from 16 base pairs to 1000 base pairs, or it can be a chromosome.

The term "plasmid" is a closed circular double-stranded DNA molecule other than a chromosome (or pseudonucleus) in organisms such as bacteria, yeasts and actinomycetes, present in the cytoplasm, having an autonomously replication ability, allowing it to maintain a constant copy number in descendant cells, and expressing its carried genetic information.

The term "Next-generation sequencing (NGS)" refers to the method of determining DNA base sequence with an instrument (such as Illumina, PacBio and Nanopore) which has been currently used on the market. NGS refers to a large number of sequencing technologies based on High-throughput short-reading long-sequence production as well as large-scale sequence splicing and alignment analysis, emerging at the beginning of this century. As compared to the first-generation sequencing technology (Pyrosequencing represented by the Sanger method), NGS sequencing technology has advantages of high throughput, low cost, and high accuracy, but also has limitations such as huge initial investment and high barriers to entry.

The term "wild type of a gene or a wild-type DNA" refers to an allele in the most locus of its natural population, referred to as a wild-type gene. Its opposite is a mutant-type gene.

The term "standard" refers to one or more uniform enough substances with their biometric property (quantity) values (such as content, sequence, activity, structure, or typing) well determined, for calibrating instruments, evaluating biometric methods, or assigning a value to a material.

The term "a reference standard" refers to a substance that can be used as a reference standard for the determination of tumor mutant molecules in clinical samples; wherein "a reference standard DNA" refers to DNA used as a reference standard, sometimes referred to herein as "a standard DNA" or "a DNA standard"; "a reference standard cell" refers to a cell used as a reference standard, sometimes referred to herein as "a standard cell" or "a cell standard", and is from the cell line used as a reference standard.

The term "immediately adjacent to" means that there is no base inbetween.

The term "normal cell line" or "wild-type cell line", as a term relative to a pathological cell line (for example a tumor cell line), refers to the cell population that was propagated after the first successful passage of the original conventional cell culture, and also refers to conventionally cultured cell that can be serially passaged for a long period of time.

The term "CRISPER-Cas9" is an adaptive immune defense mechanism formed by bacteria and archaea during long-term evolution, and is able to be used against invading viruses and exogenous DNA. The CRISPR-Cas9 gene editing technology is a technique for specific DNA modification in a target gene, and this technology is a frontier method used in gene editing, currently. A CRISPR-Cas9-based gene editing technology has shown a great application prospect in a series of gene therapy application fields, for example blood diseases, tumors and other genetic diseases.

The term "TALEN (transcription activator-like (TAL) effector nuclease, abbreviated as TAL effector nuclease)" is an enzyme that can targeted modify a specific DNA sequence, and can recognize a specific DNA base pair with the help of TAL effector (a natural protein secreted by a plant bacteria). TAL effector can be designed to recognize and bind to all DNA sequences of interest. A TALEN is generated by adding a nuclease to the TAL effector. TAL effector nuclease can bind to DNA and cleave the DNA strand at a specific site, thereby introducing a new genetic material. Since TALEN has some superior characteristics over ZFN, it is now an important tool for researchers to study gene function and for potential gene therapy applications.

The term "Zinc-finger nuclease (ZFN)" consists of two different domains: a zinc finger domain, being able to recognize a DNA sequence and bind to it; an endonuclease *Fok* I cleavage domain, being able to cleave DNA. ZFN technology can significantly improve the efficiency of genome editing and is successfully applied to a variety of organisms.

The term "Precision Medicine" refers to an emerging approach for preventing and treating diseases taking into account differences in personal genes, environment and lifestyle habits. It is also a novel medical concept and medical model based on individualized medicine and developed with a rapid advancement of the genome sequencing technology as well as the cross-application of bioinformatics and big data science.

The term "Liquid Biopsy", as a branch of in vitro diagnostics, refers to a non-invasive blood assay that can monitor a circulating tumor cell (CTC) and a circulating tumor DNA (ctDNA) fragment released into the blood by tumors or metastases, and is regarded as a breakthrough technology for detecting tumor and cancer and for adjuvant therapy.

The term "PCR (Polymerase Chain Reaction)" refers to a molecular biology technique for amplifying a specific DNA fragment, which can be regarded as a special DNA replication in vitro. The basic feature of PCR is the ability to dramatically increase trace amounts of DNA.

The term "cell-free DNA (cfDNA)" refers to a free, extracellular, partially degraded endogenous DNA in circulating blood. Most of the cell-free DNAs are double-stranded DNA molecules, and the cell-free DNA fragments in the blood are much smaller than the genomic DNA, with a length concentrated between 0.18-21 kb.

**BRIEF DESCRIPTION OF THE DRAWINGS**

Figure 1 shows construction a vector backbone of a donor clone that carries a marker able to be spiked into a sample to be detected and an EGFR L858R mutation;

Figure 2 shows the electropherogram of PCR results. Lane M: Marker 6000; Lane 1: PCR product L (787 bp); Lane 2: PCR product R1 (255 bp); Lane 3: PCR product R2 (717 bp).

Figure 3 shows the plasmid digestion photograph. Lane M: DNA Ladder 100, 6000, 15000; Lane1: DC-HTN001161-D04 plasmid; Lane2: two expected bands~1703/5537bp obtained by cleaving the DC-HTN001161-D04 plasmid with Af1III; Lane3: two expected bands of -3277/3963 bp obtained by cleaving the DC-HTN001161-D04 plasmid with SapI, wherein the M1 lane indicates DNA Ladder 6000, the M2 lane indicates DNA Ladder 100, and the M3 lane indicates DNA Ladder 15000.

Figure 4 shows the left arm blast result of DC-HTN001161-D04-L and G80608.

Figure 5 shows the right arm blast result of DC-HTN001161-D04-R and G80789.

Figure 6 shows the construction of the vector backbone of the sgRNA clone.

Figure 7 shows the plasmid digestion photograph. Lane M: DNA Ladder 100, 6000, 15000; Lane1: HCP001161-CG08-3-10-a plasmid; Lane2: two expected bands ~2098/7505bp obtained by cleaving the HCP001161-CG08-3-10-a plasmid with PvuI; Lane3: two expected bands ~4069/5534bp obtained by cleaving the HCP001161-CG08-3-10-a plasmid with EcoRI, wherein M1 lane indicates DNA Ladder 6000, M2 lane indicates DNA Ladder 100, and M3 lane indicates DNA Ladder 15000.

Figure 8 shows the analysis of sequencing results of sgRNA clones.

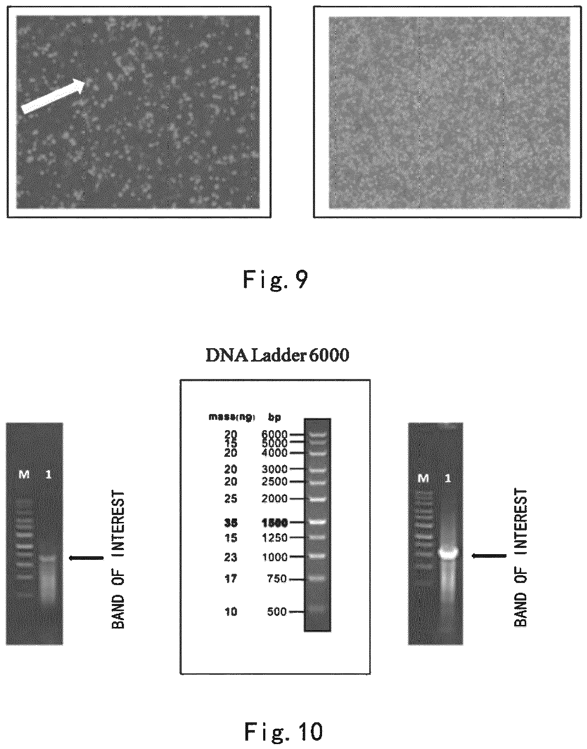

Figure 9 shows the transfection efficiency of HCT 116 cells (48h). The left panel shows the efficiency represented by red fluorescence which was observed by fluorescence microscopy after the transfection of HCT 116 cells with a donor and sgRNA plasmids for 48 h (using green light as the excitation source), wherein the arrow indicates red fluorescence-labeled cells. The sgRNA plasmid carries a mcherry red fluorescent label, so the effective transfection efficiency of the cells can be judged by observing the ratio of the red fluorescent cells. The right panel shows the total cell density observed in the bright field of the microscope after transfection of HCT 116 cells with donor and sgRNA plasmids for 48 h.

Figure 10 shows the electropherogram of junction PCR. The left panel indicates a PCR electropherogram for the 5'-end junction, and the right panel indicates a PCR electropherogram for the 3'-end junction, wherein the M lane indicates DNA Ladder 6000.

Figure 11 shows the sequencing result of the PCR product for confirming the positive cell strain sequence. The box in the figure indicates the sequencing peak map--L at position 858 of exon 21 of EGFR (wherein exon 21 is shown by the black box in the figure) was mutated to R (CTG is changed into CGG), wherein T was substituted with G, i.e., G is the resulting base X1 which has been mutated.

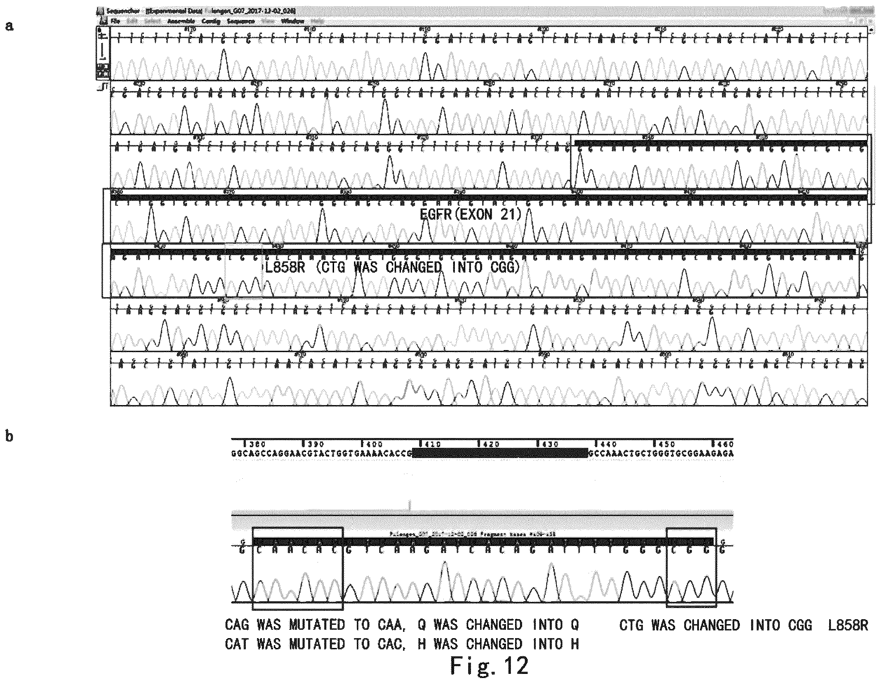

Figure 12 shows a homozygote map (i.e., a sequence map comprising only a mutant base(s)). Figure 12a shows a sequencing peak map of EGFR exon 21 (shown in black box of the figure), wherein the peak map showed at position 858 is a single base peak map (CGG), i.e., the mutation that CGG is substituted for CTG has taken place; a sense mutation has taken place at position 849 (i.e., CAG is changed into CAA, which is resulted from the substitution of base X2, i.e., A is substituted for the third base G, still encoding amino acid Q); a sense mutation has taken place at position 850(i.e., CAT is changed into CAC, which is resulted from the substitution of base X2, i.e., C is substituted for the third base T, still encoding amino acid H); Figure 12b shows an enlarged view of the sequencing peak of the mutant bases involved in Figure 12a, wherein the top panel shows the base sequences, and the bottom panel shows the base sequences and the sequenced single bases peak map.

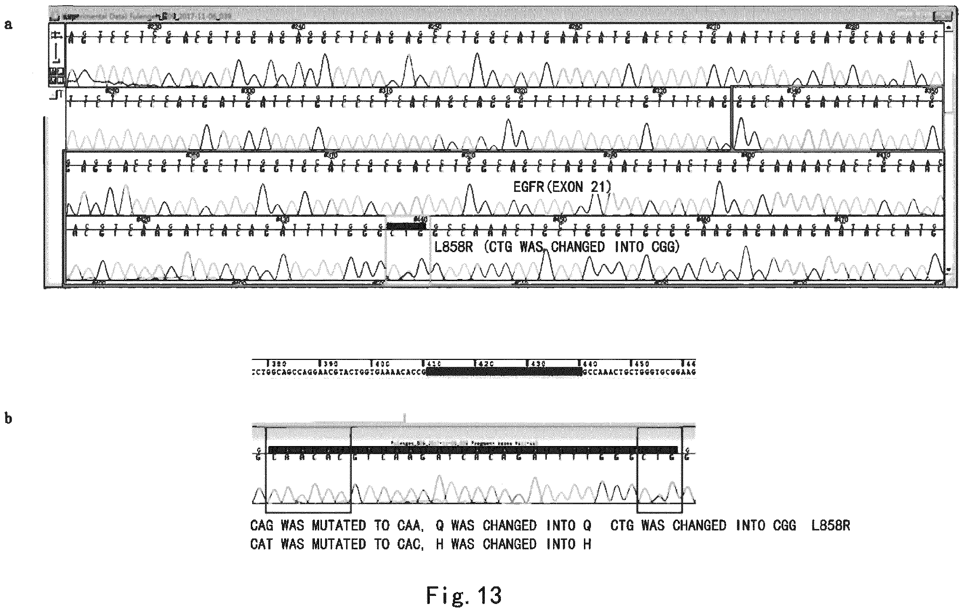

Figure 13 shows a heterozygote map (i.e., a map comprising both an original sequence and a mutant base sequence). Figure 13a shows a partial sequencing peak map of EGFR exon 21, wherein the peak map showed at position 858 (shown in black box) is a heterozygous base peak map with two peaks (this position is in a heterozygous form of 858L/858R, i.e., codons CTG and CGG are present simultaneously at this position); the peak map showed at position 849 is a heterozygous base peak map with two peaks (codons CAG and CAA are present simultaneously at this position, wherein CAA is resulted from the substitution of base X2 of A for the third base G of the original codon CAG, which is a sense mutation, still encoding amino acid Q); and the peak map showed at position 850 is a heterozygous base peak map with two peaks (codons CAT and CAC are present simultaneously at this position, wherein CAC is resulted from the substitution of base X2 of C for the third base T of the original codon CAT, still encoding amino acid H). Figure 13b shows an enlarged view of the sequencing peak of the mutant base portion involved in Figure 13a, wherein the top panel shows the base sequences, and the bottom panel shows the base sequences and the sequenced heterozygous bases peak map.

Figure 14 shows the detection of human HCT 116 (WT) (left panel) and HCT 116 L858R cell strain (right panel) by FISH: the VividFISH™ human CEP07/EGFR specific detection probe can be hybridized to HCT 116 (WT) wild-type cell strain and HCT 116 EGFR-L858R mutant cell strain.

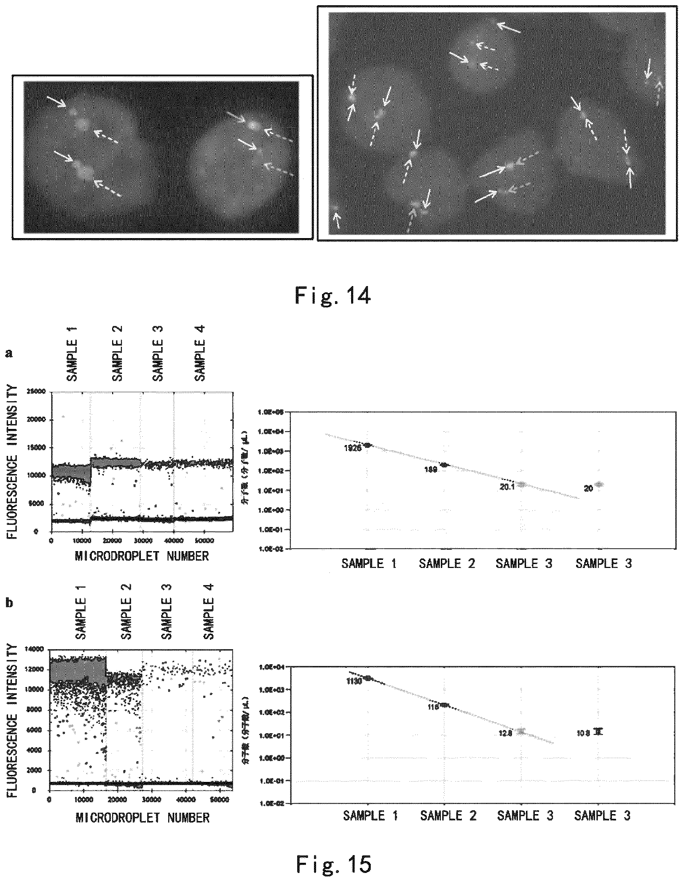

Figure 15 shows the molecule number of the mutant gene (EGFR L858R or BRAF V600E) in the gDNA standard detected by ddPCR, and the scattergrams of the numbers of positive microdroplets and negative microdroplets detected by Bio-Rad QX200 ddPCR in the gDNA standard diluted sample 1 (100 ng/$\mu$L), sample 2 (10 ng/$\mu$L), and sample 3 (1 ng/$\mu$L) (the left panel of Figure 15a relates to EGFR L858R; the left panel of Figure 15b relates to BRAF V600E); according to the ratio of the number of negative microdroplets to the total number of microdroplets, the molecule number (molecular number/$\mu$L) of the mutant gene in the ddPCR system (20 $\mu$L) was calculated (the right panel of Fig. 15a relates to EGFR L858R; the right panel of Fig. 15b relates to BRAF V600E).

Figure 16 shows the overall process of the NGS experiment.

Figure 17 shows a schematic diagram of a reference DNA with a substitution mutation. The base X2 is located upstream of the defined base X1, and the "*" indicates that the base is altered at that position.

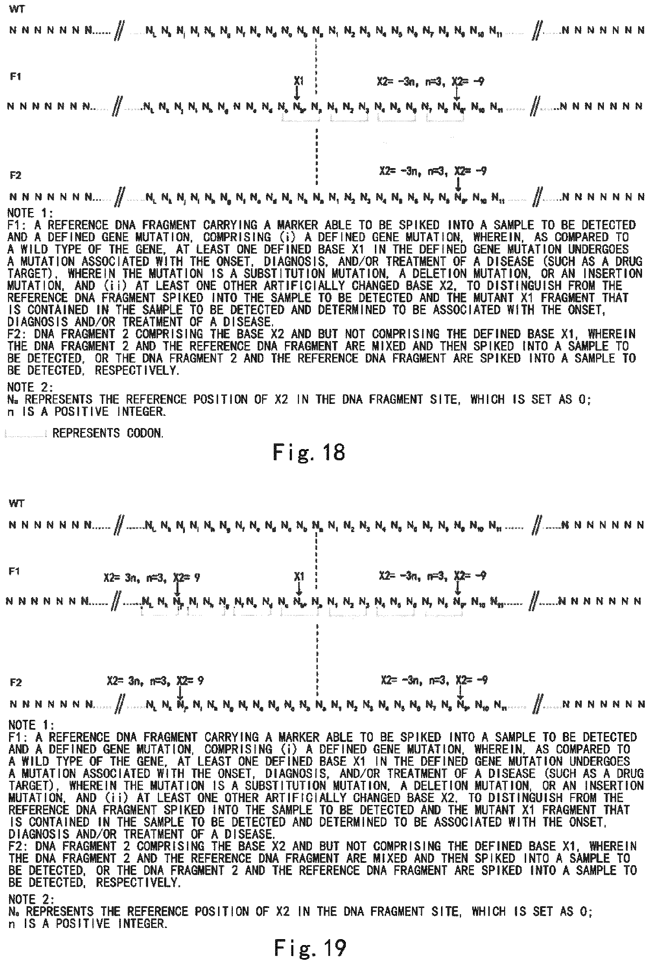

Figure 18 shows a schematic diagram of a reference DNA with a substitution mutation. The base X2 is located downstream of the defined base X1, and the "*" indicates that the base is altered at that position.

Figure 19 shows a schematic diagram of a reference DNA with a substitution mutation. The base X2s are located upstream and downstream of the defined base X1, and the "*" indicates that the base is altered at that position.

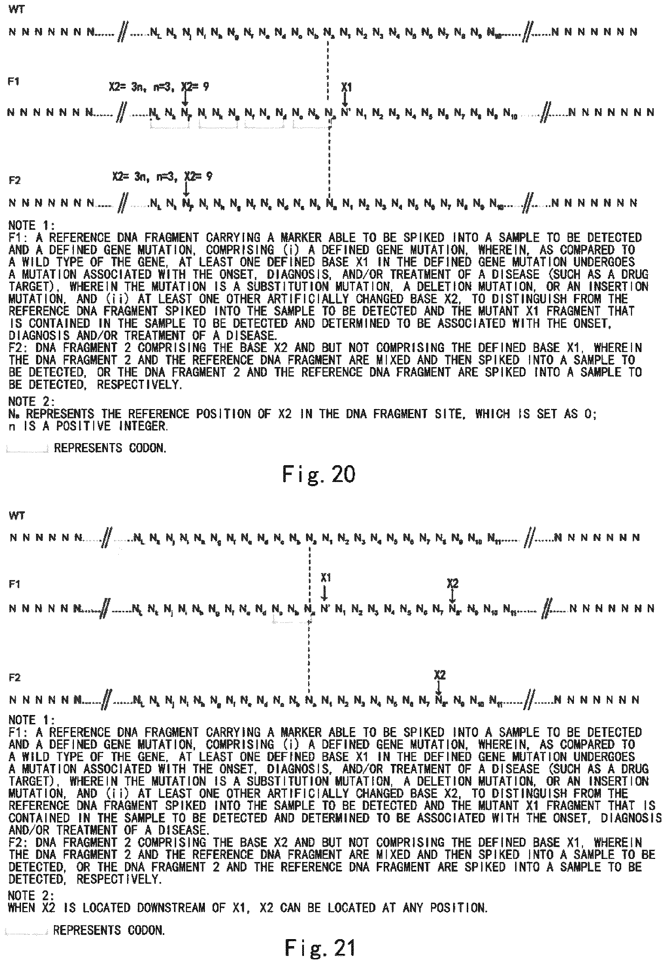

Figure 20 shows a schematic diagram of a reference DNA with an insertion mutation(s) (one of the defined base X1 is inserted). The base X2 is located upstream of the defined base X1, and the "*" indicates that the base is altered at that position.

Figure 21 shows a schematic diagram of a reference DNA with an insertion mutation(s) (one of the defined base X1 is inserted). The base X2 is located downstream of the defined base X1, and the "*" indicates that the base is altered at that position.

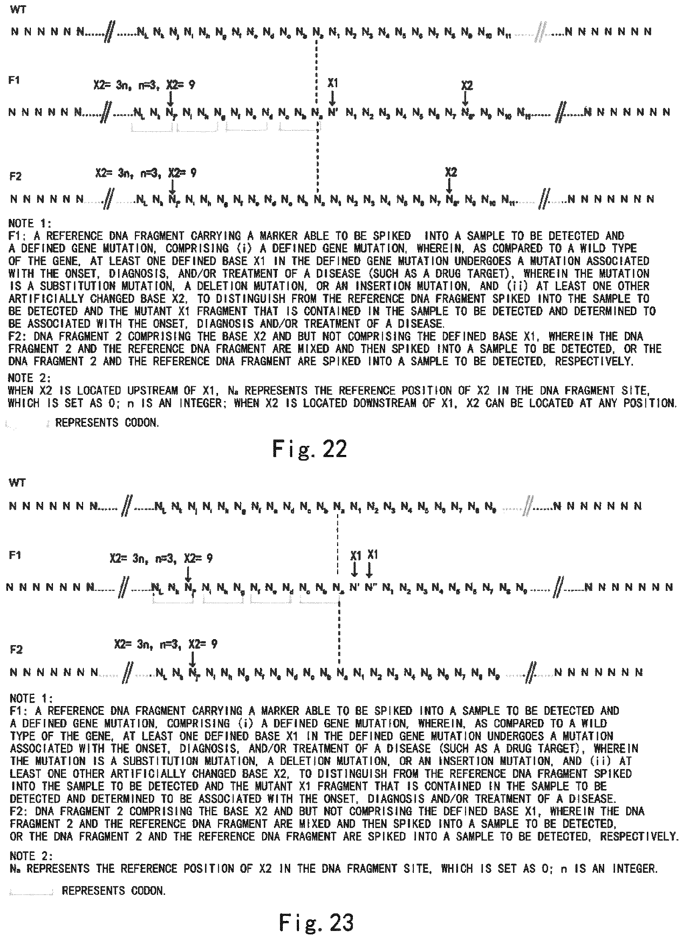

Figure 22 shows a schematic diagram of a reference DNA with an insertion mutation(s) (one of the defined base X1 is inserted). The base X2s are located upstream and downstream of the defined base X1, and the "*" indicates that the base is altered at that position.

Figure 23 shows a schematic diagram of a reference DNA with an insertion mutation(s) (two of the defined base X1 are inserted). The base X2 is located upstream of the defined base X1, and the "*" indicates that the base is altered at that position.

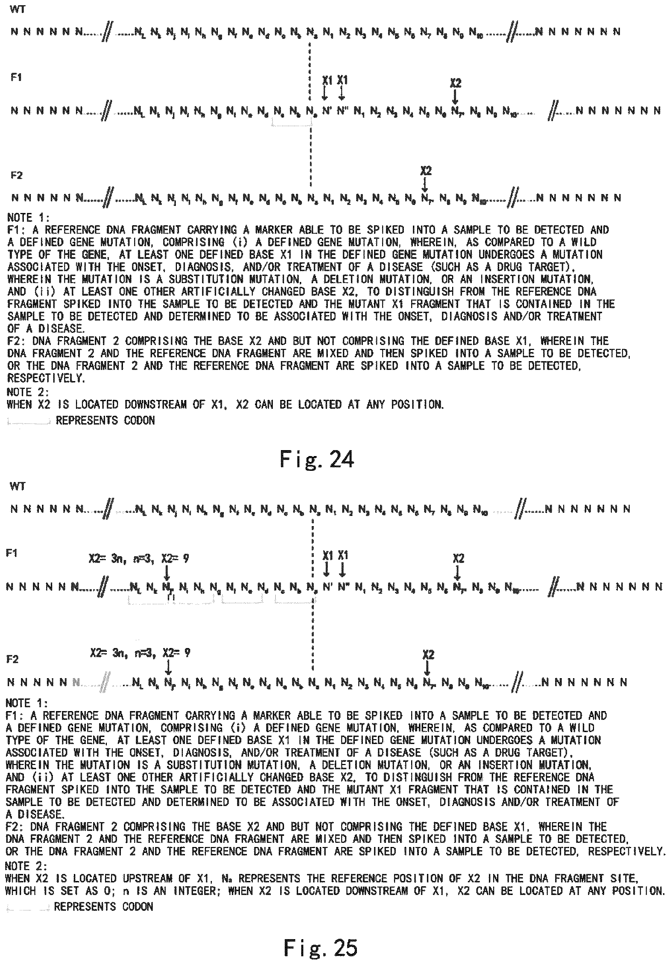

Figure 24 shows a schematic diagram of a reference DNA with an insertion mutation(s) (two of the defined base X1 are inserted). The base X2 is located downstream of the defined base X1, and the "*" indicates that the base is altered at that position.

Figure 25 shows a schematic diagram of a reference DNA with an insertion mutation(s) (two of the defined base X1 are inserted). The base X2s are located upstream and downstream of the defined base X1, and the "*" indicates that the base is altered at that position.

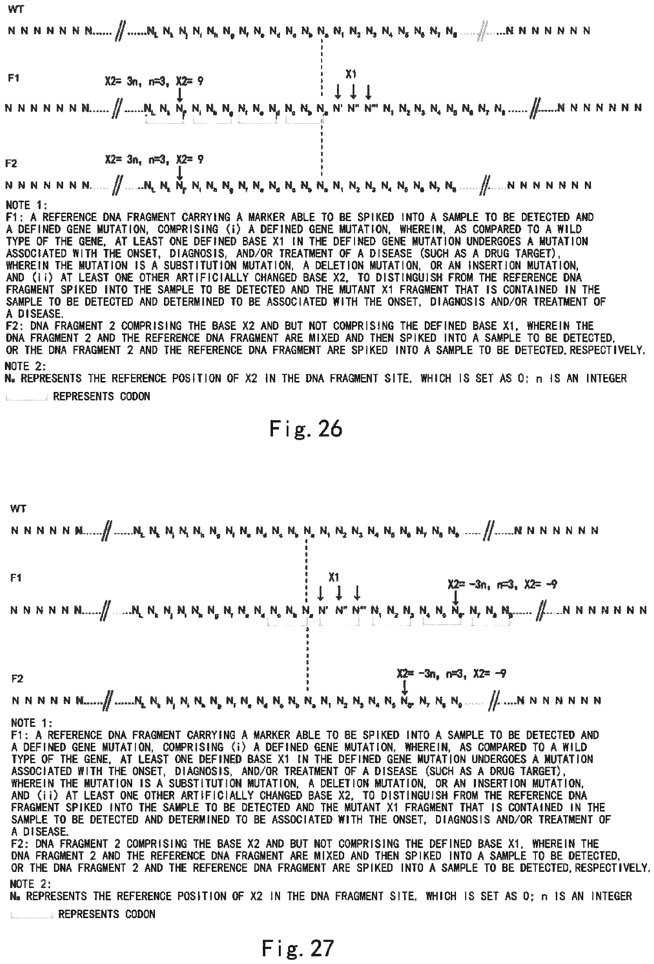

Figure 26 shows a schematic diagram of a reference DNA with an insertion mutation(s) (three of the defined base X1 are inserted). The base X2 is located upstream of the defined base X1, and the "*" indicates that the base is altered at that position.

Figure 27 shows a schematic diagram of a reference DNA with an insertion mutation(s) (three of the defined base X1 are inserted). The base X2 is located downstream of the defined base X1, and the "*" indicates that the base is altered at that position.

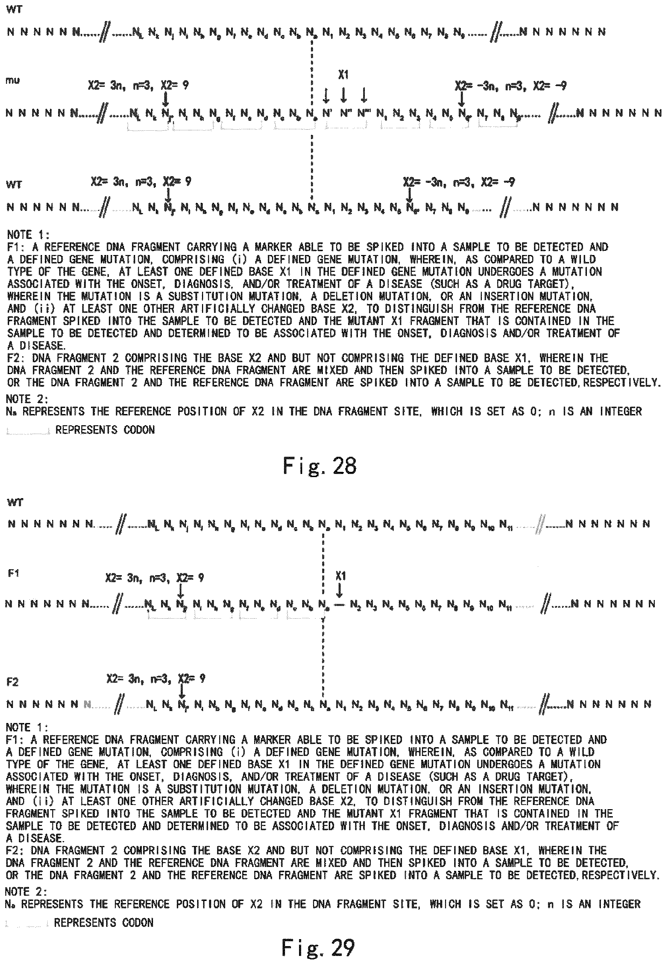

Figure 28 shows a schematic diagram of a reference DNA with an insertion mutation(s) (three of the defined base X1 are inserted). The base X2s are located upstream and downstream of the defined base X1, and the "*" indicates that the base is altered at that position.

Figure 29 shows a schematic diagram of a reference DNA with a deletion mutation(s) (one of the defined base X1s is deleted). The base X2 is located upstream of the defined base X1, and the "*" indicates that the base is altered at that position.

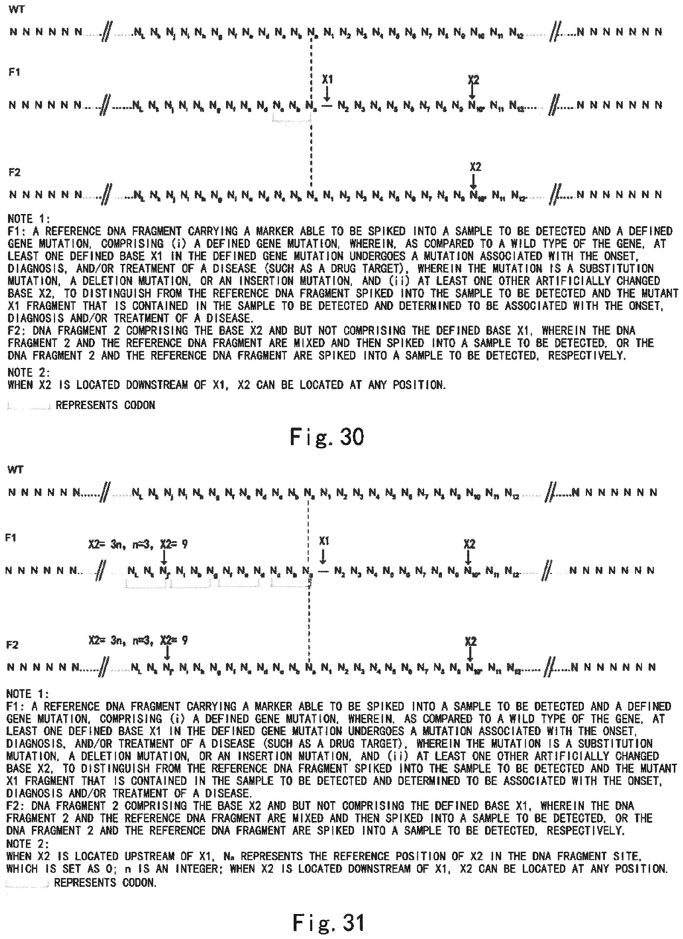

Figure 30 shows a schematic diagram of a reference DNA with a deletion mutation(s) (one of the defined base X1s is deleted). The base X2 is located downstream of the defined base X1, and the "*" indicates that the base is altered at that position.

Figure 31 shows a schematic diagram of a reference DNA with a deletion mutation(s) (one of the defined base X1s is deleted). The base X2s are located upstream and downstream of the defined base X1, and the "*" indicates that the base is altered at that position.

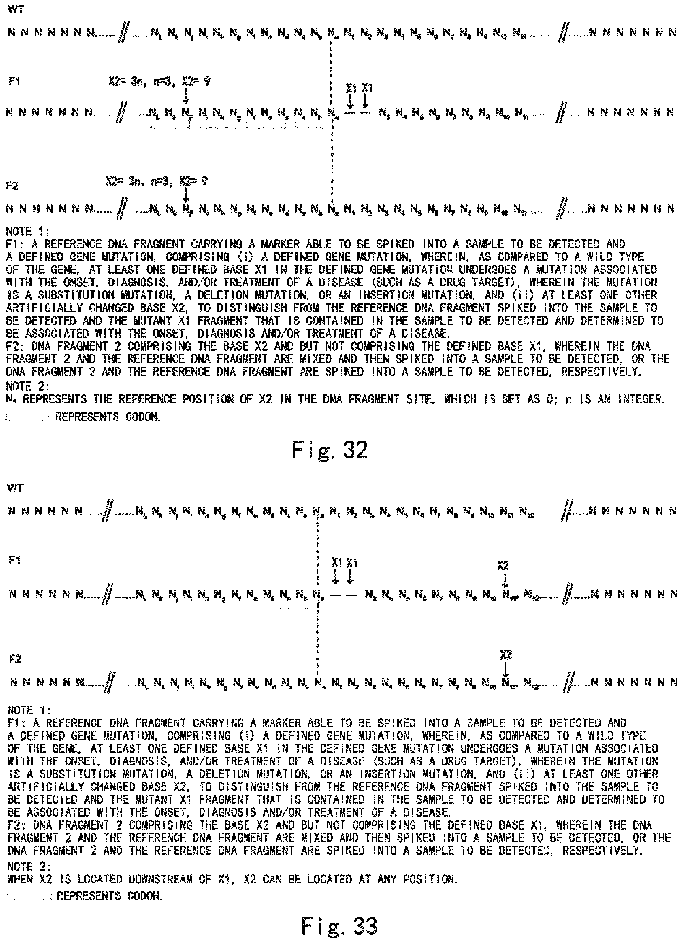

Figure 32 shows a schematic diagram of a reference DNA with a deletion mutation(s) (two of the defined base X1s are deleted). The base X2 is located upstream of the defined base X1, and the "*" indicates that the base is altered at that position.

Figure 33 shows a schematic diagram of a reference DNA with a deletion mutation(s) (two of the defined base X1s are deleted). The base X2 is located downstream of the defined base X1, and the "*" indicates that the base is altered at that position.

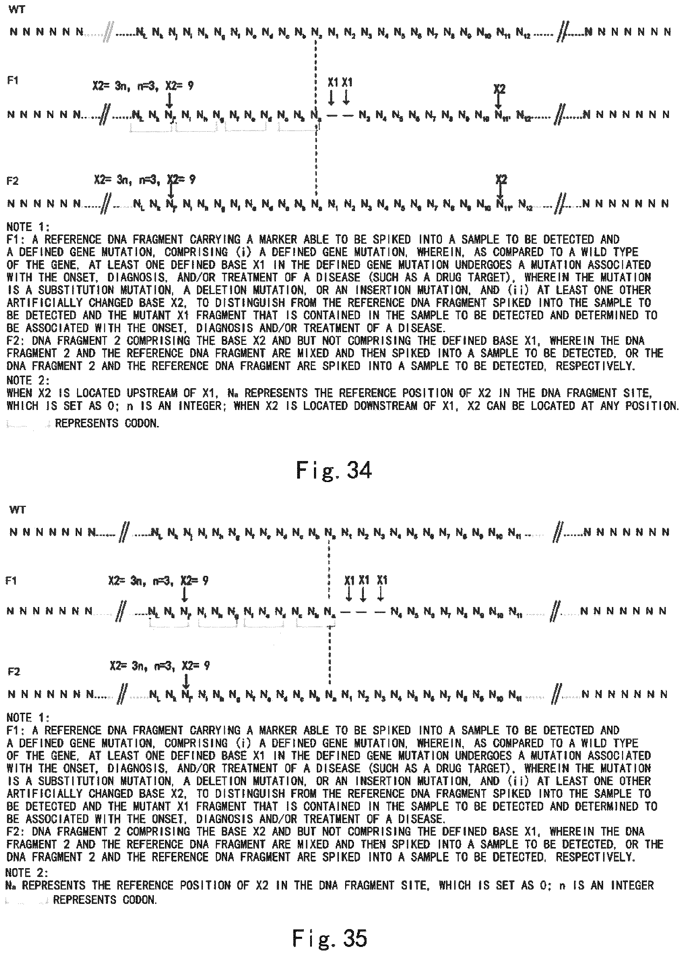

Figure 34 shows a schematic diagram of a reference DNA with a deletion mutation(s) (two of the defined base X1 are deleted). The base X2s are located upstream and downstream of the defined base X1, and the "*" indicates that the base is altered at that position.

Figure 35 shows a schematic diagram of a reference DNA with a deletion mutation(s) (three of the defined base X1s are deleted). The base X2 is located upstream of the defined base X1, and the "*" indicates that the base is altered at that position.

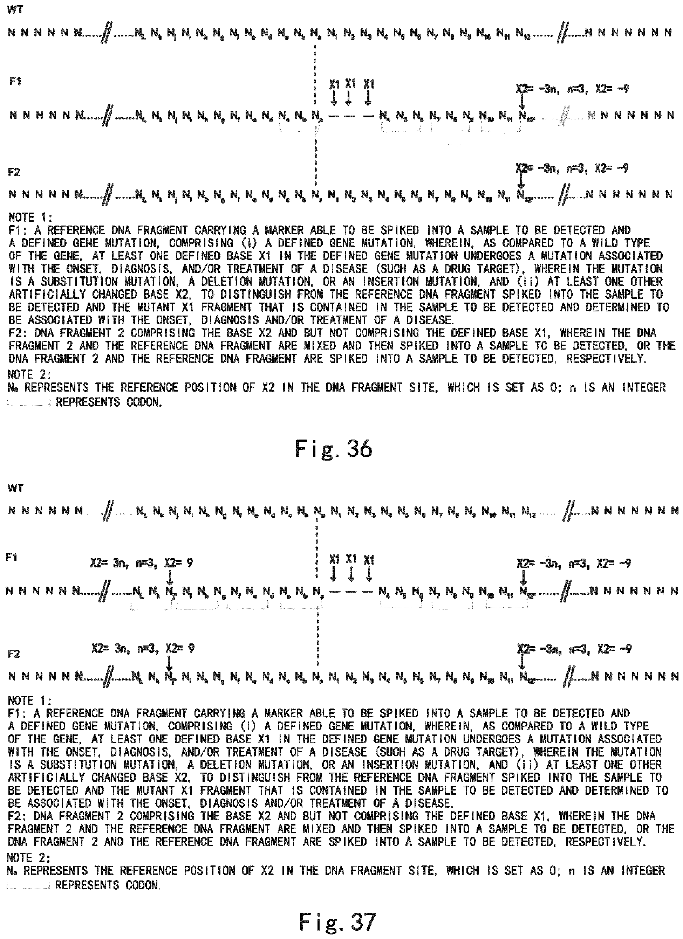

Figure 36 shows a schematic diagram of a reference DNA with a deletion mutation(s) (three of the defined base X1s are deleted). The base X2 is located downstream of the defined base X1, and the "*" indicates that the base is altered at that position.

Figure 37 shows a schematic diagram of a reference DNA with a deletion mutation(s) (three of the defined base X1s are deleted). The base X2s are located upstream and downstream of the defined base X1, and the "*" indicates that the base is altered at that position.

Figure 38 shows a reference DNA fragment with a base changed at a specific position and the use thereof.

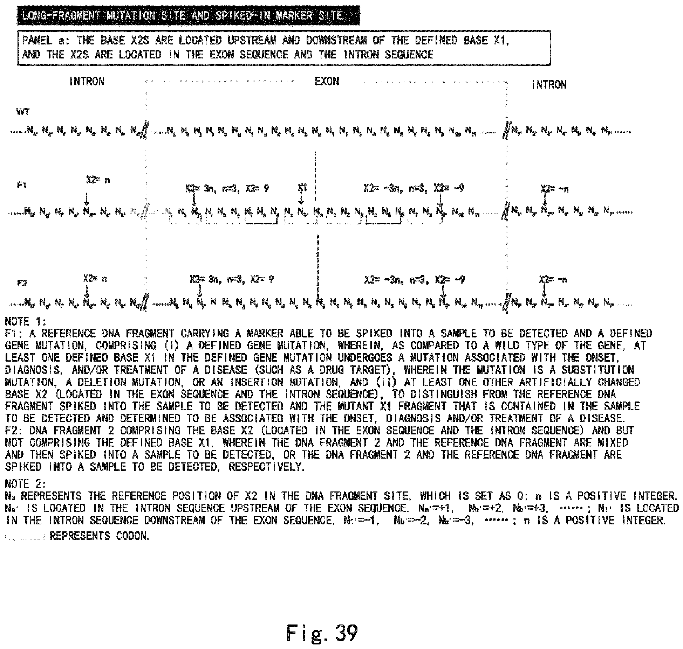

Figure 39 shows a schematic diagram of a reference DNA with a long-fragment mutation site. The base X2s are located upstream and downstream of the defined base X1, and the X2s are located in the Exon sequence and the Intron sequence, wherein the "*" indicates that the base is altered at that position.

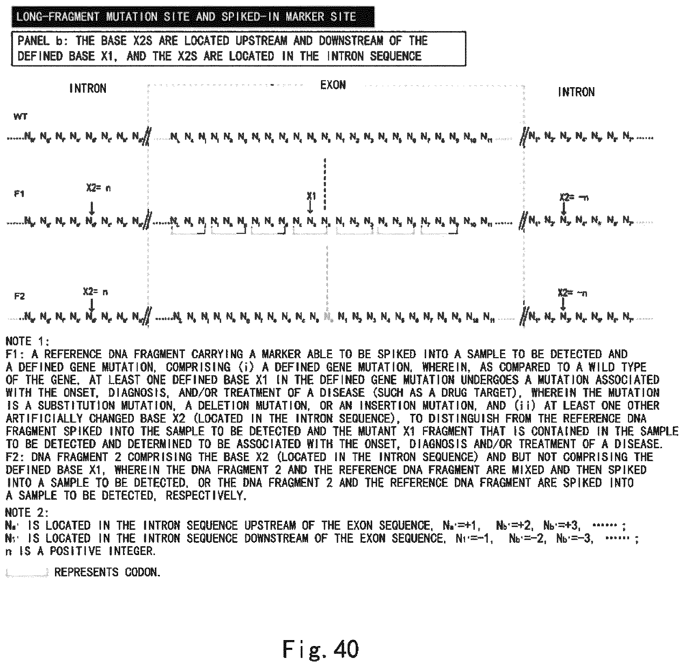

Figure 40 shows a schematic diagram of a reference DNA with a long-fragment mutation site. The base X2s are located upstream and downstream of the defined base X1, and the X2s are located in the Intron sequence, wherein the "*" indicates that the base is altered at that position.

## DETAILED DESCRIPTION OF THE INVENTION

In order to make the above described objects, features and advantages of the present disclosure more apparent, the embodiments of the present disclosure will be described in detail below with reference to the accompanying drawings. Numerous specific details are set forth in the description below in order to provide a thorough understanding of the invention. The present disclosure can be implemented in many other ways which are different from those described herein, and those skilled in the art can make similar improvements without departing from the spirit of the present disclosure. Therefore, the protection scope of the present disclosure is defined by the claims, and is not be limited by the Examples disclosed below.

### Example 1: Construction of a cell strain carrying a marker able to be spiked into a sample to be detected and an EGFR L858R mutation

Experimental instruments and reagents

DNA Polymerase (GeneCopoeia, C0103A); Primer Oligo (Invitrogen); Donor cloning vector pDonor-D04.1 (GeneCopoeia); T4 DNA Ligase (GeneCopoeia, A0101A); Fast-Fusion™ Cloning Kit (GeneCopoeia, FFPC-C020); Gel Extraction Kit (Omega); 2T1 competent cell (GeneCopoeia, U0104A); STBL3 competent cell (GeneCopoeia, U0103A); restriction enzyme (Fermentas); DNA Ladder(GeneCopoeia); E.Z.N.A.® Gel Extraction Kit (OMEGA); UltraPF™ DNA Polymerase Kit(GeneCopoeia, C0103A); E.Z.N.A.® Plasmid Mini Kit I (OMEGA); Endotoxin-free Plasmid mini/Mid Kit (Omega); PCR instrument(Takara).

**A. Construction of a donor clone that carries a marker able to be spiked into a sample to be detected and an EGFR L858R mutation;**

**A1. Design of the vector**

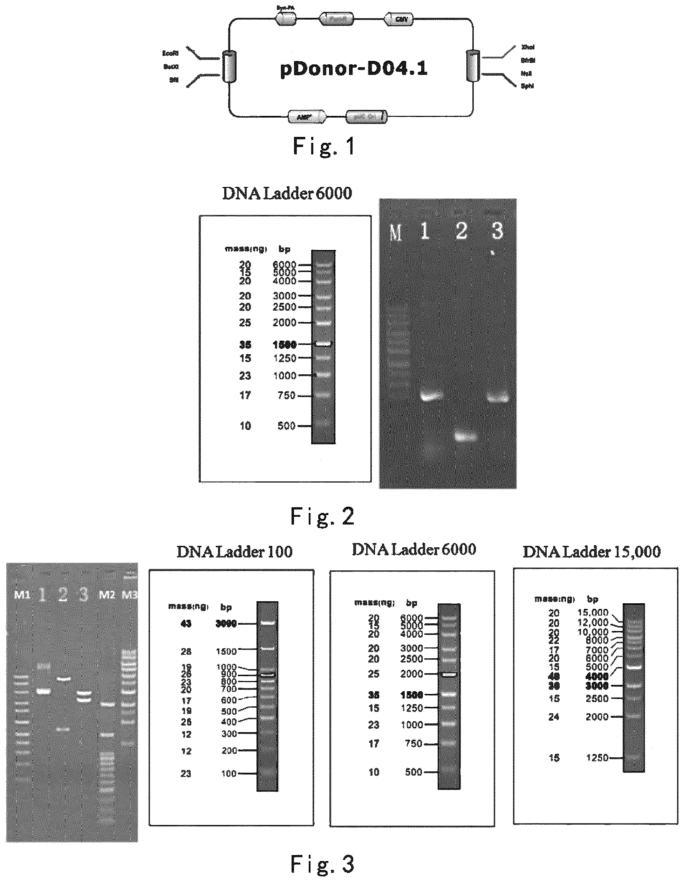

**1. The backbone of the vector is shown in Fig. 1.**

**2. Information of the donor clone**

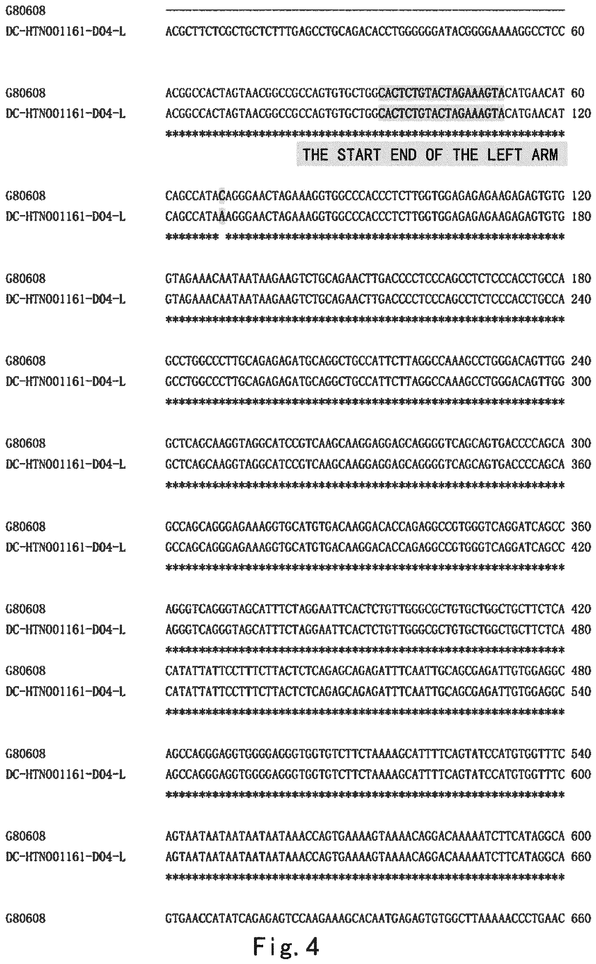

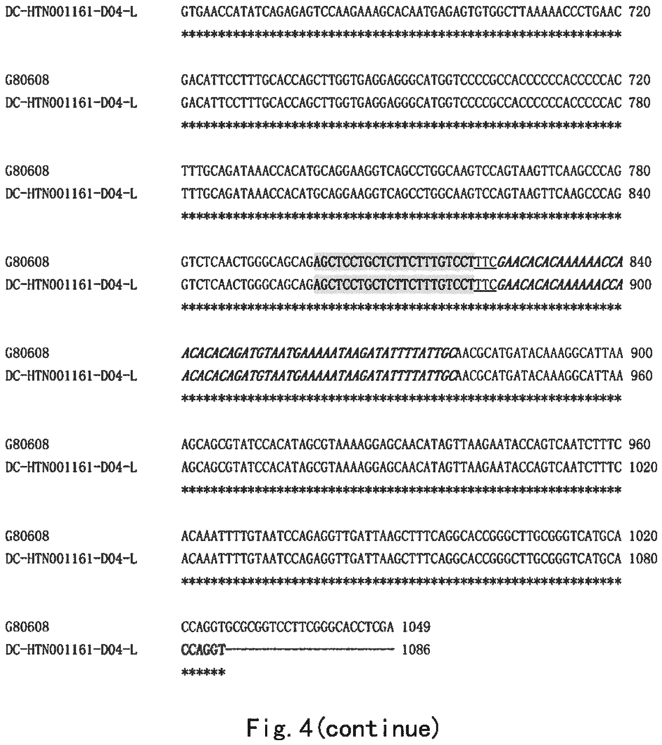

Firstly, a reference sequence was designed according to the NCBI number NG_007726.3 of EGFR as a left arm, and the sequence is set forth in DC-HTN001161-D04-L in Fig. 4.

Then, according to the NCBI number NG_007726.3 of EGFR, the sequence comprising the L858R mutation (wherein, CTG was substituted with CGG), 849Q (wherein, CAG was substituted with CAA) and 850H (wherein, CAT was substituted with CAC) was designed as the reference sequence of the right arm, and the sequence is set forth in DC-HTN001161-D04-R in Fig. 4.

**3. Construction steps**

**3.1 The obtaining of the left arm of a homogenous arm**

PCR primers

RD05348_PF:TAGTAACGGCCGCCAGTGTGCTGGCACTCTGTACTAGAAAGTACAT
GAACATCAG (SEQ ID NO:1)

RD05348_PR:GTGTTGGTTTTTTGTGTGTTCGAAAGGACAAAGAAGAGCAGGAGC
TCTGCTGCV (SEQ ID NO:2)

The cell line HEK-293 (ATCC) was used to extract human genomic DNA as a template, RD05348_PF + RD05348_PR was used as a primer, and the template was amplified by PCR reaction (98°C, 3 min, 1 cycle; then 98°C, 20 sec, 58°C, 30 Sec, 72°C, 1 min, 35 cycles; then 72°C, 10 min) to obtain the left arm fragment L, which is of about 836 bp.

**3.2 The obtaining of the right arm of the homogenous arm**

The chemically synthesized fragment R1 has 354 bp in total, and its sequence is shown as follows: >HTN001161R1 (SEQ ID NO: 3), comprising a sense mutation at positions 849 and 850 as described above, and a mutation at position 858 (L858R)

AGTCAATAATCAATGTCAACtggatggagaaaagttaatggtcagcagcgggttacatcttctttcatgcgcctttc
cattctttggatcagtagtcactaacgttcgccagccataagtcctcgacgtggagaggctcagagcctggcatgaacatgaccctgaattcg
gatgcagagcttcttcccatgatgatctgtccctcacagcagggtcttctctgtttcagggcatgaactacttggaggaccgtcgcttggtgca
ccgcgacctggcagccaggaacgtactggtgaaaacaccgcaAcaCgtcaagatcacagattttgggcGggccaaactgctgggtgc
gga

PCR was performed by using the human genome from the cell line HEK-293 as a template to amplify the fragment R2 according to the procedure in 3.1, wherein the PCR primers are:
RD05349_PF:GCCAAACTGCTGGGTGCGGAAGAGAAAGAATA (SEQ ID NO:4)

RD05349_PR:TAAAATTGACGCATGCATCTCGAGGCCAGTGTAGAAGAGGCTCTG

TCAGA (SEQ ID NO:5).

The obtained product R2 fragment is of about 824 bp (SEQ ID NO: 6), and the electrophoresis results are shown in Fig. 2. Then, Z.N.A.® Cycle Pure Kit from OMEGAE was used to purify the PCR products and the synthesized fragments.

>HTN001161R2 (SEQ ID NO:6)

gccaaactgctgggtgcggaagagaaagaataccatgcagaaggaggcaaagtaaggaggtggctttaggtcagccagcattttc

ctgacaccagggaccaggctgccttcccactagctgtattgtttaacacatgcaggggaggatgctctccagacattctgggtgagctcgca

gcagctgctgctggcagctgggtccagccagggtctcctggtagtgtgagccagagctgctttgggaacAgtacttgctgggacagtgaa

tgaggatgttatccccaggtgatcattagcaaatgttaggtttcagtctctccctgcaggatatataagtccccttcaatagcgcaattgggaaa

ggtcacagctgccttggtggtccactgctgtcaaggacacctaaggaacaggaaaggccccatgcggacccgagctcccagggctgtct

gtggctcgtggctgggacaggcagcaatggagtccttctctcccttcactggctcggtttctcttagggaccctcacagcactaaggggtgc

gcgtcccctgtcaggccctcgaatgccctcccacagccaggcccctctgaggtttcactctggcctgcttggctcctagcagccaccaaccc

atgatgctgggccctgaaaacacacgcagacctggatgagtgaggccactgggcacaaccagggctcccagctcaccagagcagcctg

ggacacagagggtgctcagaaacctaccagagcagccctgaactccgtcagactgaaatcccctgttgccgggaggactcgagatgcat

gcgtcaatttta.

**A2. Cloning the left arm of the homogenous arm into a vector of interest**

**1. Enzymatic cleavage of the vector**

The vector pDonor-D04.1 was cleaved with EcoRI (NEB). The cleavage product of the vector was recovered by E.Z.N.A.®GelExtraction Kit from OMEGA.

**2. The ligation of the left arm and the plasmid vector**

The In-fusion reaction was carried out by using a Fast-Fusion Cloning Kit, and the left arm L obtained in 3.1 and the cleaved vector pDonor-D04.1 were ligated to obtain the plasmid HTN001161L-D04. After the reaction is finished, E. coli competent cells 2T1 were transformed with the plasmid. The plasmid DNA was extracted with E.Z.N.A.® Plasmid Mini Kit I from OMEGA. The plasmid was then sequenced, and its left arm sequence was confirmed to be correct by comparison with the reference sequence, as shown by G80608 in Fig. 4 (SEQ ID NO: 7).

**3. Insertion of the right arm to the vector of interest HTN001161L-D04**

The plasmid HTN001161L-D04 was cleaved with XhoI (NEB). The cleavage product of the vector was recovered by E.Z.N.A.®Gel Extraction Kit from OMEGA.

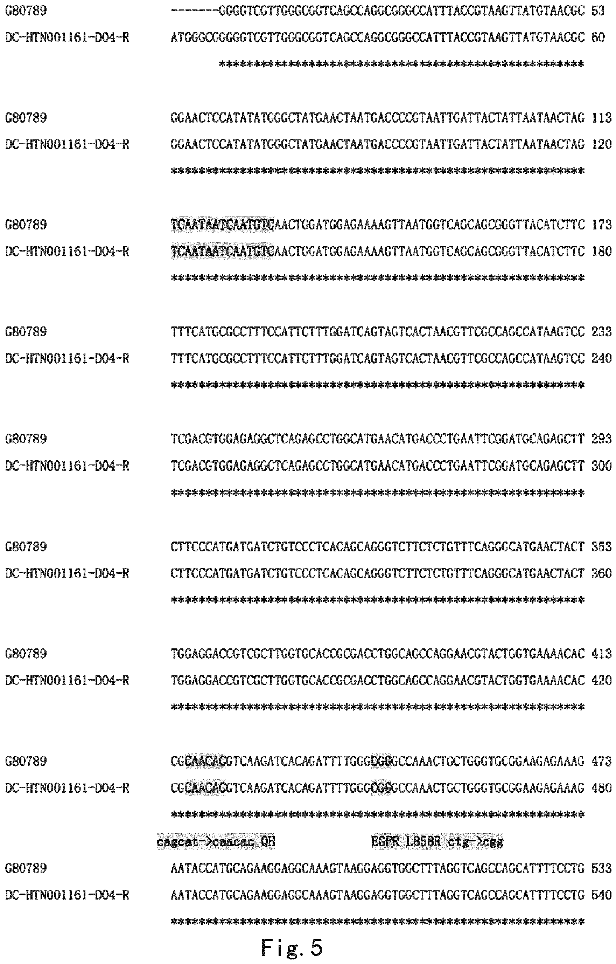

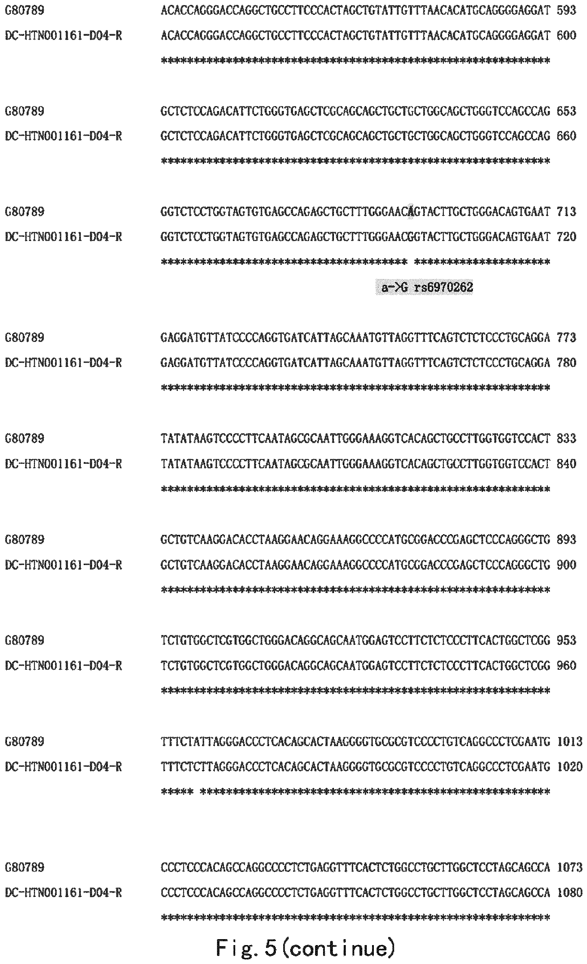

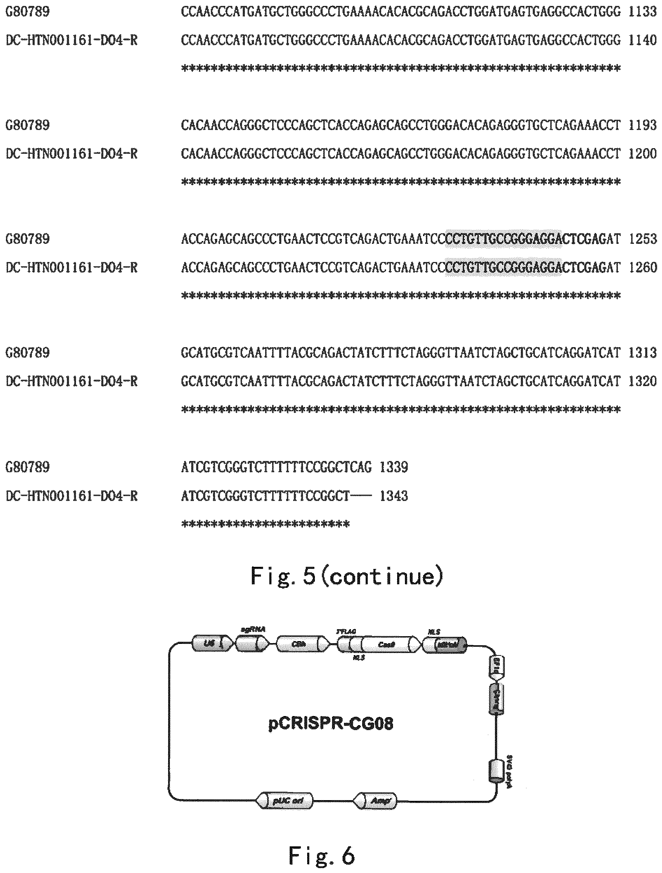

The In-fusion reaction was carried out for the right arm R1 and R2 obtained in 3.2 and the cleaved plasmid HTN001161L-D04 by using an In-Fusion® HD EcoDry™ Cloning Kit. After the reaction is finished, a transformation was carried out. The plasmid DNA was extracted with E.Z.N.A.® Plasmid Mini Kit I from OMEGA. The plasmid DC-HTN001161-D04 was sequenced, and its right arm sequence was confirmed to be correct by comparison with the reference sequence, as shown by G80789 in Fig. 5 (SEQ ID NO: 8). The donor DNA was thereby obtained.

The plasmid DC-HTN001161-D04 was cleaved with AflIII(NEB)/SapI(NEB). As shown in Fig. 3, Lane1: DC-HTN001161-D04 plasmid; Lane2: two expected bands~1703/5537bp obtained by cleaving the DC-HTN001161-D04 plasmid with Af1III; Lane3: two expected bands ~3277/3963bp obtained by cleaving the DC-HTN001161-D04 plasmid with SapI.

**B. Construction of the sgRNA clone**

**B1. Design of the vector**

The vector backbone is shown in Fig. 6.

The sgRNA target sequence is TCTGTGATCTTGACATGCTG (SEQ ID NO: 9). The sequencing primer SeqL-A sequence (5' to 3') is Ttcttgggtagtttgcag (SEQ ID NO: 10), which is a universal sequencing primer for a vector backbone.

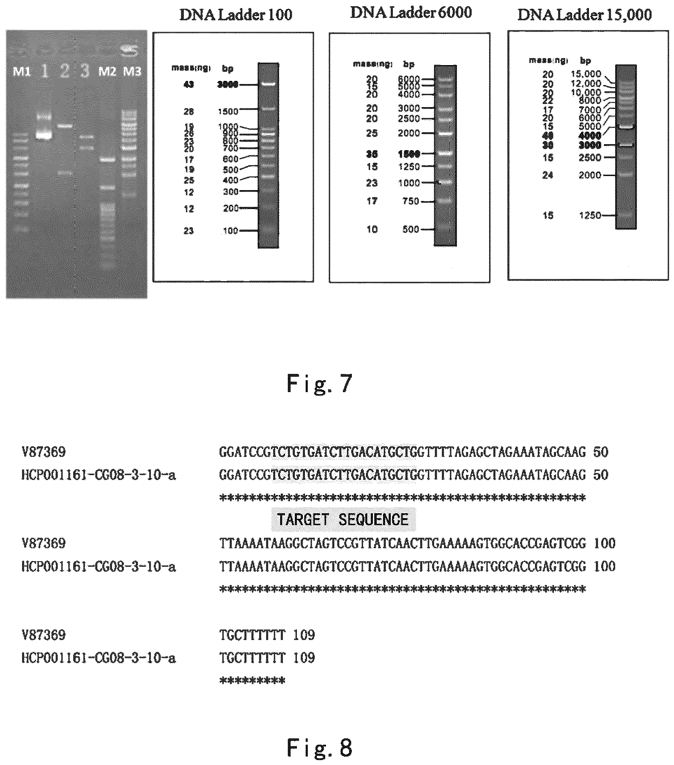

The sequence of the sgRNA was designed as shown in V87369 of Fig. 8.

Experimental instruments and reagents

Primer Oligo (Invitrogen); sgRNA cloning vector pCRISPR-CG08 (GeneCopoeia); STE Buffer; T4 DNA Ligase (GeneCopoeia, A0101A); Gel Extraction Kit (Omega); 2T1 competent cell (GeneCopoeia, U0104A); DNA Ladder (GeneCopoeia); Taq DNA Polymerase Kit (GeneCopoeia, C0101A); restriction enzyme (NEB); Endotoxin-free Plasmid mini/Mid Kit (Omega); PCR instrument(Takara).

**1. Experimental steps**

The fragment of interest was obtained by using $1\mu L$ (5 pmol/$\mu L$) of the primer PF1: 5'-atccgTCTGTGATCTTGACATGCTG-3'_(SEQ ID NO: 11) and the primer PR1: 5'-aaacCAGCATGTCAAGATCACAGAc-3' (SEQ ID NO: 12).

Annealing reaction system: $2\mu L$ STE buffer, $1\mu L$ primer PF1 (5 pmol/$\mu L$), $1\mu L$ primer PR1 (5 pmol/$\mu L$), $16\mu L$ ddH$_2$O.

Annealing reaction procedure: 95 °C 1 min, 1 cycle; 95 °C, (-1) 20 sec, 94 °C, (-1) 20 sec, 70 cycles; 25 °C, 7 min, 1 cycle; placed at 4 °C. Note: (-1) means that the temperature in each cycle is lowered by 1 °C. After the annealing reaction was completed, product A was diluted by adding 30 $\mu L$ of H$_2$O.

**2. Cloned into a vector of interest**

The vector pCRISPR-CG08 was cleaved with BbsI (NEB) and recovered by 1% agarose gel electrophoresis to obtain a linear vector.

The ligation of the annealed product and the sgRNA interference cloning vector

The annealed product A was ligated with the enzymatically cleaved vector, which is then used to transform E.coli. The plasmid DNA was extracted with EZNA® Plasmid Mini Kit I from OMEGA, and designated as HCP001161-CG08-3-10-a. By using the primer SeqL-A, the sequencing result is shown in Fig. 8 (SEQ ID NO: 13); endotoxin-free plasmid was extracted with the endotoxin-free plasmid mini/mid kit from OMEGA.

The plasmid HCP001161-CG08-3-10-a was cleaved with PvuI (NEB)/EcoRI (NEB). The result is shown in Fig. 7. Lane1: HCP001161-CG08-3-10-a plasmid; Lane2: two expected bands ~2098/7505bp obtained by cleaving the HCP001161-CG08-3-10-a plasmid with PvuI; Lane3: two expected bands ~4069/5534bp obtained by cleaving the HCP001161-CG08-3-10-a plasmid with EcoRIP.

**C. Construction of HCT 116-L858R cell strain**

**Materials and equipments**

HCT 116 cells, Junction PCR 5' primer and 3' primer (Life Technologies), various restriction enzymes (NEB), Taq DNA Polymerase Kit (GeneCopoeia, C0101A), RPMI1640 medium (Corning, Cat. No. R10-040-CVR), Gibco South American Fetal Bovine Serum (FBS) (Cat. No. 10270-106), Opti-MEM® I Reduced-Serum Medium (Gibco, 31985062), puromycin (PM) (MDBio (P/C 101-58-58-2)), STE buffer and T4 DNA ligase (GeneCopoeia, A0101A), EndoFectin™ Max Transfection Reagent (GeneCopoeia, Cat. No. EF003), Gel Recovery Kit (Omega), Endotoxin-free Plasmid mini/Mid Kit (Omega), PCR instrument (Takara), E. coli competent cells DH5$\alpha$ (GeneCopoeia, CC001), Hipure plasmid kmicro kit (OMEGA, p1001-03), MycoGuard™ Mycoplasma Bioluminescent Detection Kit (Lonza, LT07-318) and VividFISH™ CEP Kit (GeneCopoeia, FP204 and FP504), Genome Lysate (GeneCopoeia, IC003-02), Opti-MEM® I (Invitrogen, Cat. No. 31985070), 2× SuperHero PCR Mix (GeneCopoeia, IC003-01), Blunt vector (GeneCopoeia), Tissue DNA kit (Omega, D3396-02).

The culture condition of wild-type cells (a wild type HCT 116 cell strain): RPMI 1640 (90%); Heat inactivated FBS (10%).

The culture condition of a cell strain with bases altered at a specific position: RPMI1640 (90%), Heat inactivated FBS (10%), and Puromycin (0.6 µg/mL).

**C1. Culture, transfection and screening of HCT 116**

1. Culture: HCT 116 cells (ATCC® CCL-247TM) were cultured in RPMI1640 medium containing 10% FBS.
Determination of the minimum lethal concentration of puromycin to HCT 116 cells: discarding the medium and adding the medium containing different dilutions (0.1, 0.2, 0.4, 0.6, 0.8, 1.0 and 1.2, respectively) of puromycin to a 96-well plate. The 96-well plate (3 replicate wells per gradient) was placed in a CO2 incubator, at 37 °C for 3 to 5 days, to detect the minimum lethal concentration. The minimum lethal concentration determined in the experiment was 0.6 µg/mL.
2. Transfection and screening:
The HCT 116 cells were transfected with a plasmid as shown in Table 2.

**Table 2 Transfection scheme**

| Type of the well plate | Plasmid (donor DNA: sgRNA = 1:1) | Opti-MEM | EndoFectin™-MAX | Medium |
|---|---|---|---|---|
| 24-well plate | 500 ng | 50 µL | 1.5 µL | 500 µL |

Transfection efficiency was observed 24-48 h after the transfection (Fig. 9). When the cultured monoclone was expanded to about 80% confluency, it was directly lysed with Lysis buffer (the specific amount of Lysis buffer can be adjusted according to the number of cells; it is recommended to lyse $5\times10^4$ to $5\times10^5$ cells per 25 µL of Lysis Buffer); then the genomic lysate was inactivated at 56 °C for 30 min and then at 95 °C for 10 min for use; the following 5' and 3' Junction PCR primers were used to validate a positive monoclonal cell strain.
1) 5' end-Junction PCR:
One end of the primer was set upstream of the 5' homologous arm of the chromosome, and the other end was set in the vector sequence region, and the positive clone is a successfully integrated cell strain.
5' end-Junction PCR primers:

L858R-5-PF: 5'-AGCATCTTTGCGAGACCCTA-3' (SEQ ID NO: 14)
L858R-5-PR: 5'-GCAACCTCCCCTTCTACGAG-3' (SEQ ID NO: 15)

2) 3' end-Junction PCR:
One end of the primer was set downstream of the 3' homologous arm of the chromosome, and the other end was set in the vector sequence region, and the positive clone is a successfully integrated cell strain.
3' end-Junction PCR primers

L858R-3-PF: 5'-GGCGTTACTATGGGAACATACGTC-3' (SEQ ID NO: 16)
L858R-3-PR: 5'-CTTGGGATGGTGAGAGATGAGGC-3' (SEQ ID NO: 17)

3) The Junction PCR reaction system is listed as follows:

**Table 3: Reaction system**

| ingredient | volume |
|---|---|
| 2× SuperHero PCR Mix | 12.5 µL |
| Template (cell lysate) | 2 µL |
| Primer (F+R=10µM) | 1.25 µL |
| DMSO | 1.25 µL |
| ddH$_2$O | 8 µL |
| Total | 25 µL |

**Table 4: The PCR reaction procedure is as follows:**

| Temperature | Duration | Number of cycles |
|---|---|---|
| 94 °C | 5 min | 1 |
| 94 °C | 30 sec | 40 |
| 58-60 °C | 30 sec | |
| 72 °C | 1.5 min | |
| 72 °C | 7 min | 1 |
| 4 °C | ∞ | 1 |

4) Junction PCR Results

L858R-5-PF + L858R-5-PR was subjected to 5' Junction PCR by using different numbered genomes as templates to obtain a 1350 bp of 5' Junction fragment; L858R-3-PF + L858R-3-PR was subjected to 5' Junction PCR by using different numbered genomes as templates to obtain a 1437 bp of 3' Junction PCR fragment, as shown in Figure 10 (each gene site produces a different size of Junction PCR product, depending on its primers).

**C2. Sequencing the PCR products to confirm positive cell strain sequences**

The positive clone lysate identified above was subjected to Junction PCR again, and the reaction system was as follows:

**Table 5: Reaction system**

| ingredient | volume |
|---|---|
| RD PCR Mix | 15 μL |
| Template (cell lysate) | 2 μL |
| Primer (F+R=10μM) | 1 μL |
| 10×TMAC | 5 μL |
| ddH$_2$O | 2 μL |
| Total | 25 μL |

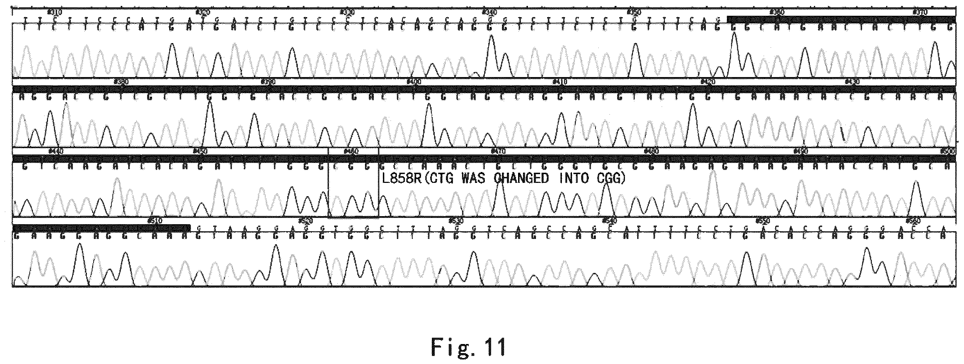

The reaction procedure was: 98°C 3 min, 1 cycle; 98°C 20 sec, 58°C, 30sec, 72°C 55 sec, 25 cycles; 72°C 7 min, 1 cycle; 98°C 3 min, 1 cycle; 98°C 20 sec, 58°C 30 sec, 72°C 55 sec, 25 cycles; 72°C 7 min, 1 cycle; then placed at 16°C. The results of the band of interest detected by 2% gel are the same as those in Fig. 10. The sequencing results are shown in Figure 11.

**C3. Identifying whether genes in the monoclonal cell strain being homozygous or heterozygous by Junction PCR**

On the basis of the identification results of the above positive clone, a batch of cell genomic DNA was re-extracted for identifying whether the following genes are homozygous or heterozygous.

3' end-Junction PCR (for identifying genes being homozygous or heterozygous): one end of the primer was set on the 3' homology arm of the chromosome, and the other end was set in the Intron region of the vector sequence; the primer sequences are shown in Table 6; the PCR product was sequenced; if a single peak was shown at the mutation position, it indicated a homozygote cell strain, as shown in Figure 12; if a double peak was shown at the mutation position, it indicated a heterozygote cell strain, as shown in Figure 13.

**Table 6. The primers for identification whether the genes in the monoclonal cell strain being homozygous or heterozygous**

| Type | Primer name | Sequence | Product |
|---|---|---|---|
| L858R-3'end | RD05702_PF | GGAGAAAAGTTAATGGTCAGCAGCG (SEQ ID NO: 18) | 1251bp |
| | L858R-PR | CTTGGGATGGTGAGAGATGAGGC (SEQ ID NO: 19) | |

**C4. Fluorescence in situ hybridization to verify the chromosome or gene status**

VividFISH™ CEP07/EGFR gene detection probes (VividFISH™ CEP Kit, FP204 and FP504) can specifically detect abnormal amplification of the EGFR gene located on chromosome 7. EGFR is a typical proto-oncogene whose activity is associated with a variety of cancers with low survival rates, including lung cancer and breast cancer.

1. Probe description: the hybridization solution of the VividFISH™ FISH probe contains fluorophore-labeled DNA and blocked DNA. The VividFISH™ FISH LSI probe is in the form of ready to use.
2. Materials
Solution preparation (not included in the kit): Pretreatment solution: 50 mL 2×SSC, 0.5% NP-40, pH 7.0, stored at 4 °C. Denaturing solution: 50 mL of 70% formamide; freshly prepared 1 × SSC, pH 7.0. Washing buffer: 100 mL of 0.5 × SSC, 0.1% NP-40, stored at 4 °C.
3. Slide pretreatment: cell slide specimen

1) 50 mL of the pretreatment solution was added to the slide specimen bottle and prewarmed in a 37 °C water bath.
2) The positive monoclonal cell strain and the normal cell strain slide specimens were placed in a pretreatment solution which had been prewarmed to 37° C, and incubated for 30 minutes.
3) The slide specimens were dehydrated in 70%, 90%, and 100% ethanol for 1 minute, respectively, and then air dried.
4) The slide specimens were placed into the specimen box and stored at room temperature until the next step.

4. Hybridization

The FISH probe was melted at room temperature, and hybridization was carried out according to the instructions of VividFISH™ CEP Kit (GeneCopoeia, FP204 and FP504) and then the slide specimens were washed. The result was observed using a fluorescence microscope.
The fluorescence microscope parameters are as follows:

Microscope: a fluorescence microscope with a 100 watt mercury bulb.

Objective lens: 25× to 100× objective lens, used in combination with 10× ocular lens.

For FISH signal counting, the desired effect can be achieved with a 60× or 100× oil immersion objective lens.
Filters: filters are designed with specific fluorescent dyes and must be selected in a targeted manner.
The results of fluorescence development are shown in Fig. 14. The number of chromosomes of the mutant cells edited by GE is consistent with that of normal cells (no difference). Red represents gene hybridization (as indicated by the solid arrow) and green represents chromosome hybridization (as indicated by the dotted arrow). As can be seen from the figure, the numbers of genes and chromosomes are the same no matter they are mutant or wild-type.
According to the method of the above example, DNA fragments carrying the defined mutant base X1 and the marker base X2 able to be spiked into the sample to be detected are also constructed as shown in Table 7 and Table 25 below.

**Table 7 List of other reference DNA fragments (according to X2=3n, construction of spiked-in markers at 500 bp upstream and downstream)**

| Gene name | GeneBank number of wild-type gene | Defined mutation (substitution) base X1 | spiked-in marker base X2 | Exon sequence of the reference DNA carrying a defined mutation and a marker able to be spiked-in |
|---|---|---|---|---|
| BRAF | Gene ID:673 | V600E(gtg was mutated into gag) | Located upstream, 597L(CT*A* was mutated into CT*T*); Located downstream, 603R(CG*A* was mutated into CG*T*) | atatatttcttcatgaagacctcacagtaaaaataggtgattttggtctTgctacag AgaaatctcgTtggagtgggtcccatcagtttgaacagttgtctggatccattttg tggatg  (SEQ ID NO:20) |
| cKIT | Gene ID: 3815 | D816V(gac was mutated into gtc) | Located upstream, 813L(CT*A* was mutated into CT*T*); Located downstream, 819N(AA*T* was mutated into AA*C*) | Tgtattcacagagacttggcagccagaaatatcctccttactcatggtcggatca caaagatttgtgattttggtctTgccagagTcatcaagaaCgattctaattatgtg gttaaaggaaac  (SEQ ID NO:**21**) |
| EGFR | Gene ID: 1956 | ΔE746-A750 (del gaattaagagaagca) | Located upstream, 743A(GC*T* was mutated into GC*A*); Located downstream, 753P(CC*G* was mutated into CC*A*) | GgactctggatcccagaaggtgagaaagttaaaattcccgtcgcAatcaagac atctccAaaagccaacaaggaaatcctcgat (SEQ ID NO:**22**) |
| EGFR | Gene ID: 1956 | T790M(ACG was mutated into ATG) | Located upstream, 787Q(CA*G* was mutated into CA*A*); Located downstream, 792L(CT*C* was mutated into CT*T*) | Gaagcctacgtgatggccagcgtggacaacccccacgtgtgccgcctgctgg gcatctgcctcacctccaccgtgcaActcatcaTgcagctTatgcccttcggct gcctcctggactatgtccgggaacacaaagacaatattggctcccagtacctgct caactggtgtgtgcagatcgcaaag (SEQ ID NO:23) |
| EGFR | Gene ID: 1956 | V769-D770insASV(ins CCAGCGTGG) | Located upstream, 767A(GC*C* was mutated into GC*T*); Located downstream, 772P(CC*C* was mutated into CC*T*) | GaagcctacgtgatggcTagcgtgCCAGCGTGGgacaacccTcacg tgtgccgcctgctgggcatctgcctcacctccaccgtgcaActcatcacgcagc tcatgcccttcggctgcctcctggactatgtccgggaacacaaagacaatattgg ctcccagtacctgctcaactggtgtgtgcagatcgcaaag (SEQ ID NO:**24**) |

| Gene name | GeneBank number of wild-type gene | Defined mutation (substitution) base X1 | spiked-in marker base X2 | Exon sequence of the reference DNA carrying a defined mutation and a marker able to be spiked-in |
|---|---|---|---|---|
| EGFR | Gene ID: 1956 | T790M+C797S(T790M : acg was mutated into atg; C797S : tgc was mutated into agc) | 787Q upstream of T790M (CA**G** was mutated into CA**A**); 794P between T790M and C797S (CC**C** was mutated into CC**T**); 800D downstream of C797S of EGFR (GA**C** was mutated into GA**T**) | Gaagcctacgtgatggccagcgtggacaacccccacgtgtgccgcctgctgg gcatctgcctcacctccaccgtgcaActcatcaTgcagctcatgccTttcggc AgcctcctggaTtatgtccgggaacacaaagacaatattggctcccagtacctg ctcaactggtgtgtgcagatcgcaaag   (SEQ ID NO:**25**) |
| EGFR | Gene ID: 1956 | G719S(GGC was mutated into AGC) | Located upstream, 716K(AA**A** was mutated into AA**G**); Located downstream, 722A(GC**G** was mutated into GC**A**) | Cttgtggagcctcttacacccagtggagaagctcccaaccaagctctcttgagg atcttgaaggaaactgaattcaaaaagatcaaGgtgctgAgctccggtgcAtt cggcacggtgtataag   (SEQ ID NO:**26**) |
| KRAS | Gene ID: 3735 | G12D(GGT was mutated into GAT) | Located upstream, 9V(GT**T** was mutated into GT**A**); Located downstream, 15G(GG**C** was mutated into GG**A**) | AtgactgaatataaacttgtggtagtAggagctgAtggcgtaggAaagagtg ccttgacgatacagctaattcagaatcattttgtggacgaatatgatccaacaatag ag (SEQ ID NO:**27**) |
| KRAS | Gene ID: 3735 | G13D(GGC was mutated into GAC) | Located upstream, 10G(GG**A** was mutated into GG**G**); Located downstream, 16K(AA**G** was mutated into AA**A**) | AtgactgaatataaacttgtggtagttggGgctggtgAcgtaggcaaAagtgc cttgacgatacagctaattcagaatcattttgtggacgaatatgatccaacaataga g (SEQ ID NO:**28**) |
| NRAS | Gene ID: 4893 | Q61K(CAA was mutated into AAA) | Located upstream, 58T(AC**A** was mutated into AC**T**); Located downstream, 64Y(TA**C** was mutated into TA**T**) | Gattcttacagaaaacaagtggttatagatggtgaaacctgtttgttggacatact ggatacTgctggaAaagaagagtaTagtgccatgagagaccaatacatgag gacaggcgaaggcttcctctgtgtatttgccatcaataatagcaagtcatttgcgg atattaacctctacag (SEQ ID NO:**29**) |

| Gene name | GeneBank number of wild-type gene | Defined mutation (substitution) base X1 | spiked-in marker base X2 | Exon sequence of the reference DNA carrying a defined mutation and a marker able to be spiked-in |
|---|---|---|---|---|
| NRAS | Gene ID: 4893 | A59T(gct was mutated into Act) | Located upstream, 56L(CT**G** was mutated into CT**A**); Located downstream, 62E(GA**A** was mutated into GA**G**) | Gattcttacagaaaacaagtggttatagatggtgaaacctgtttgttggacatact AgatacaActggacaagaGgagtacagtgccatgagagaccaatacatgag gacaggcgaaggcttcctctgtgtatttgccatcaataatagcaagtcatttgcgg atattaacctctacag  (SEQ ID NO:**30**) |
| PIK3CA | Gene ID: 5290 | H1047R(CAT was mutated into CGT) | Located upstream, 1044N(AA**T** was mutated into AA**C**); | Gtttcaggagatgtgttacaaggcttatctagctattcgacagcatgccaatctctt cataaatcttttctcaatgatgcttggctctggaatgccagaactacaatcttttgatg |
|  |  |  | Located downstream, 1050G(GG**C** was mutated into GG**A**) | acattgcatacattcgaaagaccctagccttagataaaactgagcaagaggcttt ggagtatttcatgaaacaaatgaaCgatgcacGtcatggtggAtggacaacaa aaatggattggatcttccacacaattaaacagcatgcattgaactga (SEQ ID NO:**31**) |
| PIK3CA | Gene ID: 5290 | E545K(GAG was mutated into AAG) | Located upstream, 542E(GA**A** was mutated into GA**G**); Located downstream, 548K(AA**A** was mutated into AA**G**) | Agtaacagactagctagagacaatgaattaagggaaaatgacaaagaacagct caaagcaatttctacacgagatcctctctctgaGatcactAagcaggagaaGg attttctatggagtcacag  (SEQ ID NO:**32**) |

EP 3 910 066 A1

22

**Example 2: Extraction and purification of genomic DNA (gDNA) of the positive monoclonal cell strain**

**1. Extraction of gDNA**

The gDNA of the positive monoclonal cells was extracted and purified according to the instructions of QIAamp DNA Blood Mini Kit (Qiagen, 51104). Finally, according to the requirement of extracting the genome of the cell, the gDNA was eluted with a volume of 200 $\mu$L of Tris-EDTA (10 mM Tris-HCl, 1 mM EDTA, pH 8.1) and added to a 1.5 mL centrifuge tube for use.

**Table 8. Result of gDNA extraction**

| Cell type | gDNA concentration(ng/$\mu$L) | gDNA volume ($\mu$L) | 260/280 | 260/230 | gDNA yield ($\mu$g) |
|---|---|---|---|---|---|
| Positive monoclonal cell | 300.4 | 100 | 1.90 | 2.17 | 30.04 |

**Example 3. Determination of the molecule number of a mutant gene in standard cells by ddPCR**

ddPCR currently is generally accepted as the best method for determining the DNA molecule number . ddPCR was used in this example to determine the mutant gene molecule numbers of the two genomic standard DNAs of EGFR L858R (homozygous) (hereinafter referred to as: EGFR L858R) of HCT 116 cells and BRAF V600E (homozygous) (hereinafter referred to as: BRAF V600E) of HCT 116 cells derived from the homozygous standard cell strains.

**ddPCR detection for the molecule number of a mutant gene in a standard cell strain**

**1. Design the primers of EGFR L858R and BRAF V600E**

According to different gene-mutant gDNA standards, Taqman probes and corresponding upstream and downstream primers were designed at the position of base mutation in each standard, as shown in Table 9.

**Table 9. design of the primers of EGFR L858R and BRAF V600E**

| Primer name | Primer sequence |
|---|---|
| PrimerF(L858R) | 5'-GCAGCATGTCAAGATCACAGATT-3'(SEQ ID NO:33) |
| Primer R(L858R) | 5'-CCTCCTTCTGCATGGTATTCTTTCT-3'(SEQ ID NO:34) |
| Taqman probe (L858R) | FAM- AGTTTGGCCCGCCCAA-MGBNFQ(SEQ ID NO:35) |
| Primer F(V600E) | 5'-CTACTGTTTTCCTTTACTTACTACTACACCTCAGA-3'(SEQ ID NO:36) |
| Primer R(V600E) | 5'-ATCCAGACAACTGTTCAAACTGATG-3 '(SEQ ID NO:37) |
| Taqman probe(V600E) | FAM-TAGCTACAGAGAAATC-MGBNFQ(SEQ ID NO:38) |

**2. Genomic DNA extraction**

A certain number of ($10^5$-$10^6$) standard cells (EGFR L858R or BRAF V600E) were taken and gDNAs were extracted with a QIAGEN tissue DNA kit. The concentrations of gDNAs were measured with ThermoFisher Nanodrop 8000 UV spectrophotometer as the loading ranges of ddPCR method, and the sequences were diluted into three ddPCR test samples at 100 ng/$\mu$L, 10 ng/$\mu$L and 1 ng/$\mu$L.

**3. ddPCR test (Bio-rad QX200)**

(1) The reaction sample was prepared according to the ddPCR system of Table 10:

**Table 10. ddPCR reaction system**

| components | Volume | Final concentration |
|---|---|---|
| 2x supermix for probe (Bio-rad) | 10 µL | 1× |
| Primer F(L858R or V600E), 10µM | 1.8 µL | 900 nM |
| Primer R(L858R or V600E), 10µM | 1.8 µL | 900 nM |
| Taqman probe (L858R or V600E) | 1.25 µL | 250 nM |
| Sample to be tested (standard cell gDNA) | 1.0 µL | |
| ddH$_2$O | 4.15 µL | |
| Total volume | 20 µL | |

Microdroplets generation and ddPCR amplification were performed according to the instructions of ddPCR Supermix for probes kit (Bio-rad, 186-3010). The ddPCR amplification procedure is shown in Table 11 below.

**Table 11. The ddPCR amplification procedure is listed as follows:**

| Step | Temperature (°C) | Duration | Temperature change rate | Number of cycles |
|---|---|---|---|---|
| Enzyme activation | 95 °C | 10 min | | 1 |
| Denaturation | 94 °C | 30 sec | | 40 |
| Annealing/Extension | 60 °C | 1 min | 2 °C/sec | 40 |
| Inactivation of Enzyme | 98 °C | 10 min | | 1 |
| Heat Preservation (optional) | 4 °C | ∞ | | 1 |
| * The heating lid temperature was set to 105 °C, and the volume of the sample which had generated microdroplets was set to 40 µL. | | | | |

Then microdroplets detection was carried out.

### 4. Data Analysis

After the microdroplets detection was completed, the molecule number (molecule number/µL) of EGFR L858R or BRAF V600E in the ddPCR system was calculated based on the number of negative microdroplets in the FAM channel, as shown in Figure 15, and then was converted into the molecule number (molecule number/µL) of EGFR L858R or BRAF V600E in the gDNA standard.

**Quantifying the molecule numbers of EGFR L858R and BRAF V600E by ddPCR:**

From the results of ddPCR analysis, the loading range of gDNA standards of EGFR L858R and BRAF V600E (1ng-100ng) showed a good linear relationship with the measured molecule number of mutant genes, and the molecule number measured in the same sample also had good reproducibility. According to the absolute quantitative results of ddPCR, the molecule number of mutant genes in the EGFR L858R genomic DNA standard was 401±3 (molecule number/µL), and the molecule number of mutant genes in the BRAF V600E genomic DNA standard was 226±2 (µL).

### Example 4: Validation of the number of mutant molecules in cells by using NGS

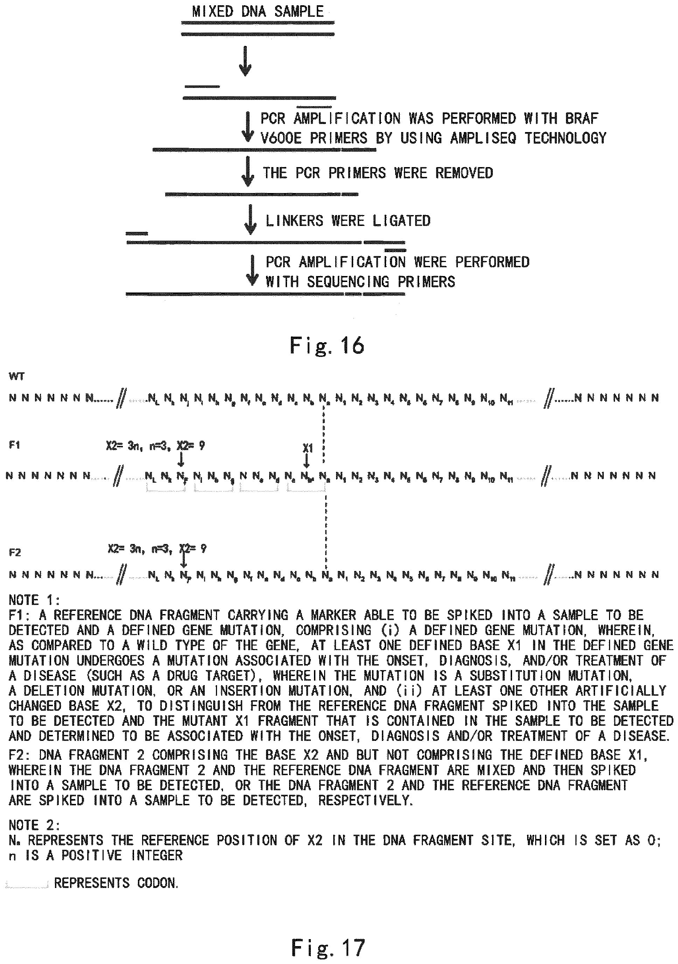

**A. The overall process is shown in Figure 16.**

**B. Amplification DNA targets**

1. According to the standard DNAs for spiked-in of the different molecular number of BRAF V600E (homozygous) of HCT 116 cells (or EGFR L858R (homozygous) of HCT 116 cell ) in Table 15, the DNA mixture of HCT 116 and HEK-293 (ATCC® CRL-1573™) and RKO (ATCC® CRL-2577™) (or HCT 116 and HEK-293 and NCI-H1957 (ATCC® CRL-5908™)) was added. 2 ng DNA of RKO BRAF V600E (or NCI-H1957 EGFR L858R) quantified by Qubit, and 8 ng DNA of HEK-293 were selected and mixed well, and then water was added to 10 μL for use.

2. Relevant operations were performed by reference to Illumina AmpliSeq™ Library PLUS (24 Reactions) of Illumina® (Catalog: 20019101) kit.

3. Design of the primers for AmpliSeq targets: primers were designed according to the website of custom Panel primer design in Illumina official website, as shown in Table 12.

**Table 12 design of primers for targets**

| Primer name | Primer sequence |
|---|---|
| μLSO(RKO BRAF V600E) | ACCTAAACTCTTCATAATGCTTGCT (SEQ ID NO:39) |
| DLSO(RKO BRAF V600E) | TTTCTAGTAACTCAGCAGCATCTCA (SEQ ID NO:40) |
| μLSO(NCI-H1957 EGFR L858R) | TGTTAAACAATACAGCTAGTGGGAA (SEQ ID NO:41) |
| DLSO(NCI-H1957 EGFR L858R) | GCAGCCAGGAACGTACTGGTGAAAA (SEQ ID NO:42) |

4. Construction of a library

After the primer design was completed, an experiment was performed by the Illumina kit of AmpliSeq™ Library PLUS (24 Reactions) for Illumina ® (Cat# 20019101). Adaptor was selected based on the following three groups (Table 13) to perform three parallel experiments, and the Index sequence was selected as in Table 13.

**Table 13. Index Sequence Selection**

| Repeat | Sequence |
|---|---|
| Repeat 1(A1) | Q5001: AGCGCTAG (SEQ ID NO:43) |
| RKO BRAF V600E | Q7005: GTGAATAT (SEQ ID NO:44) |
| Repeat 2(A2) | Q5002: GATATCGA (SEQ ID NO:45) |
| RKO BRAF V600E | Q7015: TCTCTACT (SEQ ID NO:46) |
| Repeat 3(A3) | Q5007: ACATAGCG (SEQ ID NO:47) |
| RKO BRAF V600E | Q7006: ACAGGCGC (SEQ ID NO:48) |
| Repeat 1(A1) | Q5001: AGCGCTAG (SEQ ID NO:43) |
| NCI-H1957 EGFR L858R | Q7005: GTGAATAT (SEQ ID NO:44) |
| Repeat 2(A4) | Q5008: GTGCGATA (SEQ ID NO:49) |
| NCI-H1957 EGFR L858R | Q7007: ATAGAGT (SEQ ID NO:50) |
| Repeat 3(A5) | Q5009: CCAACAGA (SEQ ID NO:51) |
| NCI-H1957 EGFR L858R | Q7016: CTCTCGTC (SEQ ID NO:52) |

**II. Confirmation of the number of mutant molecules of V600E in the RKO cell sample to be detected (or L858R in NCI-H1957 cells)**

1. Confirmation of the number of V600E mutant molecules in RKO cells: the cell type, mutation site and DNA sequence information used in this experiment are shown in Table 14.

Table 14. Cell type, mutation site and DNA sequence information

| Cell type | Mutation site information | DNA sequence |
|---|---|---|
| spiked-in standard1 ( HCT 116 cells with homozygous BRAF V600E ) | 597L(CT*A* was mutated into CT*T*), V600E(G*T*G was mutated into G*A*G ), | //-GGTCTTGCTACAGAGAAATCTCGTTGG-// (SEQ ID NO:53) |
| | 603R(CG*A* was mutated into CG*T*) | //-GGTCTTGCTACAGAGAAATCTCGTTGG-// (SEQ ID NO:54) |
| spiked-in standard2 ( HCT 116 cells with homozygous BRAF 597L, 603R) | 597L(CT*A* was mutated into CT*T*), | //-GGTCTTGCTACAGTGAAATCTCGTTGG-// (SEQ ID NO:55) |
| | 603R(CG*A* was mutated into CG*T*) | //-GGTCTTGCTACAGTGAAATCTCGTTGG-// (SEQ ID NO:56) |
| Sample to be detected ( RKO cells with heterozygous BRAF V600E ) | V600E(G*T*G was mutated into G*A*G) | //-GGTCTAGCTACAGAGAAATCTCGATGG-// (SEQ ID NO:57) |
| | | //-GGTCTAGCTACAGTGAAATCTCGATGG-// (SEQ ID NO:58) |
| Other cell background sample to be detected(HEK-293 cells) | | //-GGTCTAGCTACAGTGAAATCTCGATGG-// (SEQ ID NO:59) |
| | | //-GGTCTAGCTACAGTGAAATCTCGATGG-// (SEQ ID NO:60) |

Confirmation of the number of L858R mutant molecules in NCI-H1957 cells: the cell type, mutation site and DNA sequence information used in this experiment are shown in Table 15.

Table 15. Cell type, mutation site and DNA sequence information

| Cell type | Mutation site information | DNA sequence |
|---|---|---|
| spiked-in standard 1 ( HCT 116 cells with homozygous EGFR L858R ) | 855D(GA*T* was mutated into GA*C*), L858R(C*T*G was mutated into C*G*G ), | //-ACAGACTTTGGGC*G*GGCCAAACTCCTG-// (SEQ ID **NO:61**) |
| | | //-ACAGACTTTGGGCGGGCCAAACTCCTG-// (SEQ ID **NO:62**) |
| | 861L(CT*G* was mutated into CT*C*) | |

(continued)

| Cell type | Mutation site information | DNA sequence |
|---|---|---|
| spiked-in standard 2 ( HCT 116 cells with homozygous EGFR 855D, 861L) | 855D(GA*T* was mutated into GA*C*), | //-ACAGA**C**TTTGGGCTGGCCAAACT**C**CTG-// (SEQ ID **NO:63**) |
| | 861L(CT*G* was mutated into CT*C*) | //-ACAGA**C**TTTGGGCTGGCCAAACT**C**CTG-// (SEQ ID **NO:64**) |
| Sample to be detected (NCI-H1957 cells with heterozygous EGFR L858R) | L858R(C*T*G was mutated into C*G*G ) | //-ACAGATTTTGGGC**G**GGCCAAACTGCTG-// (SEQ ID **NO:65**) //-ACAGATTTTGGGCTGGCCAAACTGCTG-// (SEQ ID NO:66) |
| Other cell background sample to be detected(HEK-293 cells) | | //-ACAGATTTTGGGCTGGCCAAACTGCTG-// (SEQ ID **NO:67**) //-ACAGATTTTGGGCTGGCCAAACTGCTG-// (SEQ ID **NO:68**) |

2. 2 ng of genomic DNA of RKO cells (or NCI-H1957 cells) sample to be detected and 8 ng of genomic DNA of HEK-293 cells which mimic other cell background sample to be detected precisely quantified by Qubit, were selected, and at the meanwhile the two different kinds of genomic DNA molecules, spiked-in standard 1 and spiked-in standard 2, whose molecule numbers had been accurately determined by ddPCR, were added, as shown in Table 16 below.

**Table 16. Number of spiked-in molecules in experiments 1-3**

| | Genomic type | Number of the spiked-in molecules | The DNA quality of the sample to be detected (ng) |
|---|---|---|---|
| **Experiment 1** | spiked-in standard 1 | 900 | |
| | spiked-in standard 2 | 2100 | |
| | Sample to be detected (RKO cells with heterozygous BRAF V600E or NCI-H1957 cells with heterozygous EGFR L858R ) | | 2 |
| | Other cell background sample to be detected (HEK-293 cells) | | 8 |
| | | | |
| **Experiment 2** | spiked-in standard 1 | 300 | |
| | spiked-in standard 2 | 2700 | |
| | Sample to be detected (RKO cells with heterozygous BRAF V600E or NCI-H1957 cells with heterozygous EGFR L858R ) | | 2 |
| | Other cell background sample to be detected (HEK-293 cells) | | 8 |

(continued)

| | Genomic type | Number of the spiked-in molecules | The DNA quality of the sample to be detected (ng) |
|---|---|---|---|
| | | | |
| Experiment 3 | spiked-in standard 1 | 90 | |
| | spiked-in standard 2 | 2910 | |
| | Sample to be detected (RKO cells with heterozygous BRAF V600E or NCI-H1957 cells with heterozygous EGFR L858R ) | | 2 |
| | Other cell background sample to be detected (HEK-293 cells) | | 8 |

Each experiment was carried out in triplicate, and an AmpliSeq™ Library PLUS (24 Reactions) kit for Illumina ® (Cat# 20019101) from Illumina was used to build a library for sequencing with a $50\times$ sequencing depth to obtain data as show in Table 17 (for RKO cells BRAF V600E) and Table 18 (for NCI-H1957 cells EGFR L858R cells):

**Table 17 Reads number in experiments 1-3 (for RKO cells BRAF V600E)**

| | Genomic type | Reads number of replicate 1 | Reads number of replicate 2 | Reads number of replicate 3 | mean of Reads |
|---|---|---|---|---|---|
| Experiment 1 | spiked-in standard 1 | 48,645,820 | 49,159,636 | 49,862,842 | 49,222,766 |
| | spiked-in standard 2 | 116,950,524 | 117,436,138 | 115,583,846 | 116,656,836 |
| | V600E mutation in the sample to be detected | 16,614,021 | 16,057,564 | 15,976,802 | 16,216,129 |
| | | | | | |
| Experiment 2 | spiked-in standard 1 | 16,018,943 | 16,901,377 | 16,495,569 | 16,471,963 |
| | spiked-in standard 2 | 148,816,835 | 146,636,027 | 151,555,625 | 149,002,829 |
| | V600E mutation in the sample to be detected | 15,541,011 | 15,964,369 | 16,501,004 | 16,002,128 |
| | | | | | |
| Experiment 3 | spiked-in standard 1 | 4,859,682 | 4,914,196 | 4,980,918 | 4,918,265 |
| | spiked-in standard 2 | 161,731,772 | 160,732,199 | 161,026,689 | 161,163,553 |
| | V600E mutation in the sample to be detected | 16,087,936 | 16,198,394 | 15,972,579 | 16,086,303 |

**Table 18 Reads number of experiments 1-3 (for NCI-H1957 cells EGFR L858R)**

| | Genomic type | Reads number of replicate 1 | Reads number of replicate 2 | Reads number of replicate 3 | mean of Reads |
|---|---|---|---|---|---|
| Experiment 1 | spiked-in standard 1 | 49,785,692 | 49,878,296 | 49,890,660 | 49,851,549 |
| | spiked-in standard 2 | 117,150,522 | 116,746,127 | 117,057,787 | 116,984,812 |
| | L858R mutation in the sample to be detected | 16,089,264 | 15,996,582 | 15,941,223 | 16,009,023 |
| | | | | | |
| Experiment 2 | spiked-in standard 1 | 16,721,903 | 17,534,289 | 17,080,783 | 17,112,325 |
| | spiked-in standard 2 | 150,873,987 | 151,924,869 | 151,598,985 | 151,465,947 |
| | L858R mutation in the sample to be detected | 15,684,684 | 15,789,420 | 16,532,603 | 16,002,236 |
| | | | | | |
| Experiment 3 | spiked-in standard 1 | 4,969,782 | 4,973,968 | 4,986,961 | 4,976,904 |
| | spiked-in standard 2 | 161,950,796 | 160,809,236 | 161,871,672 | 161,543,901 |
| | L858R mutation in the sample to be detected | 16,125,864 | 16,005,685 | 15,905,141 | 16,012,230 |

The number of V600E mutant molecules in the RKO cells sample to be detected in the three experiments can be calculated according to the following equations, as shown in Table 19:

$$\text{The number of RKO V600E mutant molecules in the sample to be detected} = \frac{\text{Reads number of V600E in the sample to be detected}}{\text{Reads number of spiked in standard 1}} \times \text{Number of spiked in molecules of spiked in standard 1}$$

OR

$$\text{The number of RKO V600E mutant molecules in the sample to be detected} = \frac{\text{Reads number of V600E in the sample to be detected}}{\text{Reads number of spiked in standard 2}} \times \text{Number of spiked in molecules of spiked in standard 2}$$

The number of L858R mutant molecules in the NCI-H1957 cells sample to be detected in the three experiments can be calculated according to the following equations, as shown in Table 20:

$$\text{The number of NCI-H1957 L858R mutant molecules in the sample to be detected} = \frac{\text{Reads number of L858R in the sample to be detected}}{\text{Reads number of spiked in standard 1}} \times \text{Number of spiked in molecules of spiked in standard 1}$$

OR

$$\text{The number of NCI-H1957 L858R mutant molecules in the sample to be detected} = \frac{\text{Reads number of L858R in the sample to be detected}}{\text{Reads number of spiked in standard 2}} \times \text{Number of spiked in molecules of spiked in standard 2}$$

Table 21. Confirmation of the number of V600E mutant molecules in the RKO cell samples to be detected

|  | mean of reads of V600E mutant molecule in the sample to be detected | mean of reads of spiked-in standard 1 | mean of reads of spiked-in standard 2 | Number of spiked-in molecules of spiked-in standard 1 | Number of spiked-in molecules of spiked-in standard 2 | The number of V600E mutant molecules in the sample to be detected |
|---|---|---|---|---|---|---|
| Experiment 1 | 16,216,129 | 49,222,766 |  | 900 |  | 297 |
|  |  |  | 116,656,836 |  | 2100 | 292 |
| Experiment 2 | 16,002,128 | 16,471,963 |  | 300 |  | 291 |
|  |  |  | 149,002,829 |  | 2700 | 290 |
| Experiment 3 | 16,086,303 | 4,918,265 |  | 90 |  | 294 |
|  |  |  | 161,163,553 |  | 2910 | 290 |

Table 22. Statistic Analysis of the number of V600E mutant molecules in RKO cell samples to be detected

| Variance Analysis | | | | | | |
|---|---|---|---|---|---|---|
| SUMMARY | | | | | | |
| Group | Number of observations | Sum | mean | variance | | |
| a1/900 | 3 | 889.727859 | 296.575953 | 95.3513182 | | |
| a1/2100 | 3 | 875.7454306 | 291.9151435 | 33.28533031 | | |
| a2/300 | 3 | 874.5162938 | 291.5054313 | 70.13563929 | | |
| a2/2300 | 3 | 869.8825437 | 289.9608479 | 47.9832561 | | |

(continued)

| Variance Analysis | | | | | | |
|---|---|---|---|---|---|---|
| **SUMMARY** | | | | | | |
| **Group** | **Number of observations** | **Sum** | **mean** | **variance** | | |
| a3/90 | 3 | 883.214151 | 294.404717 | 25.61361948 | | |
| a3/2910 | 3 | 871.3815845 | 290.4605282 | 6.07093936 | | |
| | | | | | | |
| Variance Analysis | | | | | | |
| **Source of difference** | **SS** | **df** | **MS** | **F** | **P-value** | **F crit** |
| Intergroup | 96.52217545 | 5 | 19.30443509 | 0.415983938 | 0.828840879 | 3.105875239 |
| Intragroup | 556.8802055 | 12 | 46.40668379 | | | |
| | | | | | | |
| Total | 653.4023809 | 17 | | | | |

**Table 23. Confirmation of the number of L858R mutant molecules in NCI-H1957 cell samples to be detected**

| | mean of reads of L858R mutant molecule in the sample to be detected | mean of reads of spiked-in standard 1 | mean of reads of spiked-in standard 2 | Number of spiked-in molecules of spiked-in standard 1 | Number of spiked-in molecules of spiked-in standard 2 | The number of L858R mutant molecules in the sample to be detected |
|---|---|---|---|---|---|---|
| Experiment 1 | | 49,851,549 | | 900 | | 289 |
| | 16,009,023 | | 116,984,812 | | 2100 | 287 |
| Experiment 2 | | 17,112,325 | | 300 | | 281 |
| | 16,002,236 | | 151,465,947 | | 2700 | 285 |
| Experiment 3 | | 4,976,904 | | 90 | | 290 |
| | 16,012,230 | | 161,543,901 | | 2910 | 288 |

**Table 24. Statistical analysis of the number of L858R mutant molecules in HCT-116 cell sample to be detected**

| Variance Analysis | | | | | | |
|---|---|---|---|---|---|---|
| **SUMMARY** | | | | | | |
| **Group** | **Number of observations** | **Sum** | **mean** | **variance** | | |
| **a1/900** | 3 | 867.0653216 | 289.0217739 | 2.802450197 | | |
| **a1/2100** | 3 | 862.136382 | 287.378794 | 1.572218653 | | |
| **a2/300** | 3 | 841.9101704 | 280.6367235 | 102.6955701 | | |
| **a2/2300** | 3 | 855.7455836 | 285.2485279 | 63.47524888 | | |
| **a3/90** | 3 | 868.6817074 | 289.5605691 | 6.225331329 | | |
| **a3/2910** | 3 | 865.3247467 | 288.4415822 | 4.734664226 | | |
| | | | | | | |

(continued)

| Variance Analysis | | | | | | |
|---|---|---|---|---|---|---|
| Source of difference | SS | df | MS | F | P-value | F crit |
| Intergroup | 167.8084907 | 5 | 33.56169813 | 1.109444106 | 0.405336694 | 3.105875239 |
| Intragroup | 363.0109667 | 12 | 30.25091389 | | | |
| | | | | | | |
| Total | 530.8194574 | 17 | | | | |

By spiked-in standard 1 and spiked-in standard 2, the number of mutant molecules contained in the genomic DNA of 2 ng of sample to be detected: RKO cells (or NCI-H1957 cells) could be calculated out, and the values were very close and highly reliable.

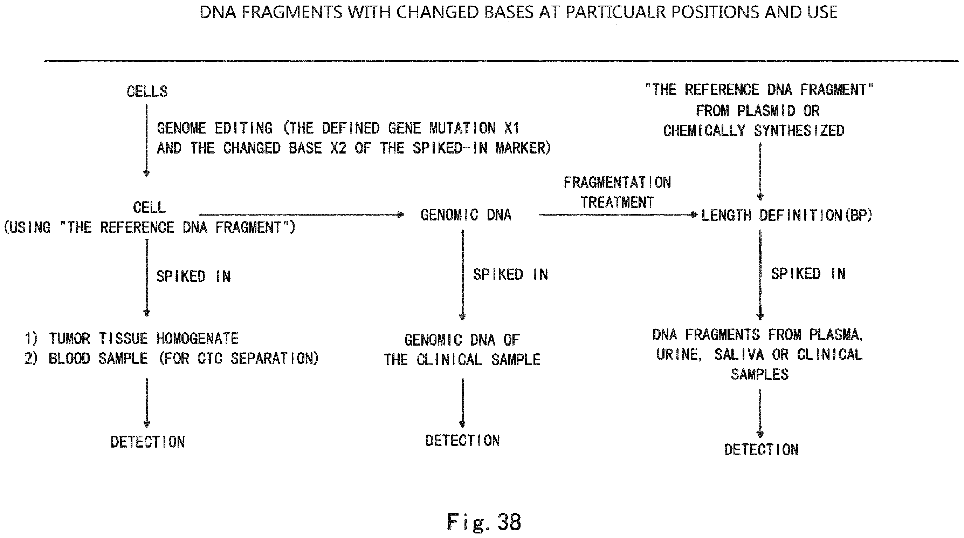

Based on the above experiments, the core strategy of the present invention is shown in FIG. 38.

**Table 25. List of examples of long-fragment reference DNA (construction of spiked-in markers according to X2=3n and/or X2=n respectively)**

| Gene name | GeneBank number of wild-type gene | Defined mutation (substitutio n) base X1 | Spiked-in marker base X2 (X2 is located in the Exon sequence) | Spiked-in marker base X2 (X2 is located in the Intron sequence ) | Exon sequence and Intron sequence of the reference DNA fragment carrying a defined mutation and a marker able to be spiked in |
|---|---|---|---|---|---|
| EGFR | Gene ID: 1956 | T790M (ACG was mutated into ATG) | Located upstream of X1, 787Q ( CA**G** to CA**A**); Located downstream of X1, 792L (CT**C** was mutated into CT**T**) | Located at 500bp upstream of X1 (**C** to **A**); Located at 500bp downstream of X1 (**A** to **C**); | ctgatgtgcagggtcagtcattacccagggtgtt**A**cggaccccacaca gattcctacaggccctcatgatattttaaaacacagcatcctcaaccttga ggcggaggtcttcataacaaagatactatcagttcccaaactcagagatc aggtgactccgactcctcctttatccaatgtgctcctcatggccactgttgc ctgggcctctctgtcatggggaatccccagatgcacccaggaggggcc ccctcccactgcatctgtcacttcacagccctgcgtaaacgtccctgtgct aggtcttttgcaggcacagcttttcctccatgagtacgtattttgaaactcaa gatcgcattcatgcgtcttcacctggaaggggtccatgtgcccctccttct ggccaccatgcgaagccacactgacgtgcctctccctccctccaggaa gcctacgtgatggccagcgtggacaaccccacgtgtgccgcctgctg ggcatctgcctcacctccaccgtgca**A**ctcatca**T**gcagct**T**atgccc ttcggctgcctcctggactatgtccgggaacacaaagacaatattggctc ccagtacctgctcaactggtgtgtgcagatcgcaaaggtaatcagggaa gggagatacggggaggggagataaggagccaggatcctcacatgcg |

| Gene name | GeneBank number of wild-type gene | Defined mutation (substitutio n) base X1 | Spiked-in marker base X2 (X2 is located in the Exon sequence) | Spiked-in marker base X2 (X2 is located in the Intron sequence ) | Exon sequence and Intron sequence of the reference DNA fragment carrying a defined mutation and a marker able to be spiked in |
|---|---|---|---|---|---|
| | | | | | gtctgcgctcctgggatagcaagagtttgccatgggggatatgtgtgtgcg tgcatgcagcacacacacattcctttattttggattcaatcaagttgatcttct tgtgcacaaatcagtgcctgtcccatctgcatgtggaaactctcatcaatc agctacctttgaagaattttctctttattgagtgctcagtgtggtctgatgtct ctgttcttatttctctggaattctttgtgaatactgtggtgatttgtagtggaga aggaatattgcttcccccattcaggacttgataacaaggtaagcaagcca ggccaaggccaggaggacccaggtgat**C**gtggtggagtggagcagg tgccttgcaggag (SEQ ID NO:69) |

EP 3 910 066 A1

34

| Gene name | GeneBank number of wild-type gene | Defined mutation (substitutio n) base X1 | Spiked-in marker base X2 (X2 is located in the Exon sequence) | Spiked-in marker base X2 (X2 is located in the Intron sequence ) | Exon sequence and Intron sequence of the reference DNA fragment carrying a defined mutation and a marker able to be spiked in |
|---|---|---|---|---|---|
| EGFR | Gene ID: 1956 | T790M (ACG was mutated into A*T*G) | / | Located at 500bp upstream of X1 (C to A); Located at 500bp downstream of X1 (A to C) ; | ctgatgtgcagggtcagtcattacccagggtgtt**A**cggaccccacaca gattcctacaggccctcatgatattttaaaacacagcatcctcaaccttga ggcggaggtcttcataacaaagatactatcagttcccaaactcagagatc aggtgactccgactcctcctttatccaatgtgctcctcatggccactgttgc ctgggcctctctgtcatggggaatccccagatgcacccaggaggggcc ccctcccactgcatctgtcacttcacagccctgcgtaaacgtccctgtgct aggtcttttgcaggcacagcttttcctccatgagtacgtattttgaaactcaa gatcgcattcatgcgtcttcacctggaaggggtccatgtgcccctccttct ggccaccatgcgaagccacactgacgtgcctctccctccctccaggaa gcctacgtgatggccagcgtggacaaccccacgtgtgccgcctgctg ggcatctgcctcacctccaccgtgcagctcatca**T**gcagctcatgccctt |

| Gene name | GeneBank number of wild-type gene | Defined mutation (substitutio n) base X1 | Spiked-in marker base X2 (X2 is located in the Exon sequence) | Spiked-in marker base X2 (X2 is located in the Intron sequence ) | Exon sequence and Intron sequence of the reference DNA fragment carrying a defined mutation and a marker able to be spiked in |
|---|---|---|---|---|---|
| | | | | | cggctgcctcctggactatgtccgggaacacaaagacaatattggctcc cagtacctgctcaactggtgtgtgcagatcgcaaaggtaatcagggaag ggagatacggggaggggagataaggagccaggatcctcacatgcggt ctgcgctcctgggatagcaagagtttgccatggggatatgtgtgtgcgtg catgcagcacacacacattcctttattttggattcaatcaagttgatcttcttg tgcacaaatcagtgcctgtcccatctgcatgtggaaactctcatcaatcag ctacctttgaagaattttctctttattgagtgctcagtgtggtctgatgtctctg ttcttatttctctggaattctttgtgaatactgtggtgatttgtagtggagaag gaatattgcttcccccattcaggacttgataacaaggtaagcaagccagg ccaaggccaggaggacccaggtgat**C**gtggtggagtggagcaggtg ccttgcaggag(SEQ ID NO:70) |

36

EP 3 910 066 A1

(continued)

| Gene name | GeneBank number of wild-type gene | Defined mutation (substitutio n) base X1 | Spiked-in marker base X2 (X2 is located in the Exon sequence) | Spiked-in marker base X2 (X2 is located in the Intron sequence ) | Exon sequence and Intron sequence of the reference DNA fragment carrying a defined mutation and a marker able to be spiked in |
|---|---|---|---|---|---|
| BRAF | Gene ID:673 | V600E (GTG was mutated into GAG) | Located upstream, 597L (CTA was mutated into CTT); Located downstream, 603R (CGA was mutated into CG*T*) | Located at 500bp upstream of X1 (G to *T*); Located at 500bp downstream of X1 (*T* to *G*); | gtagttgagatataactgaa**T**actctaaattatataacaatgaggtgagaa aaacataatatttctcttccctaagtgcagactaagatactatctgcagcat cttcattccaatgaagagcctttactgctcgcccaggagtgccaagagaa tatctgggcctacattgctaaaatctaatgggaaagtttttaggttctcctata aacttaggaaagcatctcacctcatcctaacacatttcaagccccaaaaat cttaaaagcaggttatataggctaaatagaactaatcattgttttagacata cttattgactctaagaggaaagatgaagtactatgttttaaagaatattatat tacagaattatagaaattagatctcttacctaaactcttcataatgcttgctct |

| Gene name | GeneBank number of wild-type gene | Defined mutation (substitutio n) base X1 | Spiked-in marker base X2 (X2 is located in the Exon sequence) | Spiked-in marker base X2 (X2 is located in the Intron sequence ) | Exon sequence and Intron sequence of the reference DNA fragment carrying a defined mutation and a marker able to be spiked in |
|---|---|---|---|---|---|
|  |  |  |  |  | gataggaaaatgagatctactgttttcctttacttactacacctcagatatatt tcttcatgaagacctcacagtaaaaataggtgattttggtct**T**gctacag **A**gaaatctcg**T**tggagtgggtcccatcagtttgaacagttgtctggatc cattttgtggatggtaagaattgaggctattttttccactgattaaattttttggc cctgagatgctgctgagttactagaaagtcattgaaggtctcaactatagt attttcatagttcccagtattcacaaaaatcagtgttcttattttttatgtaaata gattttttaactttttttctttacccttaaaacgaatattttgaaaccagtttcagt gtatttcaaacaaaaatatatgtcttataaacagtgtttcatattttattcttaaa taaatatgaacccttaaaacgaatattttgaaaccagtttcagtgtatttcaa acaaaaatatatgtcttataaacagtgtttcatattttattctaaattgtttaaa gtattttgtgttcaaaatgttctgtgtaccctgttgaaaaaaaaaaacaggtat gcaatttaaggcaggtg**G**gatccacagccattattatggttttgctaag( SEQ ID NO:71) |

| Gene name | GeneBank number of wild-type gene | Defined mutation (substitutio n) base X1 | Spiked-in marker base X2 (X2 is located in the Exon sequence) | Spiked-in marker base X2 (X2 is located in the Intron sequence ) | Exon sequence and Intron sequence of the reference DNA fragment carrying a defined mutation and a marker able to be spiked in |
|---|---|---|---|---|---|
| BRAF | Gene ID:673 | V600E (GTG was mutated into GAG) | / | Located at 500bp upstream of X1 (G to *T*) ; Located at 500bp downstream of X1 (*T* to *G*) ; | gtagttgagatataactgaa<u>T</u>actctaaattatataacaatgaggtgagaa aaacataatatttctcttccctaagtgcagactaagatactatctgcagcat cttcattccaatgaagagcctttactgctcgcccaggagtgccaagagaa tatctgggcctacattgctaaaatctaatgggaaagtttaggttctcctata aacttaggaaagcatctcacctcatcctaacacatttcaagccccaaaaat cttaaaagcaggttatataggctaaatagaactaatcattgttttagacata cttattgactctaagaggaaagatgaagtactatgtttttaaagaatattatat |

EP 3 910 066 A1

(continued)

| Gene name | GeneBank number of wild-type gene | Defined mutation (substitutio n) base X1 | Spiked-in marker base X2 (X2 is located in the Exon sequence) | Spiked-in marker base X2 (X2 is located in the Intron sequence ) | Exon sequence and Intron sequence of the reference DNA fragment carrying a defined mutation and a marker able to be spiked in |
|---|---|---|---|---|---|
| EP 3 910 066 A1 | | | | | tacagaattatagaaattagatctcttacctaaactcttcataatgcttgctct gataggaaaatgagatctactgttttcctttacttactacacctcagatatatt tcttcatgaagacctcacagtaaaaataggtgattttggtctagctacag**A** gaaatctcgatggagtgggtcccatcagtttgaacagttgtctggatccat tttgtggatggtaagaattgaggctatttttccactgattaaattttttggccct gagatgctgctgagttactagaaagtcattgaaggtctcaactatagtattt tcatagttcccagtattcacaaaaatcagtgttcttatttttttatgtaaatagat tttttaactttttttctttacccttaaaacgaatattttgaaaccagtttcagtgtat ttcaaacaaaaatatatgtcttataaacagtgtttcatattttattcttaaataa atatgaacccttaaaacgaatattttgaaaccagtttcagtgtatttcaaaca aaaatatatgtcttataaacagtgtttcatattttattctaaattgtttaaagtatt ttgtgttcaaaatgttctgtgtaccctgttgaaaaaaaaaaacaggtatgcaa tttaaggcaggtg**G**gatccacagccattattatggttttgctaag(SEQ ID NO:72) |

In addition, although all DNA fragments of the examples disclosed in the present invention have only one well-defined mutation, and each of the upstream and downstream of the mutation site has only one marker able to be spiked into the sample to be detected, this is intended as examples for introducing the construction method and process in detail. During the specific operation, at least one mutation and at least one marker able to be spiked into the sample to be detected can be constructed upon experimental requirements, and are not limited to the mutations and the number of markers of the examples.

The above examples only describe several embodiments of the present invention more specifically and in detail, but they should not be construed as limiting the scope of the invention. It should be noted that a number of variations and modifications may be made by those skilled in the art without departing from the spirit and scope of the invention. Therefore, the scope of the invention should be defined by the claims.

**Claims**

1. A reference DNA, selected from the group consisting of:

   (i) DNA fragment 1: **characterized in that** it carries a defined gene mutation and at least one another artificially altered base X2, wherein, as compared to a wild type of the gene, at least one defined base X1 in the defined gene mutation undergoes a mutation associated with the occurrence, diagnosis, and/or treatment (such as a target targeted by a medicament) of a disease (such as a tumor), wherein the mutation is a substitution mutation, a deletion mutation, and/or an insertion mutation, and the artificial altered base X2 is different from the mutant base X1 which is contained in a sample to be detected and defined to be associated with the occurrence, diagnosis and/or treatment of a disease,
   (ii) DNA fragment 2: **characterized in that** the DNA fragment 2 comprises the artificial altered base X2 in (i), and it differs from the DNA fragment 1 only **in that** it does not comprise the defined base X1 mutation, or
   (iii) a mixture of the DNA fragment 1 and the DNA fragment 2.

2. The reference DNA according to claim 1, wherein the base X2 is located at any position upstream, downstream or both of the defined base X1.

3. The reference DNA according to claim 1, wherein the DNA fragment 1 and the DNA fragment 2 are double stranded DNAs.

4. The reference DNA according to claim 1, **characterized in that** when the mutation in (i) is a substitution mutation, the interval between the defined base X1 and the base X2 is 1 bp, 2 bp, 3 bp, 4 bp, 5 bp, 10 bp or less, 100 bp or less, 500 bp or less, 1 kb or less, 2 kb or less, 10 kb or less, or 100 kb or less,
   preferably,

   (a) when the position of the third base in the codon comprising the defined base X1 mutation is set as 0, and the base X2 is located upstream of the defined base X1, the position of the base X2 is represented by 3n, wherein n is a positive integer, preferably, the altering of the base X2 does not cause any change to the original amino acid coded, or
   (b) when the position of the third base in the codon comprising the defined base X1 mutation is set as 0, and the base X2 is located downstream of the defined base X1, the position of the base X2 is represented by -3n, wherein n is a positive integer, preferably, the altering of the base X2 does not cause any change to the original amino acid coded; or
   (c) when the position of the third base in the codon comprising the defined base X1 mutation is set as 0, and the base X2 is located upstream and downstream of the defined base X1, respectively, the position of the base X2 located upstream of the defined base X1 is represented by 3n, and the position of the base X2 located downstream of the defined base X1 is represented by -3n, wherein both of n are positive integers, preferably, the altering of the base X2 does not cause any change to the original amino acid coded.

5. The reference DNA according to claim 4, **characterized in that** the mutation in (i) is a consecutive substitution or a discrete substitution, preferably a substitution mutations in the first and the second consecutive bases of the same codon.

6. The reference DNA according to claim 1, **characterized in that** when the mutation in (i) is a deletion mutation, the interval between the defined base X1 and the base X2 is 1 bp, 2 bp, 3 bp, 4 bp, 5 bp, 10 bp or less, 100 bp or less, 500 bp or less, 1 kb or less, 2 kb or less, 10 kb or less, or 100 kb or less,
preferably,

(a) when as compared to a wild type of the gene, one of the defined base X1 is deleted at one base position, or multiple defined base X1s are deleted consecutively at multiple base positions, and the base X2 is located upstream of the deleted defined base X1, the position of the third base of a codon immediately adjacent to the upstream of the defined base X1 and corresponding to the first codon of the wide type of the gene is set as 0, the position of the base X2 is represented by 3n, wherein n is a positive integer, preferably, the altering of the base X2 does not cause any change to the original amino acid coded, or

(b) when as compared to a wild type of the gene, one of the defined base X1 is deleted at one base position, or multiple defined base X1s are deleted consecutively at multiple base positions, and the base X2 is located downstream of the defined base X1 deleted, the base X2 is located at any position downstream of the defined base X1;

(c) when as compared to a wild type of the gene, one of the defined base X1 is deleted at one base position, or multiple defined base X1s are deleted consecutively at multiple base positions, and the base X2s are located upstream and downstream of the defined base X1, when the base X2 is located upstream of the base X1, the definition of the base X2 is described in (a), and when the base X2 is located downstream of the defined base X1, the definition of the base X2 is described in (b), preferably, the altering of the base X2 does not cause any change to the original amino acid coded.

7. The reference DNA of claim 6, **characterized in that** in the conditions of (a)-(c), the deletion is a consecutive deletion or a discrete deletion.

8. The reference DNA according to claim 1, **characterized in that** when the mutation in (i) is an insertion mutation, the interval between the defined base X1 and the base X2 is 1 bp, 2 bp, 3 bp, 4 bp, 5 bp, 10 bp or less, 100 bp or less, 500 bp or less, 1 kb or less, 2 kb or less, 10 kb or less, or 100 kb or less,
preferably,

(a) when as compared to a wild type of the gene, one of the defined base X1 is inserted between two bases, or multiple defined base X1s are consecutively inserted between two bases, and the base X2 is located upstream of the inserted defined base X1, the position of the third base of a codon immediately adjacent to the upstream of the defined base X1 and corresponding to the first codon of the wide type of the gene is set as 0, the position of the base X2 is represented by 3n, wherein n is a positive integer, preferably, the altering of the base X2 does not cause any change to the original amino acid coded,

(b) when as compared to a wild type of the gene, one of the defined base X1 is inserted between two bases, or multiple defined base X1s are consecutively inserted between two bases, and the base X2 is located downstream of the defined base X1, the base X2 is located at any position downstream of the defined base X1; or

(c) when as compared to a wild type of the gene, one of the defined base X1 is inserted between two bases, or multiple defined base X1s are consecutively inserted between two bases, and the base X2s are located upstream and downstream of the inserted defined base X1, respectively, when the base X2 is located upstream of the base X1, the definition of the base X2 is described in (a), and when the base X2 is located downstream of the base X1, the definition of the base X2 is described in (b), preferably, the altering of the base X2 does not cause any change to the original amino acid coded.

9. The reference DNA according to claim 8, **characterized in that** in the conditions of (a)-(c), the insertion is a consecutive insertion or a discrete insertion.

10. The reference DNA according to claim 5, **characterized in that**, the substitution mutation in (i) is m discrete substitution mutations, wherein the m is a integer of 2 or more, and when the distance between each two mutations is 10 bp, 10-20 bp, 10-30 bp, 10-40 bp, 10-50 bp, 10-60 bp, 10-70 bp or 10-80 bp, the artificial altered base X2 in (ii) is formed simultaneously upstream and downstream of the base X1.

11. The reference DNA according to claim 7, **characterized in that**, the deletion mutation in (i) is m discrete deletion mutations, wherein the m is a integer of 2 or more, and when the distance between each two mutations is 10 bp, 10-20 bp, 10-30 bp, 10-40 bp, 10-50 bp, 10-60 bp, 10-70 bp or 10-80 bp, the artificial altered base X2 in (ii) is formed simultaneously upstream and downstream of the base X1.

12. The reference DNA according to claim 9, **characterized in that**, the insertion mutation in (i) is m discrete insertion mutations, wherein the m is a integer of 2 or more, and when the distance between each two mutations is 10 bp, 10-20 bp, 10-30 bp, 10-40 bp, 10-50 bp, 10-60 bp, 10-70 bp or 10-80 bp, the artificial altered base X2 in (ii) is simultaneously formed upstream and downstream of the base X1.

13. Reference DNA according to claim 1, **characterized in that** the gene comprising a defined mutant base X1 associated with the occurrence, diagnosis, and/or treatment (such as a target targeted by a medicament) of a disease (such as a tumor) includes, but not limited to, EGFR, KRAS, BRAF, P53, Met, PTEN, ROS1, NRAS, PIK3CA, RET, HER2, CMET, FGFR1 and/or DDR2.

14. Reference DNA according to claim 13, **characterized in that** the position of the amino acid encoded by the codon comprising the defined base X1 mutation includes, but not limited to, amino acid position 858, 790, 768, 746, 747, 748, 749, 750, 719 and/or 797 of EGFR, amino acid position 12 and/or 13 of KRAS, amino acid position 12, 13 and/or 600 of BRAF, amino acid position 12, 59 and/or 61 of NRAS, amino acid position 880 and/or 837 of HER2, amino acid position 816 of cKIT, and amino acid position 545 and/or 1047 of PIK3CA, wherein the position is calculated by taking the position of the amino acid encoded by the start codon as 1.

15. Reference DNA according to claim 14, **characterized in that** there are deletion mutations in EGFR amino acid positions 746, 747, 748, 749, 750; a mutation of substituting arginine R for leucine L at amino acid position 858 of EGFR; a mutation of substituting serine S for cysteine C at amino acid position 797 of EGFR; a mutation of substituting serine S for glycine G at amino acid position 719 of EGFR; a mutation of substituting methionine M for threonine T at amino acid position 790 of EGFR; a mutation of substituting isoleucine I for serine S at amino acid position 768 of EGFR; a mutation of substituting glutamic acid E for valine V at amino acid position 600 of BRAF; a mutation of substituting cysteine C for glycine G at amino acid position 12 of BRAF; a mutation of substituting cysteine C for glycine G at amino acid position 13 of BARF; a mutation of substituting aspartic acid D for glycine G at amino acid position 13 of KRAS; a mutation of substituting aspartic acid D for glycine G at amino acid position 12 of KRAS; a mutation of substituting alanine A for glycine G at amino acid position 12 of KRAS; a mutation of substituting valine V for glycine G at amino acid position 12 of KRAS; a mutation of substituting serine S for glycine G at amino acid position 12 of KRAS; a mutation of substituting arginine R for glutamine Q at amino acid position 61 of NRAS; a mutation of substituting lysine K for glutamine Q at amino acid position 61 of NRAS; a mutation of substituting aspartic acid D for glycine G at amino acid position 12 of NRAS; a mutation of substituting threonine T for alanine A at the amino acid position 59 of NRAS; a mutation of substituting lysine K for alanine A at the amino acid position 59 of NRAS; a mutation of substituting asparagine N for aspartic acid D at amino acid position 880 of HER2; a mutation of substituting tyrosine Y for glutamic acid E at amino acid position 837 of HER2; a mutation of substituting valine V for aspartic acid D at amino acid position 816 of KIT; a mutation of substituting arginine R for histidine H at amino acid position 1047 of PIK3CA; a mutation of substituting lysine K for glutamic acid E at amino acid position 545 of PIK3CA.

16. The reference DNA according to any one of claims 1-15, **characterized in that** the reference DNA is synthesized by chemical methods.

17. The reference DNA according to any one of claims 1-16, which is used as a reference standard DNA.

18. A reference cell, **characterized in that** it contains the reference DNA of any one of claims 1-17.

19. The reference cell according to claim 18, **characterized in that** the gene contained in the reference DNA exist in homozygous or heterozygous state, preferably the cell is a prokaryotic cell or an eukaryotic cell.

20. The reference cell according to claim 18, **characterized in that** the cell is derived from a mammal.

21. The reference cell according to claim 18, **characterized in that** the cell is derived from a human.

22. The reference cell according to claim 18, **characterized in that** the cell is derived from a tumor tissue cell.

23. The reference cell according to claim 18, **characterized in that** the cell is constructed (engineered) by a method including, but not limited to, gene editing technology, such as the CRISPER-Cas9, TALEN or ZFN, preferably, the cell is used as a reference standard cell.

24. A vector, **characterized in that** it comprises the reference DNA of any one of claims 1-17, preferably, the vector is a plasmid vector or a viral vector, preferably a prokaryotic cell vector or an eukaryotic cell vector, more preferably, the prokaryotic vector includes, but is not limited to, a pUC19 plasmid, and the eukaryotic viral vector includes, but is not limited to, an adenovirus (AV), an adeno-associated virus (AAV).

25. A host cell, **characterized in that** it comprises the vector of claim 24, preferably the host cell is a prokaryotic cell or a eukaryotic cell, more preferably an *E. coli* cell, or a yeast cell.

26. A method of detecting whether the sample of a subject carries a defined gene mutation (preferably the method is a whole genome sequencing method or a next-generation sequencing method, more preferably a targeted sequencing of a next-generation sequencing method), **characterized in that** one or more (preferably 1-1000, 10-900, 100-800, 200-700, 300-600 or 400-500) of the reference DNA according to any one of claims 1-17, the reference cell according to any one of claims 18 to 23, the vector according to claim 24 or the host cell according to claim 25 are spiked into the sample to be detected.

27. The method according to claim 26, **characterized in that** the sample to be detected is from the subject, including, but not limited to, a cell derived from blood, saliva, urine, tissue, cerebrospinal fluid, or alveolar lavage fluid, or a DNA extract from the above sample(s).

28. The method according to claim 26 or 27, **characterized in that**, the cell contained in the sample of the subject includes, but not limited to, a tissue cell and/or a circulating tumor cell derived from a colon cancer patient, preferably, the cell comprises a protein encoded by a gene having the codon with the defined base X1 mutation, and the position of the amino acid encoded by the codon in the protein includes, but not limited to, amino acid positions 12 and/or 13 of KRAS, amino acid positions 12, 13 and/or 600 of BRAF, amino acid positions 12, 59 and/or 61 of NRAS, and/or amino acid positions 545 and/or 1047 of PIK3CA, wherein the amino acid positions are calculated taking the amino acid encoded by the start codon of the wild type of the gene as 1.

29. The method according to claim 26 or 27, **characterized in that**, the cell contained in the sample of the subject includes, but is not limited to, a tissue cell and/or a circulating tumor cell derived from a lung cancer patient, the reference cell comprises the protein encoded by the gene comprising the codon having the defined base X1 mutation in the reference DNA fragment and the position of the amino acid encoded by the codon in the protein includes, but not limited to, amino acid positions 858, 790, 768, 746, 747, 748, 749, 750, 719 and/or 797 of EGRF, amino acid position 12 and/or 13 of KRAS, amino acid positions 12, 13 and/or 600 of BRAF, wherein the amino acid positions are calculated taking the amino acid encoded by the start codon of the wild type of the gene as 1.

30. The method according to claim 26 or 27, **characterized in that**, the cell contained in the sample of the subject includes, but is not limited to, a tissue cell and/or a circulating tumor cell derived from a breast cancer patient, the reference cell comprises the protein encoded by the gene comprising the codon having the defined base X1 mutation in the reference DNA fragment and the position of the amino acid encoded by the codon in the protein includes, but not limited to, amino acid positions 858, 790, 797, 719 and/or 768 of EGRF, amino acid position 12 and/or 13 of KRAS, amino acid positions 12, 13 and/or 600 of BRAF, and/or amino acid positions 880 and/or 837 of HER2, wherein the amino acid positions are calculated taking the amino acid encoded by the start codon of the wild type of the gene as 1.

31. The method according to claim 26, **characterized in that** the reference DNA is from the genomic DNA in the reference cell of claim 18.

32. The method according to claim 31, **characterized in that**, the DNA of the sample to be detected includes, but not limited to, a DNA from a tissue or a cell derived from a colon cancer patient, wherein a protein is encoded by the gene comprising the codon having the defined base X1 mutation in the reference DNA and the position of the amino acid encoded by the codon in the protein includes, but not limited to, amino acid positions 12 and/or 13 of KRAS, amino acid positions 12, 13 and/or 600 of BRAF, amino acid positions 12, 59 and/or 61 of NRAS, and/or amino acid positions 545 and/or 1047 of PIK3CA, wherein the amino acid positions are calculated by taking the position of the amino acid encoded by the start codon of the wild type of the gene as 1.

33. The method according to claim 31, **characterized in that**, the DNA of the sample to be detected includes, but not limited to, a DNA from a tissue or a cell derived from a lung cancer patient, wherein a protein is encoded by the gene comprising the codon having the defined base X1 mutation in the reference DNA and the position of the amino acid encoded by the codon in the protein includes, but not limited to, amino acid positions 858, 790, 768, 746, 747, 748, 749, 750, 719 and/or 797 of EGRF, amino acid position 12 and/or 13 of KRAS, and/or amino acid positions 12, 13 and/or 600 of BRAF, wherein the amino acid positions are calculated by taking the position of the amino acid encoded by the start codon of the wild type of the gene as 1.

34. The method according to claim 31, **characterized in that**, the DNA of the sample to be detected includes, but not limited to, a DNA from a tissue or a cell derived from a breast cancer patient, wherein a protein is encoded by the gene comprising the codon having the defined base X1 mutation in the reference DNA and the position of the amino acid encoded by the codon in the protein includes, but not limited to, amino acid positions 858, 790, 797, 719 and/or 768 of EGRF, amino acid position 12 and/or 13 of KRAS, amino acid positions 12, 13 and/or 600 of BRAF, and/or amino acid positions 880 and/or 837 of HER2, wherein the amino acid positions are calculated by taking the position of the amino acid encoded by the wild type of the start codon of the gene as 1.

35. The method according to claim 26, **characterized in that** the DNA of the sample to be detected is fragmented, and the DNA of the sample to be detected is circulating free DNA in cells, tissues, saliva and blood, and the spiked-in reference DNA has a length of 20 bp to 500 bp, wherein about 60-90% of the reference DNAs are 140-170 bp in length.

36. The method according to claim 35, wherein when the reference DNA is a mixture of the DNA fragment 1 and the DNA fragment 2, the content percentage of the DNA fragment 1 and the DNA fragment 2 is 0.01% to 99.9%; preferably 10%, 25% or 50%; more preferably 1.0%, 2.5% or 5%; further preferably 0.01%, 0.025% or 0.05%.

37. A kit, **characterized in that** the kit comprises one or more (preferably 1-1000, 10-900, 100-800, 200-700, 300-600, 400-500) of the reference DNAs of any one of claims 1-17, preferably the number of the reference DNAs is from 1 to $10^9$.

38. The kit according to claim 37, **characterized in that** the DNA fragment 1 is or is not mixed with the DNA fragment 2.

39. The kit according to claim 38, wherein when the DNA fragment 1 is mixed with the DNA fragment 2, the content percentage of the DNA fragment 1 and the DNA fragment 2 is 0.01% to 99.9%; preferably 10%, 25% or 50%; more preferably 1.0%, 2.5% or 5%; further preferably 0.01%, 0.025% or 0.05%.

40. A kit, **characterized in that** the kit comprises one or more (preferably 1-1000, 10-900, 100-800, 200-700, 300-600, 400-500) of the reference cells of any one of claims 18-23, preferably the number of the reference cells is from 1 to $10^9$.

41. The kit according to claim 40, **characterized in that** the DNA fragment 1 and the DNA fragment 2 are present in different cells or in the same cell, alternatively, when the DNA fragment 1 and the DNA fragment 2 are present in different cells, the different cells are present in a mixed form or in an isolated form.

42. The kit according to claim 41, **characterized in that** when the DNA fragment 1 and the DNA fragment 2 are present in different cells, the content percentage of a cell containing the DNA fragment 1 and a cell containing the DNA fragment 2 is 0.01% to 99.9%; preferably 10%, 25% or 50%; more preferably 1.0%, 2.5% or 5%; further preferably 0.01%, 0.025% or 0.05%.

43. A method of ensuring sensitivity and accuracy of detection of a gene mutation associated with the occurrence, diagnosis, and/or treatment (such as a target targeted by a medicament) of a disease (such as a tumor), **characterized in that** using the reference DNA according to any one of claims 1-17 or the reference cell according to any one of claims 18-23 as a reference standard for parallel experiments of the sequencing process of the sample to be detected and a reference standard to be spiked into the sample to be detected.

44. Use of the reference DNA according to any one of claims 1-17, or the reference cell of any one of claims 18-23 in the manufacture of a reagent for detecting whether a defined gene mutation is present in a sample of a subject, preferably for quality analysis and/or quality control, preferably, the defined gene mutation is associated with the occurrence, diagnosis, and/or treatment (such as a target targeted by a medicament) of a disease (such as a tumor) .

45. Use of the reference DNA according to any one of claims 1-17 or the reference cell according to any one of claims 18-23 as a reference standard for parallel experiments of the sequencing process of the sample to be detected and a reference standard to be spiked into the sample to be detected.

Fig. 1

Fig. 2

Fig. 3

```
G80608              _____
DC-HTN001161-D04-L  ACGCTTCTCGCTGCTCTTTGAGCCTGCAGACACCTGGGGGGATACGGGGAAAAGGCCTCC 60


G80608              ACGGCCACTAGTAACGGCCGCCAGTGTGCTGGCACTCTGTACTAGAAAGTACATGAACAT 60
DC-HTN001161-D04-L  ACGGCCACTAGTAACGGCCGCCAGTGTGCTGGCACTCTGTACTAGAAAGTACATGAACAT 120
                    ************************************************************
                                     THE START END OF THE LEFT ARM

G80608              CAGCCATACAGGGAACTAGAAAGGTGGCCCACCCTCTTGGTGGAGAGAGAAGAGAGTGTG 120
DC-HTN001161-D04-L  CAGCCATAAAGGGAACTAGAAAGGTGGCCCACCCTCTTGGTGGAGAGAGAAGAGAGTGTG 180
                    ********* **************************************************

G80608              GTAGAAACAATAATAAGAAGTCTGCAGAACTTGACCCCTCCCAGCCTCTCCCACCTGCCA 180
DC-HTN001161-D04-L  GTAGAAACAATAATAAGAAGTCTGCAGAACTTGACCCCTCCCAGCCTCTCCCACCTGCCA 240
                    ************************************************************

G80608              GCCTGGCCCTTGCAGAGAGATGCAGGCTGCCATTCTTAGGCCAAAGCCTGGGACAGTTGG 240
DC-HTN001161-D04-L  GCCTGGCCCTTGCAGAGAGATGCAGGCTGCCATTCTTAGGCCAAAGCCTGGGACAGTTGG 300
                    ************************************************************

G80608              GCTCAGCAAGGTAGGCATCCGTCAAGCAAGGAGGAGCAGGGGTCAGCAGTGACCCCAGCA 300
DC-HTN001161-D04-L  GCTCAGCAAGGTAGGCATCCGTCAAGCAAGGAGGAGCAGGGGTCAGCAGTGACCCCAGCA 360
                    ************************************************************

G80608              GCCAGCAGGGAGAAAGGTGCATGTGACAAGGACACCAGAGGCCGTGGGTCAGGATCAGCC 360
DC-HTN001161-D04-L  GCCAGCAGGGAGAAAGGTGCATGTGACAAGGACACCAGAGGCCGTGGGTCAGGATCAGCC 420
                    ************************************************************

G80608              AGGGTCAGGGTAGCATTTCTAGGAATTCACTCTGTTGGGCGCTGTGCTGGCTGCTTCTCA 420
DC-HTN001161-D04-L  AGGGTCAGGGTAGCATTTCTAGGAATTCACTCTGTTGGGCGCTGTGCTGGCTGCTTCTCA 480
                    ************************************************************

G80608              CATATTATTCCTTTCTTACTCTCAGAGCAGAGATTTCAATTGCAGCGAGATTGTGGAGGC 480
DC-HTN001161-D04-L  CATATTATTCCTTTCTTACTCTCAGAGCAGAGATTTCAATTGCAGCGAGATTGTGGAGGC 540
                    ************************************************************

G80608              AGCCAGGGAGGTGGGGAGGGTGGTGTGTCTTCTAAAAGCATTTTCAGTATCCATGTGGTTTC 540
DC-HTN001161-D04-L  AGCCAGGGAGGTGGGGAGGGTGGTGTGTCTTCTAAAAGCATTTTCAGTATCCATGTGGTTTC 600
                    ************************************************************

G80608              AGTAATAATAATAATAATAAACCAGTGAAAAGTAAAACAGGACAAAAATCTTCATAGGCA 600
DC-HTN001161-D04-L  AGTAATAATAATAATAATAAACCAGTGAAAAGTAAAACAGGACAAAAATCTTCATAGGCA 660
                    ************************************************************

G80608              GTGAACCATATCAGAGAGTCCAAGAAAGCACAATGAGAGTGTGGCTTAAAAACCCTGAAC 660
```

Fig. 4

```
DC-HTN001161-D04-L    GTGAACCATATCAGAGAGTCCAAGAAAGCACAATGAGAGTGTGGCTTAAAAACCCTGAAC 720
                      ************************************************************

G80608                GACATTCCTTTGCACCAGCTTGGTGAGGAGGGCATGGTCCCCGCCACCCCCCACCCCCAC 720
DC-HTN001161-D04-L    GACATTCCTTTGCACCAGCTTGGTGAGGAGGGCATGGTCCCCGCCACCCCCCACCCCCAC 780
                      ************************************************************

G80608                TTTGCAGATAAACCACATGCAGGAAGGTCAGCCTGGCAAGTCCAGTAAGTTCAAGCCCAG 780
DC-HTN001161-D04-L    TTTGCAGATAAACCACATGCAGGAAGGTCAGCCTGGCAAGTCCAGTAAGTTCAAGCCCAG 840
                      ************************************************************

G80608                GTCTCAACTGGGCAGCAGAGCTCCTGCTCTTCTTTGTCCTTTCGAACACACAAAAAACCA 840
DC-HTN001161-D04-L    GTCTCAACTGGGCAGCAGAGCTCCTGCTCTTCTTTGTCCTTTCGAACACACAAAAAACCA 900
                      ************************************************************

G80608                ACACACAGATGTAATGAAAAATAAGATATTTTATTGCAACGCATGATACAAAGGCATTAA 900
DC-HTN001161-D04-L    ACACACAGATGTAATGAAAAATAAGATATTTTATTGCAACGCATGATACAAAGGCATTAA 960
                      ************************************************************

G80608                AGCAGCGTATCCACATAGCGTAAAAGGAGCAACATAGTTAAGAATACCAGTCAATCTTTC 960
DC-HTN001161-D04-L    AGCAGCGTATCCACATAGCGTAAAAGGAGCAACATAGTTAAGAATACCAGTCAATCTTTC 1020
                      ************************************************************

G80608                ACAAATTTTGTAATCCAGAGGTTGATTAAGCTTTCAGGCACCGGGCTTGCGGGTCATGCA 1020
DC-HTN001161-D04-L    ACAAATTTTGTAATCCAGAGGTTGATTAAGCTTTCAGGCACCGGGCTTGCGGGTCATGCA 1080
                      ************************************************************

G80608                CCAGGTGCGCGGTCCTTCGGGCACCTCGA 1049
DC-HTN001161-D04-L    CCAGGT----------------------- 1086
                      ******
```

# Fig. 4 (continue)

```
G80789            --------GGGGTCGTTGGGCGGTCAGCCAGGCGGGCCATTTACCGTAAGTTATGTAACGC 53
DC-HTN001161-D04-R ATGGGCGGGGGTCGTTGGGCGGTCAGCCAGGCGGGCCATTTACCGTAAGTTATGTAACGC 60
                          ************************************************************


G80789            GGAACTCCATATATGGGCTATGAACTAATGACCCCGTAATTGATTACTATTAATAACTAG 113
DC-HTN001161-D04-R GGAACTCCATATATGGGCTATGAACTAATGACCCCGTAATTGATTACTATTAATAACTAG 120
                   ************************************************************


G80789            TCAATAATCAATGTCAACTGGATGGAGAAAAGTTAATGGTCAGCAGCGGGTTACATCTTC 173
DC-HTN001161-D04-R TCAATAATCAATGTCAACTGGATGGAGAAAAGTTAATGGTCAGCAGCGGGTTACATCTTC 180
                   ************************************************************


G80789            TTTCATGCGCCTTTCCATTCTTTGGATCAGTAGTCACTAACGTTCGCCAGCCATAAGTCC 233
DC-HTN001161-D04-R TTTCATGCGCCTTTCCATTCTTTGGATCAGTAGTCACTAACGTTCGCCAGCCATAAGTCC 240
                   ************************************************************


G80789            TCGACGTGGAGAGGCTCAGAGCCTGGCATGAACATGACCCTGAATTCGGATGCAGAGCTT 293
DC-HTN001161-D04-R TCGACGTGGAGAGGCTCAGAGCCTGGCATGAACATGACCCTGAATTCGGATGCAGAGCTT 300
                   ************************************************************


G80789            CTTCCCATGATGATCTGTCCCTCACAGCAGGGTCTTCTCTGTTTCAGGGCATGAACTACT 353
DC-HTN001161-D04-R CTTCCCATGATGATCTGTCCCTCACAGCAGGGTCTTCTCTGTTTCAGGGCATGAACTACT 360
                   ************************************************************


G80789            TGGAGGACCGTCGCTTGGTGCACCGCGACCTGGCAGCCAGGAACGTACTGGTGAAAACAC 413
DC-HTN001161-D04-R TGGAGGACCGTCGCTTGGTGCACCGCGACCTGGCAGCCAGGAACGTACTGGTGAAAACAC 420
                   ************************************************************


G80789            CGCAACACGTCAAGATCACAGATTTTGGGCGGGCCAAACTGCTGGGTGCGGAAGAGAAAG 473
DC-HTN001161-D04-R CGCAACACGTCAAGATCACAGATTTTGGGCGGGCCAAACTGCTGGGTGCGGAAGAGAAAG 480
                   ************************************************************

                   cagcat->caacac QH              EGFR L858R ctg->cgg
G80789            AATACCATGCAGAAGGAGGCAAAGTAAGGAGGTGGCTTTAGGTCAGCCAGCATTTTCCTG 533
DC-HTN001161-D04-R AATACCATGCAGAAGGAGGCAAAGTAAGGAGGTGGCTTTAGGTCAGCCAGCATTTTCCTG 540
                   ************************************************************
```

Fig. 5

```
G80789            ACACCAGGGACCAGGCTGCCTTCCCACTAGCTGTATTGTTTAACACATGCAGGGGAGGAT 593
DC-HTN001161-D04-R ACACCAGGGACCAGGCTGCCTTCCCACTAGCTGTATTGTTTAACACATGCAGGGGAGGAT 600
                  ************************************************************


G80789            GCTCTCCAGACATTCTGGGTGAGCTCGCAGCAGCTGCTGCTGGCAGCTGGGTCCAGCCAG 653
DC-HTN001161-D04-R GCTCTCCAGACATTCTGGGTGAGCTCGCAGCAGCTGCTGCTGGCAGCTGGGTCCAGCCAG 660
                  ************************************************************


G80789            GGTCTCCTGGTAGTGTGAGCCAGAGCTGCTTTGGGAACAGTACTTGCTGGGACAGTGAAT 713
DC-HTN001161-D04-R GGTCTCCTGGTAGTGTGAGCCAGAGCTGCTTTGGGAACGGTACTTGCTGGGACAGTGAAT 720
                  *************************************** ********************
                                                       a->G rs6970262


G80789            GAGGATGTTATCCCCAGGTGATCATTAGCAAATGTTAGGTTTCAGTCTCTCCCTGCAGGA 773
DC-HTN001161-D04-R GAGGATGTTATCCCCAGGTGATCATTAGCAAATGTTAGGTTTCAGTCTCTCCCTGCAGGA 780
                  ************************************************************


G80789            TATATAAGTCCCCTTCAATAGCGCAATTGGGAAAGGTCACAGCTGCCTTGGTGGTCCACT 833
DC-HTN001161-D04-R TATATAAGTCCCCTTCAATAGCGCAATTGGGAAAGGTCACAGCTGCCTTGGTGGTCCACT 840
                  ************************************************************


G80789            GCTGTCAAGGACACCTAAGGAACAGGAAAGGCCCCATGCGGACCCGAGCTCCCAGGGCTG 893
DC-HTN001161-D04-R GCTGTCAAGGACACCTAAGGAACAGGAAAGGCCCCATGCGGACCCGAGCTCCCAGGGCTG 900
                  ************************************************************


G80789            TCTGTGGCTCGTGGCTGGGACAGGCAGCAATGGAGTCCTTCTCTCCCTTCACTGGCTCGG 953
DC-HTN001161-D04-R TCTGTGGCTCGTGGCTGGGACAGGCAGCAATGGAGTCCTTCTCTCCCTTCACTGGCTCGG 960
                  ************************************************************


G80789            TTTCTATTAGGGACCCTCACAGCACTAAGGGGTGCGCGTCCCCTGTCAGGCCCTCGAATG 1013
DC-HTN001161-D04-R TTTCTCTTAGGGACCCTCACAGCACTAAGGGGTGCGCGTCCCCTGTCAGGCCCTCGAATG 1020
                  ***** ******************************************************


G80789            CCCTCCCACAGCCAGGCCCCTCTGAGGTTTCACTCTGGCCTGCTTGGCTCCTAGCAGCCA 1073
DC-HTN001161-D04-R CCCTCCCACAGCCAGGCCCCTCTGAGGTTTCACTCTGGCCTGCTTGGCTCCTAGCAGCCA 1080
                  ************************************************************
```

# Fig. 5 (continue)

G80789              CCAACCCATGATGCTGGGCCCTGAAAACACACGCAGACCTGGATGAGTGAGGCCACTGGG 1133

DC-HTN001161-D04-R  CCAACCCATGATGCTGGGCCCTGAAAACACACGCAGACCTGGATGAGTGAGGCCACTGGG 1140

                    ************************************************************


G80789              CACAACCAGGGCTCCCAGCTCACCAGAGCAGCCTGGGACACAGAGGGTGCTCAGAAACCT 1193

DC-HTN001161-D04-R  CACAACCAGGGCTCCCAGCTCACCAGAGCAGCCTGGGACACAGAGGGTGCTCAGAAACCT 1200

                    ************************************************************


G80789              ACCAGAGCAGCCCTGAACTCCGTCAGACTGAAATCCCCTGTTGCCGGGAGGACTCGAGAT 1253

DC-HTN001161-D04-R  ACCAGAGCAGCCCTGAACTCCGTCAGACTGAAATCCCCTGTTGCCGGGAGGACTCGAGAT 1260

                    ************************************************************


G80789              GCATGCGTCAATTTTACGCAGACTATCTTTCTAGGGTTAATCTAGCTGCATCAGGATCAT 1313

DC-HTN001161-D04-R  GCATGCGTCAATTTTACGCAGACTATCTTTCTAGGGTTAATCTAGCTGCATCAGGATCAT 1320

                    ************************************************************


G80789              ATCGTCGGGTCTTTTTTCCGGCTCAG 1339

DC-HTN001161-D04-R  ATCGTCGGGTCTTTTTTCCGGCT—— 1343

                    **********************

Fig. 5(continue)

Fig. 6

Fig. 7

| V87369 | GGATCCGTCTGTGATCTTGACATGCTGGTTTTAGAGCTAGAAATAGCAAG 50 |
| HCP001161-CG08-3-10-a | GGATCCGTCTGTGATCTTGACATGCTGGTTTTAGAGCTAGAAATAGCAAG 50 |
| | ************************************************** |
| | TARGET SEQUENCE |
| V87369 | TTAAAATAAGGCTAGTCCGTTATCAACTTGAAAAAGTGGCACCGAGTCGG 100 |
| HCP001161-CG08-3-10-a | TTAAAATAAGGCTAGTCCGTTATCAACTTGAAAAAGTGGCACCGAGTCGG 100 |
| | ************************************************** |
| V87369 | TGCTTTTTT 109 |
| HCP001161-CG08-3-10-a | TGCTTTTTT 109 |
| | ********* |

Fig. 8

Fig. 9

DNA Ladder 6000

Fig. 10

L858R (CTG WAS CHANGED INTO CGG)

Fig. 11

CAG WAS MUTATED TO CAA,  Q WAS CHANGED INTO Q          CTG WAS CHANGED INTO CGG   L858R
CAT WAS MUTATED TO CAC,  H WAS CHANGED INTO H

Fig. 12

a

EGFR (EXON 21)

L858R (CTG WAS CHANGED INTO CGG)

b

CAG WAS MUTATED TO CAA, Q WAS CHANGED INTO Q    CTG WAS CHANGED INTO CGG    L858R
CAT WAS MUTATED TO CAC, H WAS CHANGED INTO H

Fig. 13

Fig. 14

Fig. 15

MIXED DNA SAMPLE

↓ PCR AMPLIFICATION WAS PERFORMED WITH BRAF
V600E PRIMERS BY USING AMPLISEQ TECHNOLOGY

↓ THE PCR PRIMERS WERE REMOVED

↓ LINKERS WERE LIGATED

↓ PCR AMPLIFICATION WERE PERFORMED
WITH SEQUENCING PRIMERS

Fig. 16

WT

$N$ $N$ $N$ $N$ $N$ $N$ $N$...... // .....$N_a$ $N_b$ $N_j$ $N_i$ $N_h$ $N_g$ $N_f$ $N_e$ $N_d$ $N_c$ $N_b$ $N_a$ $N_1$ $N_2$ $N_3$ $N_4$ $N_5$ $N_6$ $N_7$ $N_8$ $N_9$ $N_{10}$ $N_{11}$ ...... // .....$N$ $N$ $N$ $N$ $N$ $N$

F1      X2= 3n, n=3, X2= 9          X1

$N$ $N$ $N$ $N$ $N$ $N$... // ...$N_a$ $N_b$ $N_j$ $N_i$ $N_h$ $N_g$ $N$ $N_e$ $N_d$ $N_c$ $N_b$ $N_a$ $N_1$ $N_2$ $N_3$ $N_4$ $N_5$ $N_6$ $N_7$ $N_8$ $N_9$ $N_{10}$ $N_{11}$ ...... // ...$N$ $N$ $N$ $N$ $N$ $N$

F2      X2= 3n, n=3, X2= 9

$N$ $N$ $N$ $N$ $N$ $N$ $N$.... // ...$N_a$ $N_b$ $N_j$ $N_i$ $N_h$ $N_g$ $N_f$ $N_e$ $N_d$ $N_c$ $N_b$ $N_a$ $N_1$ $N_2$ $N_3$ $N_4$ $N_5$ $N_6$ $N_7$ $N_8$ $N_9$ $N_{10}$ $N_{11}$ ...... // ...$N$ $N$ $N$ $N$ $N$ $N$

NOTE 1:
F1: A REFERENCE DNA FRAGMENT CARRYING A MARKER ABLE TO BE SPIKED INTO A SAMPLE TO BE
DETECTED AND A DEFINED GENE MUTATION, COMPRISING (i) A DEFINED GENE MUTATION, WHEREIN,
AS COMPARED TO A WILD TYPE OF THE GENE, AT LEAST ONE DEFINED BASE X1 IN THE DEFINED GENE
MUTATION UNDERGOES A MUTATION ASSOCIATED WITH THE ONSET, DIAGNOSIS, AND/OR TREATMENT OF
A DISEASE (SUCH AS A DRUG TARGET), WHEREIN THE MUTATION IS A SUBSTITUTION MUTATION,
A DELETION MUTATION, OR AN INSERTION MUTATION, AND (ii) AT LEAST ONE OTHER ARTIFICIALLY
CHANGED BASE X2, TO DISTINGUISH FROM THE REFERENCE DNA FRAGMENT SPIKED INTO THE SAMPLE
TO BE DETECTED AND THE MUTANT X1 FRAGMENT THAT IS CONTAINED IN THE SAMPLE TO BE DETECTED
AND DETERMINED TO BE ASSOCIATED WITH THE ONSET, DIAGNOSIS AND/OR TREATMENT OF A DISEASE.

F2: DNA FRAGMENT 2 COMPRISING THE BASE X2 AND BUT NOT COMPRISING THE DEFINED BASE X1,
WHEREIN THE DNA FRAGMENT 2 AND THE REFERENCE DNA FRAGMENT ARE MIXED AND THEN SPIKED
INTO A SAMPLE TO BE DETECTED, OR THE DNA FRAGMENT 2 AND THE REFERENCE DNA FRAGMENT
ARE SPIKED INTO A SAMPLE TO BE DETECTED, RESPECTIVELY.

NOTE 2:
$N_a$ REPRESENTS THE REFERENCE POSITION OF X2 IN THE DNA FRAGMENT SITE, WHICH IS SET AS O;
n IS A POSITIVE INTEGER

REPRESENTS CODON.

Fig. 17

WT

F1

X1

X2= -3n, n=3, X2= -9

F2

X2= -3n, n=3, X2= -9

NOTE 1:

F1: A REFERENCE DNA FRAGMENT CARRYING A MARKER ABLE TO BE SPIKED INTO A SAMPLE TO BE DETECTED AND A DEFINED GENE MUTATION, COMPRISING (i) A DEFINED GENE MUTATION, WHEREIN, AS COMPARED TO A WILD TYPE OF THE GENE, AT LEAST ONE DEFINED BASE X1 IN THE DEFINED GENE MUTATION UNDERGOES A MUTATION ASSOCIATED WITH THE ONSET, DIAGNOSIS, AND/OR TREATMENT OF A DISEASE (SUCH AS A DRUG TARGET), WHEREIN THE MUTATION IS A SUBSTITUTION MUTATION, A DELETION MUTATION, OR AN INSERTION MUTATION, AND (ii) AT LEAST ONE OTHER ARTIFICIALLY CHANGED BASE X2, TO DISTINGUISH FROM THE REFERENCE DNA FRAGMENT SPIKED INTO THE SAMPLE TO BE DETECTED AND THE MUTANT X1 FRAGMENT THAT IS CONTAINED IN THE SAMPLE TO BE DETECTED AND DETERMINED TO BE ASSOCIATED WITH THE ONSET, DIAGNOSIS AND/OR TREATMENT OF A DISEASE.

F2: DNA FRAGMENT 2 COMPRISING THE BASE X2 AND BUT NOT COMPRISING THE DEFINED BASE X1, WHEREIN THE DNA FRAGMENT 2 AND THE REFERENCE DNA FRAGMENT ARE MIXED AND THEN SPIKED INTO A SAMPLE TO BE DETECTED, OR THE DNA FRAGMENT 2 AND THE REFERENCE DNA FRAGMENT ARE SPIKED INTO A SAMPLE TO BE DETECTED, RESPECTIVELY.

NOTE 2:

$N_0$ REPRESENTS THE REFERENCE POSITION OF X2 IN THE DNA FRAGMENT SITE, WHICH IS SET AS 0;
n IS A POSITIVE INTEGER.

REPRESENTS CODON.

Fig. 18

WT

F1

X2= 3n, n=3, X2= 9

X1

X2= -3n, n=3, X2= -9

F2

X2= 3n, n=3, X2= 9

X2= -3n, n=3, X2= -9

NOTE 1:

F1: A REFERENCE DNA FRAGMENT CARRYING A MARKER ABLE TO BE SPIKED INTO A SAMPLE TO BE DETECTED AND A DEFINED GENE MUTATION, COMPRISING (i) A DEFINED GENE MUTATION, WHEREIN, AS COMPARED TO A WILD TYPE OF THE GENE, AT LEAST ONE DEFINED BASE X1 IN THE DEFINED GENE MUTATION UNDERGOES A MUTATION ASSOCIATED WITH THE ONSET, DIAGNOSIS, AND/OR TREATMENT OF A DISEASE (SUCH AS A DRUG TARGET), WHEREIN THE MUTATION IS A SUBSTITUTION MUTATION, A DELETION MUTATION, OR AN INSERTION MUTATION, AND (ii) AT LEAST ONE OTHER ARTIFICIALLY CHANGED BASE X2, TO DISTINGUISH FROM THE REFERENCE DNA FRAGMENT SPIKED INTO THE SAMPLE TO BE DETECTED AND THE MUTANT X1 FRAGMENT THAT IS CONTAINED IN THE SAMPLE TO BE DETECTED AND DETERMINED TO BE ASSOCIATED WITH THE ONSET, DIAGNOSIS AND/OR TREATMENT OF A DISEASE.

F2: DNA FRAGMENT 2 COMPRISING THE BASE X2 AND BUT NOT COMPRISING THE DEFINED BASE X1, WHEREIN THE DNA FRAGMENT 2 AND THE REFERENCE DNA FRAGMENT ARE MIXED AND THEN SPIKED INTO A SAMPLE TO BE DETECTED, OR THE DNA FRAGMENT 2 AND THE REFERENCE DNA FRAGMENT ARE SPIKED INTO A SAMPLE TO BE DETECTED, RESPECTIVELY.

NOTE 2:

$N_0$ REPRESENTS THE REFERENCE POSITION OF X2 IN THE DNA FRAGMENT SITE, WHICH IS SET AS 0;
n IS A POSITIVE INTEGER.

Fig. 19

WT

N N N N N N N...... // ...... N_L N_k N_j N_i N_h N_g N_f N_e N_d N_c N_b N_a N_0 N_1 N_2 N_3 N_4 N_5 N_6 N_7 N_8 N_9 N_{10}...... // ...... N N N N N N

F1   X2= 3n, n=3, X2= 9   X1

N N N N N N N...... // ...... N_L N_k N_j N_i N_h N_g N_f N_e N_d N_c N_b N_a N' N_1 N_2 N_3 N_4 N_5 N_6 N_7 N_8 N_9 N_{10} ...... // ...... N N N N N N

F2   X2= 3n, n=3, X2= 9

N N N N N N N..... // ...... N_L N_k N_j N_i N_h N_g N_f N_e N_d N_c N_b N_a N_1 N_2 N_3 N_4 N_5 N_6 N_7 N_8 N_9 N_{10}... // ...... N N N N N N

NOTE 1:
F1: A REFERENCE DNA FRAGMENT CARRYING A MARKER ABLE TO BE SPIKED INTO A SAMPLE TO BE DETECTED AND A DEFINED GENE MUTATION, COMPRISING (i) A DEFINED GENE MUTATION, WHEREIN, AS COMPARED TO A WILD TYPE OF THE GENE, AT LEAST ONE DEFINED BASE X1 IN THE DEFINED GENE MUTATION UNDERGOES A MUTATION ASSOCIATED WITH THE ONSET, DIAGNOSIS, AND/OR TREATMENT OF A DISEASE (SUCH AS A DRUG TARGET), WHEREIN THE MUTATION IS A SUBSTITUTION MUTATION, A DELETION MUTATION, OR AN INSERTION MUTATION, AND (ii) AT LEAST ONE OTHER ARTIFICIALLY CHANGED BASE X2, TO DISTINGUISH FROM THE REFERENCE DNA FRAGMENT SPIKED INTO THE SAMPLE TO BE DETECTED AND THE MUTANT X1 FRAGMENT THAT IS CONTAINED IN THE SAMPLE TO BE DETECTED AND DETERMINED TO BE ASSOCIATED WITH THE ONSET, DIAGNOSIS AND/OR TREATMENT OF A DISEASE.
F2: DNA FRAGMENT 2 COMPRISING THE BASE X2 AND BUT NOT COMPRISING THE DEFINED BASE X1, WHEREIN THE DNA FRAGMENT 2 AND THE REFERENCE DNA FRAGMENT ARE MIXED AND THEN SPIKED INTO A SAMPLE TO BE DETECTED, OR THE DNA FRAGMENT 2 AND THE REFERENCE DNA FRAGMENT ARE SPIKED INTO A SAMPLE TO BE DETECTED, RESPECTIVELY.

NOTE 2:
N_0 REPRESENTS THE REFERENCE POSITION OF X2 IN THE DNA FRAGMENT SITE, WHICH IS SET AS 0;
n IS A POSITIVE INTEGER.

⌐‗‗‗⌐ REPRESENTS CODON.

Fig. 20

WT

N N N N N N...... // ...... N_L N_k N_j N_i N_h N_g N_f N_e N_d N_c N_b N_a N_1 N_2 N_3 N_4 N_5 N_6 N_7 N_8 N_9 N_{10} N_{11}...... // ...... N N N N N N

F1   X1   X2

N N N N N N...... // ...... N_L N_k N_j N_i N_h N_g N_f N_e N_d N_c N_b N_a N' N_1 N_2 N_3 N_4 N_5 N_6 N_7 N_{8'} N_9 N_{10} N_{11}...... // ...... N N N N N N

F2   X2

N N N N N N...... // ...... N_L N_k N_j N_i N_h N_g N_f N_e N_d N_c N_b N_a N_1 N_2 N_3 N_4 N_5 N_6 N_7 N_{8'} N_9 N_{10} N_{11}...... // ...... N N N N N N

NOTE 1:
F1: A REFERENCE DNA FRAGMENT CARRYING A MARKER ABLE TO BE SPIKED INTO A SAMPLE TO BE DETECTED AND A DEFINED GENE MUTATION, COMPRISING (i) A DEFINED GENE MUTATION, WHEREIN, AS COMPARED TO A WILD TYPE OF THE GENE, AT LEAST ONE DEFINED BASE X1 IN THE DEFINED GENE MUTATION UNDERGOES A MUTATION ASSOCIATED WITH THE ONSET, DIAGNOSIS, AND/OR TREATMENT OF A DISEASE (SUCH AS A DRUG TARGET), WHEREIN THE MUTATION IS A SUBSTITUTION MUTATION, A DELETION MUTATION, OR AN INSERTION MUTATION, AND (ii) AT LEAST ONE OTHER ARTIFICIALLY CHANGED BASE X2, TO DISTINGUISH FROM THE REFERENCE DNA FRAGMENT SPIKED INTO THE SAMPLE TO BE DETECTED AND THE MUTANT X1 FRAGMENT THAT IS CONTAINED IN THE SAMPLE TO BE DETECTED AND DETERMINED TO BE ASSOCIATED WITH THE ONSET, DIAGNOSIS AND/OR TREATMENT OF A DISEASE.
F2: DNA FRAGMENT 2 COMPRISING THE BASE X2 AND BUT NOT COMPRISING THE DEFINED BASE X1, WHEREIN THE DNA FRAGMENT 2 AND THE REFERENCE DNA FRAGMENT ARE MIXED AND THEN SPIKED INTO A SAMPLE TO BE DETECTED, OR THE DNA FRAGMENT 2 AND THE REFERENCE DNA FRAGMENT ARE SPIKED INTO A SAMPLE TO BE DETECTED, RESPECTIVELY.

NOTE 2:
WHEN X2 IS LOCATED DOWNSTREAM OF X1, X2 CAN BE LOCATED AT ANY POSITION.

⌐‗‗‗⌐ REPRESENTS CODON.

Fig. 21

WT

N N N N N N......//.......N<sub>L</sub> N<sub>k</sub> N<sub>j</sub> N<sub>i</sub> N<sub>h</sub> N<sub>g</sub> N<sub>f</sub> N<sub>e</sub> N<sub>d</sub> N<sub>c</sub> N<sub>b</sub> N<sub>a</sub> N<sub>1</sub> N<sub>2</sub> N<sub>3</sub> N<sub>4</sub> N<sub>5</sub> N<sub>6</sub> N<sub>7</sub> N<sub>8</sub> N<sub>9</sub> N<sub>10</sub> N<sub>11</sub> ......// ....N N N N N N

F1       $X2 = 3n$, $n=3$, $X2 = 9$                                X1                    X2

N N N N N N......// ....N<sub>L</sub> N<sub>k</sub> N<sub>f</sub> N<sub>i</sub> N<sub>h</sub> N<sub>g</sub> N<sub>f</sub> N<sub>e</sub> N<sub>d</sub> N<sub>c</sub> N<sub>b</sub> N<sub>a</sub> N' N<sub>1</sub> N<sub>2</sub> N<sub>3</sub> N<sub>4</sub> N<sub>5</sub> N<sub>6</sub> N<sub>7</sub> N<sub>8'</sub> N<sub>9</sub> N<sub>10</sub> N<sub>11</sub>----- //......N N N N N N

F2       $X2 = 3n$, $n=3$, $X2 = 9$                                                     X2

N N N N N N ...// ....N<sub>L</sub> N<sub>k</sub> N<sub>f</sub> N<sub>i</sub> N<sub>h</sub> N<sub>g</sub> N<sub>f</sub> N<sub>e</sub> N<sub>d</sub> N<sub>c</sub> N<sub>b</sub> N<sub>a</sub> N<sub>1</sub> N<sub>2</sub> N<sub>3</sub> N<sub>4</sub> N<sub>5</sub> N<sub>6</sub> N<sub>7</sub> N<sub>8'</sub> N<sub>9</sub> N<sub>10</sub> N<sub>11</sub>...// ...N N N N N N

NOTE 1:
F1: A REFERENCE DNA FRAGMENT CARRYING A MARKER ABLE TO BE SPIKED INTO A SAMPLE TO BE DETECTED AND A DEFINED GENE MUTATION, COMPRISING (i) A DEFINED GENE MUTATION, WHEREIN, AS COMPARED TO A WILD TYPE OF THE GENE, AT LEAST ONE DEFINED BASE X1 IN THE DEFINED GENE MUTATION UNDERGOES A MUTATION ASSOCIATED WITH THE ONSET, DIAGNOSIS, AND/OR TREATMENT OF A DISEASE (SUCH AS A DRUG TARGET), WHEREIN THE MUTATION IS A SUBSTITUTION MUTATION, A DELETION MUTATION, OR AN INSERTION MUTATION, AND (ii) AT LEAST ONE OTHER ARTIFICIALLY CHANGED BASE X2, TO DISTINGUISH FROM THE REFERENCE DNA FRAGMENT SPIKED INTO THE SAMPLE TO BE DETECTED AND THE MUTANT X1 FRAGMENT THAT IS CONTAINED IN THE SAMPLE TO BE DETECTED AND DETERMINED TO BE ASSOCIATED WITH THE ONSET, DIAGNOSIS AND/OR TREATMENT OF A DISEASE.
F2: DNA FRAGMENT 2 COMPRISING THE BASE X2 AND BUT NOT COMPRISING THE DEFINED BASE X1, WHEREIN THE DNA FRAGMENT 2 AND THE REFERENCE DNA FRAGMENT ARE MIXED AND THEN SPIKED INTO A SAMPLE TO BE DETECTED, OR THE DNA FRAGMENT 2 AND THE REFERENCE DNA FRAGMENT ARE SPIKED INTO A SAMPLE TO BE DETECTED, RESPECTIVELY.
NOTE 2:
WHEN X2 IS LOCATED UPSTREAM OF X1, N<sub>a</sub> REPRESENTS THE REFERENCE POSITION OF X2 IN THE DNA FRAGMENT SITE, WHICH IS SET AS 0; n IS AN INTEGER; WHEN X2 IS LOCATED DOWNSTREAM OF X1, X2 CAN BE LOCATED AT ANY POSITION.

        REPRESENTS CODON.

## Fig. 22

WT

N N N N N N......// ....N<sub>L</sub> N<sub>k</sub> N<sub>j</sub> N<sub>h</sub> N<sub>g</sub> N<sub>f</sub> N<sub>e</sub> N<sub>d</sub> N<sub>c</sub> N<sub>b</sub> N<sub>a</sub> N<sub>1</sub> N<sub>2</sub> N<sub>3</sub> N<sub>4</sub> N<sub>5</sub> N<sub>6</sub> N<sub>7</sub> N<sub>8</sub> N<sub>9</sub> ...//......N N N N N N

F1       $X2 = 3n$, $n=3$, $X2 = 9$                                X1 X1

N N N N N N......// ....N<sub>L</sub> N<sub>k</sub> N<sub>f</sub> N<sub>h</sub> N<sub>g</sub> N<sub>f</sub> N<sub>e</sub> N<sub>d</sub> N<sub>c</sub> N<sub>b</sub> N<sub>a</sub> N' N" N<sub>1</sub> N<sub>2</sub> N<sub>3</sub> N<sub>4</sub> N<sub>5</sub> N<sub>6</sub> N<sub>7</sub> N<sub>8</sub> N<sub>9</sub> ...... //....N N N N N N

F2       $X2 = 3n$, $n=3$, $X2 = 9$

N N N N N N.....// ...N<sub>L</sub> N<sub>k</sub> N<sub>f</sub> N<sub>h</sub> N<sub>g</sub> N<sub>f</sub> N<sub>e</sub> N<sub>d</sub> N<sub>c</sub> N<sub>b</sub> N<sub>a</sub> N<sub>1</sub> N<sub>2</sub> N<sub>3</sub> N<sub>4</sub> N<sub>5</sub> N<sub>6</sub> N<sub>7</sub> N<sub>8</sub> N<sub>9</sub> .....//... N N N N N N

NOTE 1:
F1: A REFERENCE DNA FRAGMENT CARRYING A MARKER ABLE TO BE SPIKED INTO A SAMPLE TO BE DETECTED AND A DEFINED GENE MUTATION, COMPRISING (i) A DEFINED GENE MUTATION, WHEREIN, AS COMPARED TO A WILD TYPE OF THE GENE, AT LEAST ONE DEFINED BASE X1 IN THE DEFINED GENE MUTATION UNDERGOES A MUTATION ASSOCIATED WITH THE ONSET, DIAGNOSIS, AND/OR TREATMENT OF A DISEASE (SUCH AS A DRUG TARGET), WHEREIN THE MUTATION IS A SUBSTITUTION MUTATION, A DELETION MUTATION, OR AN INSERTION MUTATION, AND (ii) AT LEAST ONE OTHER ARTIFICIALLY CHANGED BASE X2, TO DISTINGUISH FROM THE REFERENCE DNA FRAGMENT SPIKED INTO THE SAMPLE TO BE DETECTED AND THE MUTANT X1 FRAGMENT THAT IS CONTAINED IN THE SAMPLE TO BE DETECTED AND DETERMINED TO BE ASSOCIATED WITH THE ONSET, DIAGNOSIS AND/OR TREATMENT OF A DISEASE.
F2: DNA FRAGMENT 2 COMPRISING THE BASE X2 AND BUT NOT COMPRISING THE DEFINED BASE X1, WHEREIN THE DNA FRAGMENT 2 AND THE REFERENCE DNA FRAGMENT ARE MIXED AND THEN SPIKED INTO A SAMPLE TO BE DETECTED, OR THE DNA FRAGMENT 2 AND THE REFERENCE DNA FRAGMENT ARE SPIKED INTO A SAMPLE TO BE DETECTED, RESPECTIVELY.
NOTE 2:
N<sub>a</sub> REPRESENTS THE REFERENCE POSITION OF X2 IN THE DNA FRAGMENT SITE, WHICH IS SET AS 0; n IS AN INTEGER.

        REPRESENTS CODON.

## Fig. 23

Fig. 24

NOTE 1:
F1: A REFERENCE DNA FRAGMENT CARRYING A MARKER ABLE TO BE SPIKED INTO A SAMPLE TO BE DETECTED AND A DEFINED GENE MUTATION, COMPRISING (i) A DEFINED GENE MUTATION, WHEREIN, AS COMPARED TO A WILD TYPE OF THE GENE, AT LEAST ONE DEFINED BASE X1 IN THE DEFINED GENE MUTATION UNDERGOES A MUTATION ASSOCIATED WITH THE ONSET, DIAGNOSIS, AND/OR TREATMENT OF A DISEASE (SUCH AS A DRUG TARGET), WHEREIN THE MUTATION IS A SUBSTITUTION MUTATION, A DELETION MUTATION, OR AN INSERTION MUTATION, AND (ii) AT LEAST ONE OTHER ARTIFICIALLY CHANGED BASE X2, TO DISTINGUISH FROM THE REFERENCE DNA FRAGMENT SPIKED INTO THE SAMPLE TO BE DETECTED AND THE MUTANT X1 FRAGMENT THAT IS CONTAINED IN THE SAMPLE TO BE DETECTED AND DETERMINED TO BE ASSOCIATED WITH THE ONSET, DIAGNOSIS AND/OR TREATMENT OF A DISEASE.
F2: DNA FRAGMENT 2 COMPRISING THE BASE X2 AND BUT NOT COMPRISING THE DEFINED BASE X1, WHEREIN THE DNA FRAGMENT 2 AND THE REFERENCE DNA FRAGMENT ARE MIXED AND THEN SPIKED INTO A SAMPLE TO BE DETECTED, OR THE DNA FRAGMENT 2 AND THE REFERENCE DNA FRAGMENT ARE SPIKED INTO A SAMPLE TO BE DETECTED, RESPECTIVELY.
NOTE 2:
WHEN X2 IS LOCATED DOWNSTREAM OF X1, X2 CAN BE LOCATED AT ANY POSITION.

REPRESENTS CODON

Fig. 24

NOTE 1:
F1: A REFERENCE DNA FRAGMENT CARRYING A MARKER ABLE TO BE SPIKED INTO A SAMPLE TO BE DETECTED AND A DEFINED GENE MUTATION, COMPRISING (i) A DEFINED GENE MUTATION, WHEREIN, AS COMPARED TO A WILD TYPE OF THE GENE, AT LEAST ONE DEFINED BASE X1 IN THE DEFINED GENE MUTATION UNDERGOES A MUTATION ASSOCIATED WITH THE ONSET, DIAGNOSIS, AND/OR TREATMENT OF A DISEASE (SUCH AS A DRUG TARGET), WHEREIN THE MUTATION IS A SUBSTITUTION MUTATION, A DELETION MUTATION, OR AN INSERTION MUTATION, AND (ii) AT LEAST ONE OTHER ARTIFICIALLY CHANGED BASE X2, TO DISTINGUISH FROM THE REFERENCE DNA FRAGMENT SPIKED INTO THE SAMPLE TO BE DETECTED AND THE MUTANT X1 FRAGMENT THAT IS CONTAINED IN THE SAMPLE TO BE DETECTED AND DETERMINED TO BE ASSOCIATED WITH THE ONSET, DIAGNOSIS AND/OR TREATMENT OF A DISEASE.
F2: DNA FRAGMENT 2 COMPRISING THE BASE X2 AND BUT NOT COMPRISING THE DEFINED BASE X1, WHEREIN THE DNA FRAGMENT 2 AND THE REFERENCE DNA FRAGMENT ARE MIXED AND THEN SPIKED INTO A SAMPLE TO BE DETECTED, OR THE DNA FRAGMENT 2 AND THE REFERENCE DNA FRAGMENT ARE SPIKED INTO A SAMPLE TO BE DETECTED, RESPECTIVELY.
NOTE 2:
WHEN X2 IS LOCATED UPSTREAM OF X1, $N_a$ REPRESENTS THE REFERENCE POSITION OF X2 IN THE DNA FRAGMENT SITE, WHICH IS SET AS 0; n IS AN INTEGER; WHEN X2 IS LOCATED DOWNSTREAM OF X1, X2 CAN BE LOCATED AT ANY POSITION.

REPRESENTS CODON

Fig. 25

WT

N N N N N N N...... // ......N$_L$ N$_k$ N$_j$ N$_i$ N$_h$ N$_g$ N$_f$ N$_e$ N$_d$ N$_c$ N$_b$ N$_a$ N$_1$ N$_2$ N$_3$ N$_4$ N$_5$ N$_6$ N$_7$ N$_8$ ...... // ...N N N N N N

F1    X2= 3n, n=3, X2= 9                    X1
                                           ↓ ↓ ↓

N N N N N N...... // ......N$_L$ N$_k$ N$_f$ N$_i$ N$_h$ N$_g$ N$_f$ N$_e$ N$_d$ N$_c$ N$_b$ N$_a$ N' N'' N''' N$_1$ N$_2$ N$_3$ N$_4$ N$_5$ N$_6$ N$_7$ N$_8$ ...... // ....N N N N N N

F2    X2= 3n, n=3, X2= 9

N N N N N N N...... // ...N$_L$ N$_k$ N$_f$ N$_i$ N$_h$ N$_g$ N$_f$ N$_e$ N$_d$ N$_c$ N$_b$ N$_a$ N$_1$ N$_2$ N$_3$ N$_4$ N$_5$ N$_6$ N$_7$ N$_8$ ...... // N N N N N N

NOTE 1:
F1: A REFERENCE DNA FRAGMENT CARRYING A MARKER ABLE TO BE SPIKED INTO A SAMPLE TO BE DETECTED AND A DEFINED GENE MUTATION, COMPRISING (i) A DEFINED GENE MUTATION, WHEREIN, AS COMPARED TO A WILD TYPE OF THE GENE, AT LEAST ONE DEFINED BASE X1 IN THE DEFINED GENE MUTATION UNDERGOES A MUTATION ASSOCIATED WITH THE ONSET, DIAGNOSIS, AND/OR TREATMENT OF A DISEASE (SUCH AS A DRUG TARGET), WHEREIN THE MUTATION IS A SUBSTITUTION MUTATION, A DELETION MUTATION, OR AN INSERTION MUTATION, AND (ii) AT LEAST ONE OTHER ARTIFICIALLY CHANGED BASE X2, TO DISTINGUISH FROM THE REFERENCE DNA FRAGMENT SPIKED INTO THE SAMPLE TO BE DETECTED AND THE MUTANT X1 FRAGMENT THAT IS CONTAINED IN THE SAMPLE TO BE DETECTED AND DETERMINED TO BE ASSOCIATED WITH THE ONSET, DIAGNOSIS AND/OR TREATMENT OF A DISEASE.
F2: DNA FRAGMENT 2 COMPRISING THE BASE X2 AND BUT NOT COMPRISING THE DEFINED BASE X1, WHEREIN THE DNA FRAGMENT 2 AND THE REFERENCE DNA FRAGMENT ARE MIXED AND THEN SPIKED INTO A SAMPLE TO BE DETECTED, OR THE DNA FRAGMENT 2 AND THE REFERENCE DNA FRAGMENT ARE SPIKED INTO A SAMPLE TO BE DETECTED, RESPECTIVELY.
NOTE 2:
N$_a$ REPRESENTS THE REFERENCE POSITION OF X2 IN THE DNA FRAGMENT SITE, WHICH IS SET AS 0; n IS AN INTEGER

[____] REPRESENTS CODON

Fig. 26

WT

N N N N N N...... // ......N$_L$ N$_k$ N$_j$ N$_i$ N$_h$ N$_g$ N$_f$ N$_e$ N$_d$ N$_c$ N$_b$ N$_a$ N$_1$ N$_2$ N$_3$ N$_4$ N$_5$ N$_6$ N$_7$ N$_8$ N$_9$ ...... // ......N N N N N N

F1                                         X1              X2= −3n, n=3, X2= −9
                                        ↓ ↓ ↓                    ↓

N N N N N N...... // ......N$_L$ N$_k$ N$_j$ N$_i$ N$_h$ N$_g$ N$_f$ N$_e$ N$_d$ N$_c$ N$_b$ N$_a$ N' N'' N''' N$_1$ N$_2$ N$_3$ N$_4$ N$_5$ N$_6$ N$_7$ N$_8$ N$_9$ ...... // ....N N N N N N N

F2                                                         X2= −3n, n=3, X2= −9
                                                                 ↓

N N N N N N...... // ......N$_L$ N$_k$ N$_j$ N$_i$ N$_h$ N$_g$ N$_f$ N$_e$ N$_d$ N$_c$ N$_b$ N$_a$ N$_1$ N$_2$ N$_3$ N$_4$ N$_5$ N$_6$ N$_7$ N$_8$ N$_9$ ...... // ...N N N N N N

NOTE 1:
F1: A REFERENCE DNA FRAGMENT CARRYING A MARKER ABLE TO BE SPIKED INTO A SAMPLE TO BE DETECTED AND A DEFINED GENE MUTATION, COMPRISING (i) A DEFINED GENE MUTATION, WHEREIN, AS COMPARED TO A WILD TYPE OF THE GENE, AT LEAST ONE DEFINED BASE X1 IN THE DEFINED GENE MUTATION UNDERGOES A MUTATION ASSOCIATED WITH THE ONSET, DIAGNOSIS, AND/OR TREATMENT OF A DISEASE (SUCH AS A DRUG TARGET), WHEREIN THE MUTATION IS A SUBSTITUTION MUTATION, A DELETION MUTATION, OR AN INSERTION MUTATION, AND (ii) AT LEAST ONE OTHER ARTIFICIALLY CHANGED BASE X2, TO DISTINGUISH FROM THE REFERENCE DNA FRAGMENT SPIKED INTO THE SAMPLE TO BE DETECTED AND THE MUTANT X1 FRAGMENT THAT IS CONTAINED IN THE SAMPLE TO BE DETECTED AND DETERMINED TO BE ASSOCIATED WITH THE ONSET, DIAGNOSIS AND/OR TREATMENT OF A DISEASE.
F2: DNA FRAGMENT 2 COMPRISING THE BASE X2 AND BUT NOT COMPRISING THE DEFINED BASE X1, WHEREIN THE DNA FRAGMENT 2 AND THE REFERENCE DNA FRAGMENT ARE MIXED AND THEN SPIKED INTO A SAMPLE TO BE DETECTED, OR THE DNA FRAGMENT 2 AND THE REFERENCE DNA FRAGMENT ARE SPIKED INTO A SAMPLE TO BE DETECTED, RESPECTIVELY.
NOTE 2:
N$_a$ REPRESENTS THE REFERENCE POSITION OF X2 IN THE DNA FRAGMENT SITE, WHICH IS SET AS 0; n IS AN INTEGER

[____] REPRESENTS CODON

Fig. 27

NOTE 1:
F1: A REFERENCE DNA FRAGMENT CARRYING A MARKER ABLE TO BE SPIKED INTO A SAMPLE TO BE DETECTED AND A DEFINED GENE MUTATION, COMPRISING (i) A DEFINED GENE MUTATION, WHEREIN, AS COMPARED TO A WILD TYPE OF THE GENE, AT LEAST ONE DEFINED BASE X1 IN THE DEFINED GENE MUTATION UNDERGOES A MUTATION ASSOCIATED WITH THE ONSET, DIAGNOSIS, AND/OR TREATMENT OF A DISEASE (SUCH AS A DRUG TARGET), WHEREIN THE MUTATION IS A SUBSTITUTION MUTATION, A DELETION MUTATION, OR AN INSERTION MUTATION, AND (ii) AT LEAST ONE OTHER ARTIFICIALLY CHANGED BASE X2, TO DISTINGUISH FROM THE REFERENCE DNA FRAGMENT SPIKED INTO THE SAMPLE TO BE DETECTED AND THE MUTANT X1 FRAGMENT THAT IS CONTAINED IN THE SAMPLE TO BE DETECTED AND DETERMINED TO BE ASSOCIATED WITH THE ONSET, DIAGNOSIS AND/OR TREATMENT OF A DISEASE.
F2: DNA FRAGMENT 2 COMPRISING THE BASE X2 AND BUT NOT COMPRISING THE DEFINED BASE X1, WHEREIN THE DNA FRAGMENT 2 AND THE REFERENCE DNA FRAGMENT ARE MIXED AND THEN SPIKED INTO A SAMPLE TO BE DETECTED, OR THE DNA FRAGMENT 2 AND THE REFERENCE DNA FRAGMENT ARE SPIKED INTO A SAMPLE TO BE DETECTED, RESPECTIVELY.
NOTE 2:
$N_0$ REPRESENTS THE REFERENCE POSITION OF X2 IN THE DNA FRAGMENT SITE, WHICH IS SET AS 0; n IS AN INTEGER
REPRESENTS CODON

Fig. 28

NOTE 1:
F1: A REFERENCE DNA FRAGMENT CARRYING A MARKER ABLE TO BE SPIKED INTO A SAMPLE TO BE DETECTED AND A DEFINED GENE MUTATION, COMPRISING (i) A DEFINED GENE MUTATION, WHEREIN, AS COMPARED TO A WILD TYPE OF THE GENE, AT LEAST ONE DEFINED BASE X1 IN THE DEFINED GENE MUTATION UNDERGOES A MUTATION ASSOCIATED WITH THE ONSET, DIAGNOSIS, AND/OR TREATMENT OF A DISEASE (SUCH AS A DRUG TARGET), WHEREIN THE MUTATION IS A SUBSTITUTION MUTATION, A DELETION MUTATION, OR AN INSERTION MUTATION, AND (ii) AT LEAST ONE OTHER ARTIFICIALLY CHANGED BASE X2, TO DISTINGUISH FROM THE REFERENCE DNA FRAGMENT SPIKED INTO THE SAMPLE TO BE DETECTED AND THE MUTANT X1 FRAGMENT THAT IS CONTAINED IN THE SAMPLE TO BE DETECTED AND DETERMINED TO BE ASSOCIATED WITH THE ONSET, DIAGNOSIS AND/OR TREATMENT OF A DISEASE.
F2: DNA FRAGMENT 2 COMPRISING THE BASE X2 AND BUT NOT COMPRISING THE DEFINED BASE X1, WHEREIN THE DNA FRAGMENT 2 AND THE REFERENCE DNA FRAGMENT ARE MIXED AND THEN SPIKED INTO A SAMPLE TO BE DETECTED, OR THE DNA FRAGMENT 2 AND THE REFERENCE DNA FRAGMENT ARE SPIKED INTO A SAMPLE TO BE DETECTED, RESPECTIVELY.
NOTE 2:
$N_0$ REPRESENTS THE REFERENCE POSITION OF X2 IN THE DNA FRAGMENT SITE, WHICH IS SET AS 0; n IS AN INTEGER
REPRESENTS CODON

Fig. 29

WT

F1

X1    X2

F2

X2

NOTE 1:
F1: A REFERENCE DNA FRAGMENT CARRYING A MARKER ABLE TO BE SPIKED INTO A SAMPLE TO BE DETECTED AND A DEFINED GENE MUTATION, COMPRISING (i) A DEFINED GENE MUTATION, WHEREIN, AS COMPARED TO A WILD TYPE OF THE GENE, AT LEAST ONE DEFINED BASE X1 IN THE DEFINED GENE MUTATION UNDERGOES A MUTATION ASSOCIATED WITH THE ONSET, DIAGNOSIS, AND/OR TREATMENT OF A DISEASE (SUCH AS A DRUG TARGET), WHEREIN THE MUTATION IS A SUBSTITUTION MUTATION, A DELETION MUTATION, OR AN INSERTION MUTATION, AND (ii) AT LEAST ONE OTHER ARTIFICIALLY CHANGED BASE X2, TO DISTINGUISH FROM THE REFERENCE DNA FRAGMENT SPIKED INTO THE SAMPLE TO BE DETECTED AND THE MUTANT X1 FRAGMENT THAT IS CONTAINED IN THE SAMPLE TO BE DETECTED AND DETERMINED TO BE ASSOCIATED WITH THE ONSET, DIAGNOSIS AND/OR TREATMENT OF A DISEASE.
F2: DNA FRAGMENT 2 COMPRISING THE BASE X2 AND BUT NOT COMPRISING THE DEFINED BASE X1, WHEREIN THE DNA FRAGMENT 2 AND THE REFERENCE DNA FRAGMENT ARE MIXED AND THEN SPIKED INTO A SAMPLE TO BE DETECTED, OR THE DNA FRAGMENT 2 AND THE REFERENCE DNA FRAGMENT ARE SPIKED INTO A SAMPLE TO BE DETECTED, RESPECTIVELY.
NOTE 2:
WHEN X2 IS LOCATED DOWNSTREAM OF X1, X2 CAN BE LOCATED AT ANY POSITION.

REPRESENTS CODON

Fig. 30

WT

F1

X2= 3n, n=3, X2= 9    X1    X2

F2

X2= 3n, n=3, X2= 9    X2

NOTE 1:
F1: A REFERENCE DNA FRAGMENT CARRYING A MARKER ABLE TO BE SPIKED INTO A SAMPLE TO BE DETECTED AND A DEFINED GENE MUTATION, COMPRISING (i) A DEFINED GENE MUTATION, WHEREIN, AS COMPARED TO A WILD TYPE OF THE GENE, AT LEAST ONE DEFINED BASE X1 IN THE DEFINED GENE MUTATION UNDERGOES A MUTATION ASSOCIATED WITH THE ONSET, DIAGNOSIS, AND/OR TREATMENT OF A DISEASE (SUCH AS A DRUG TARGET), WHEREIN THE MUTATION IS A SUBSTITUTION MUTATION, A DELETION MUTATION, OR AN INSERTION MUTATION, AND (ii) AT LEAST ONE OTHER ARTIFICIALLY CHANGED BASE X2, TO DISTINGUISH FROM THE REFERENCE DNA FRAGMENT SPIKED INTO THE SAMPLE TO BE DETECTED AND THE MUTANT X1 FRAGMENT THAT IS CONTAINED IN THE SAMPLE TO BE DETECTED AND DETERMINED TO BE ASSOCIATED WITH THE ONSET, DIAGNOSIS AND/OR TREATMENT OF A DISEASE.
F2: DNA FRAGMENT 2 COMPRISING THE BASE X2 AND BUT NOT COMPRISING THE DEFINED BASE X1, WHEREIN THE DNA FRAGMENT 2 AND THE REFERENCE DNA FRAGMENT ARE MIXED AND THEN SPIKED INTO A SAMPLE TO BE DETECTED, OR THE DNA FRAGMENT 2 AND THE REFERENCE DNA FRAGMENT ARE SPIKED INTO A SAMPLE TO BE DETECTED, RESPECTIVELY.
NOTE 2:
WHEN X2 IS LOCATED UPSTREAM OF X1, $N_a$ REPRESENTS THE REFERENCE POSITION OF X2 IN THE DNA FRAGMENT SITE, WHICH IS SET AS 0; n IS AN INTEGER; WHEN X2 IS LOCATED DOWNSTREAM OF X1, X2 CAN BE LOCATED AT ANY POSITION.
REPRESENTS CODON.

Fig. 31

WT

N N N N N N...... // ...$N_L$ $N_k$ $N_j$ $N_i$ $N_h$ $N_g$ $N_f$ $N_e$ $N_d$ $N_c$ $N_b$ $N_a$ $N_1$ $N_2$ $N_3$ $N_4$ $N_5$ $N_6$ $N_7$ $N_8$ $N_9$ $N_{10}$ $N_{11}$ ...... // ...N N N N N N

F1          X2= 3n, n=3, X2= 9                    X1 X1
                       ↓                          ↓ ↓
N N N N N N.... // ...$N_L$ $N_k$ $N_j$ $N_i$ $N_h$ $N_g$ $N_f$ $N_e$ $N_d$ $N_c$ $N_b$ $N_a$ — — $N_3$ $N_4$ $N_5$ $N_6$ $N_7$ $N_8$ $N_9$ $N_{10}$ $N_{11}$...... // ...N N N N N N

F2          X2= 3n, n=3, X2= 9
                       ↓
N N N N N N...... // ...$N_L$ $N_k$ $N_f$ $N_i$ $N_h$ $N_g$ $N_f$ $N_e$ $N_d$ $N_c$ $N_b$ $N_a$ $N_1$ $N_2$ $N_3$ $N_4$ $N_5$ $N_6$ $N_7$ $N_8$ $N_9$ $N_{10}$ $N_{11}$ ...... // ...N N N N N N

NOTE 1:
F1: A REFERENCE DNA FRAGMENT CARRYING A MARKER ABLE TO BE SPIKED INTO A SAMPLE TO BE DETECTED AND
A DEFINED GENE MUTATION, COMPRISING (i) A DEFINED GENE MUTATION, WHEREIN, AS COMPARED TO A WILD TYPE
OF THE GENE, AT LEAST ONE DEFINED BASE X1 IN THE DEFINED GENE MUTATION UNDERGOES A MUTATION ASSOCIATED
WITH THE ONSET, DIAGNOSIS, AND/OR TREATMENT OF A DISEASE (SUCH AS A DRUG TARGET), WHEREIN THE MUTATION
IS A SUBSTITUTION MUTATION, A DELETION MUTATION, OR AN INSERTION MUTATION, AND (ii) AT LEAST ONE OTHER
ARTIFICIALLY CHANGED BASE X2, TO DISTINGUISH FROM THE REFERENCE DNA FRAGMENT SPIKED INTO THE SAMPLE TO
BE DETECTED AND THE MUTANT X1 FRAGMENT THAT IS CONTAINED IN THE SAMPLE TO BE DETECTED AND DETERMINED TO
BE ASSOCIATED WITH THE ONSET, DIAGNOSIS AND/OR TREATMENT OF A DISEASE.
F2: DNA FRAGMENT 2 COMPRISING THE BASE X2 AND BUT NOT COMPRISING THE DEFINED BASE X1, WHEREIN THE DNA
FRAGMENT 2 AND THE REFERENCE DNA FRAGMENT ARE MIXED AND THEN SPIKED INTO A SAMPLE TO BE DETECTED, OR THE
DNA FRAGMENT 2 AND THE REFERENCE DNA FRAGMENT ARE SPIKED INTO A SAMPLE TO BE DETECTED, RESPECTIVELY.
NOTE 2:
$N_a$ REPRESENTS THE REFERENCE POSITION OF X2 IN THE DNA FRAGMENT SITE, WHICH IS SET AS 0; n IS AN INTEGER.
[____] REPRESENTS CODON.

## Fig. 32

WT

N N N N N N...// ...$N_L$ $N_k$ $N_j$ $N_i$ $N_h$ $N_g$ $N_f$ $N_e$ $N_d$ $N_c$ $N_b$ $N_a$ $N_1$ $N_2$ $N_3$ $N_4$ $N_5$ $N_6$ $N_7$ $N_8$ $N_9$ $N_{10}$ $N_{11}$ $N_{12}$ ... //...N N N N N N

F1                                               X1 X1                    X2
                                                 ↓ ↓                      ↓
N N N N N N......// ...$N_L$ $N_k$ $N_j$ $N_i$ $N_h$ $N_g$ $N_f$ $N_e$ $N_d$ $N_c$ $N_b$ $N_a$ — — $N_3$ $N_4$ $N_5$ $N_6$ $N_7$ $N_8$ $N_9$ $N_{10}$ $N_{11'}$ $N_{12}$... //...N N N N N N

F2                                                                        X2
                                                                          ↓
N N N N N N......// ... $N_L$ $N_k$ $N_j$ $N_i$ $N_h$ $N_g$ $N_f$ $N_e$ $N_d$ $N_c$ $N_b$ $N_a$ $N_1$ $N_2$ $N_3$ $N_4$ $N_5$ $N_6$ $N_7$ $N_8$ $N_9$ $N_{10}$ $N_{11'}$ $N_{12}$... //...N N N N N N

NOTE 1:
F1: A REFERENCE DNA FRAGMENT CARRYING A MARKER ABLE TO BE SPIKED INTO A SAMPLE TO BE DETECTED AND
A DEFINED GENE MUTATION, COMPRISING (i) A DEFINED GENE MUTATION, WHEREIN, AS COMPARED TO A WILD TYPE
OF THE GENE, AT LEAST ONE DEFINED BASE X1 IN THE DEFINED GENE MUTATION UNDERGOES A MUTATION ASSOCIATED
WITH THE ONSET, DIAGNOSIS, AND/OR TREATMENT OF A DISEASE (SUCH AS A DRUG TARGET), WHEREIN THE MUTATION
IS A SUBSTITUTION MUTATION, A DELETION MUTATION, OR AN INSERTION MUTATION, AND (ii) AT LEAST ONE OTHER
ARTIFICIALLY CHANGED BASE X2, TO DISTINGUISH FROM THE REFERENCE DNA FRAGMENT SPIKED INTO THE SAMPLE TO
BE DETECTED AND THE MUTANT X1 FRAGMENT THAT IS CONTAINED IN THE SAMPLE TO BE DETECTED AND DETERMINED TO
BE ASSOCIATED WITH THE ONSET, DIAGNOSIS AND/OR TREATMENT OF A DISEASE.
F2: DNA FRAGMENT 2 COMPRISING THE BASE X2 AND BUT NOT COMPRISING THE DEFINED BASE X1, WHEREIN THE DNA
FRAGMENT 2 AND THE REFERENCE DNA FRAGMENT ARE MIXED AND THEN SPIKED INTO A SAMPLE TO BE DETECTED, OR THE
DNA FRAGMENT 2 AND THE REFERENCE DNA FRAGMENT ARE SPIKED INTO A SAMPLE TO BE DETECTED, RESPECTIVELY.
NOTE 2:
WHEN X2 IS LOCATED DOWNSTREAM OF X1, X2 CAN BE LOCATED AT ANY POSITION.
[____] REPRESENTS CODON.

## Fig. 33

WT

NOTE 1:
F1: A REFERENCE DNA FRAGMENT CARRYING A MARKER ABLE TO BE SPIKED INTO A SAMPLE TO BE DETECTED AND A DEFINED GENE MUTATION, COMPRISING (i) A DEFINED GENE MUTATION, WHEREIN, AS COMPARED TO A WILD TYPE OF THE GENE, AT LEAST ONE DEFINED BASE X1 IN THE DEFINED GENE MUTATION UNDERGOES A MUTATION ASSOCIATED WITH THE ONSET, DIAGNOSIS, AND/OR TREATMENT OF A DISEASE (SUCH AS A DRUG TARGET), WHEREIN THE MUTATION IS A SUBSTITUTION MUTATION, A DELETION MUTATION, OR AN INSERTION MUTATION, AND (ii) AT LEAST ONE OTHER ARTIFICIALLY CHANGED BASE X2, TO DISTINGUISH FROM THE REFERENCE DNA FRAGMENT SPIKED INTO THE SAMPLE TO BE DETECTED AND THE MUTANT X1 FRAGMENT THAT IS CONTAINED IN THE SAMPLE TO BE DETECTED AND DETERMINED TO BE ASSOCIATED WITH THE ONSET, DIAGNOSIS AND/OR TREATMENT OF A DISEASE.
F2: DNA FRAGMENT 2 COMPRISING THE BASE X2 AND BUT NOT COMPRISING THE DEFINED BASE X1, WHEREIN THE DNA FRAGMENT 2 AND THE REFERENCE DNA FRAGMENT ARE MIXED AND THEN SPIKED INTO A SAMPLE TO BE DETECTED, OR THE DNA FRAGMENT 2 AND THE REFERENCE DNA FRAGMENT ARE SPIKED INTO A SAMPLE TO BE DETECTED, RESPECTIVELY.
NOTE 2:
WHEN X2 IS LOCATED UPSTREAM OF X1, $N_a$ REPRESENTS THE REFERENCE POSITION OF X2 IN THE DNA FRAGMENT SITE, WHICH IS SET AS 0; n IS AN INTEGER; WHEN X2 IS LOCATED DOWNSTREAM OF X1, X2 CAN BE LOCATED AT ANY POSITION.
[____] REPRESENTS CODON.

Fig. 34

WT

NOTE 1:
F1: A REFERENCE DNA FRAGMENT CARRYING A MARKER ABLE TO BE SPIKED INTO A SAMPLE TO BE DETECTED AND A DEFINED GENE MUTATION, COMPRISING (i) A DEFINED GENE MUTATION, WHEREIN, AS COMPARED TO A WILD TYPE OF THE GENE, AT LEAST ONE DEFINED BASE X1 IN THE DEFINED GENE MUTATION UNDERGOES A MUTATION ASSOCIATED WITH THE ONSET, DIAGNOSIS, AND/OR TREATMENT OF A DISEASE (SUCH AS A DRUG TARGET), WHEREIN THE MUTATION IS A SUBSTITUTION MUTATION, A DELETION MUTATION, OR AN INSERTION MUTATION, AND (ii) AT LEAST ONE OTHER ARTIFICIALLY CHANGED BASE X2, TO DISTINGUISH FROM THE REFERENCE DNA FRAGMENT SPIKED INTO THE SAMPLE TO BE DETECTED AND THE MUTANT X1 FRAGMENT THAT IS CONTAINED IN THE SAMPLE TO BE DETECTED AND DETERMINED TO BE ASSOCIATED WITH THE ONSET, DIAGNOSIS AND/OR TREATMENT OF A DISEASE.
F2: DNA FRAGMENT 2 COMPRISING THE BASE X2 AND BUT NOT COMPRISING THE DEFINED BASE X1, WHEREIN THE DNA FRAGMENT 2 AND THE REFERENCE DNA FRAGMENT ARE MIXED AND THEN SPIKED INTO A SAMPLE TO BE DETECTED, OR THE DNA FRAGMENT 2 AND THE REFERENCE DNA FRAGMENT ARE SPIKED INTO A SAMPLE TO BE DETECTED, RESPECTIVELY.
NOTE 2:
$N_a$ REPRESENTS THE REFERENCE POSITION OF X2 IN THE DNA FRAGMENT SITE, WHICH IS SET AS 0; n IS AN INTEGER
[____] REPRESENTS CODON.

Fig. 35

WT

F1

X1 X1 X1

X2= -3n, n=3, X2= -9

F2

X2= -3n, n=3, X2= -9

NOTE 1:
F1: A REFERENCE DNA FRAGMENT CARRYING A MARKER ABLE TO BE SPIKED INTO A SAMPLE TO BE DETECTED AND
A DEFINED GENE MUTATION, COMPRISING (i) A DEFINED GENE MUTATION, WHEREIN, AS COMPARED TO A WILD TYPE
OF THE GENE, AT LEAST ONE DEFINED BASE X1 IN THE DEFINED GENE MUTATION UNDERGOES A MUTATION ASSOCIATED
WITH THE ONSET, DIAGNOSIS, AND/OR TREATMENT OF A DISEASE (SUCH AS A DRUG TARGET), WHEREIN THE MUTATION
IS A SUBSTITUTION MUTATION, A DELETION MUTATION, OR AN INSERTION MUTATION, AND (ii) AT LEAST ONE OTHER
ARTIFICIALLY CHANGED BASE X2, TO DISTINGUISH FROM THE REFERENCE DNA FRAGMENT SPIKED INTO THE SAMPLE TO
BE DETECTED AND THE MUTANT X1 FRAGMENT THAT IS CONTAINED IN THE SAMPLE TO BE DETECTED AND DETERMINED TO
BE ASSOCIATED WITH THE ONSET, DIAGNOSIS AND/OR TREATMENT OF A DISEASE.
F2: DNA FRAGMENT 2 COMPRISING THE BASE X2 AND BUT NOT COMPRISING THE DEFINED BASE X1, WHEREIN THE DNA
FRAGMENT 2 AND THE REFERENCE DNA FRAGMENT ARE MIXED AND THEN SPIKED INTO A SAMPLE TO BE DETECTED, OR THE
DNA FRAGMENT 2 AND THE REFERENCE DNA FRAGMENT ARE SPIKED INTO A SAMPLE TO BE DETECTED, RESPECTIVELY.
NOTE 2:
$N_o$ REPRESENTS THE REFERENCE POSITION OF X2 IN THE DNA FRAGMENT SITE, WHICH IS SET AS 0; n IS AN INTEGER
▭ REPRESENTS CODON.

Fig. 36

WT

F1

X2= 3n, n=3, X2= 9

X1 X1 X1

X2= -3n, n=3, X2= -9

F2

X2= 3n, n=3, X2= 9

X2= -3n, n=3, X2= -9

NOTE 1:
F1: A REFERENCE DNA FRAGMENT CARRYING A MARKER ABLE TO BE SPIKED INTO A SAMPLE TO BE DETECTED AND
A DEFINED GENE MUTATION, COMPRISING (i) A DEFINED GENE MUTATION, WHEREIN, AS COMPARED TO A WILD TYPE
OF THE GENE, AT LEAST ONE DEFINED BASE X1 IN THE DEFINED GENE MUTATION UNDERGOES A MUTATION ASSOCIATED
WITH THE ONSET, DIAGNOSIS, AND/OR TREATMENT OF A DISEASE (SUCH AS A DRUG TARGET), WHEREIN THE MUTATION
IS A SUBSTITUTION MUTATION, A DELETION MUTATION, OR AN INSERTION MUTATION, AND (ii) AT LEAST ONE OTHER
ARTIFICIALLY CHANGED BASE X2, TO DISTINGUISH FROM THE REFERENCE DNA FRAGMENT SPIKED INTO THE SAMPLE TO
BE DETECTED AND THE MUTANT X1 FRAGMENT THAT IS CONTAINED IN THE SAMPLE TO BE DETECTED AND DETERMINED TO
BE ASSOCIATED WITH THE ONSET, DIAGNOSIS AND/OR TREATMENT OF A DISEASE.
F2: DNA FRAGMENT 2 COMPRISING THE BASE X2 AND BUT NOT COMPRISING THE DEFINED BASE X1, WHEREIN THE DNA
FRAGMENT 2 AND THE REFERENCE DNA FRAGMENT ARE MIXED AND THEN SPIKED INTO A SAMPLE TO BE DETECTED, OR THE
DNA FRAGMENT 2 AND THE REFERENCE DNA FRAGMENT ARE SPIKED INTO A SAMPLE TO BE DETECTED, RESPECTIVELY.
NOTE 2:
$N_o$ REPRESENTS THE REFERENCE POSITION OF X2 IN THE DNA FRAGMENT SITE, WHICH IS SET AS 0; n IS AN INTEGER
▭ REPRESENTS CODON.

Fig. 37

DNA FRAGMENTS WITH CHANGED BASES AT PARTICUALR POSITIONS AND USE

Fig. 38

EP 3 910 066 A1

**LONG-FRAGMENT MUTATION SITE AND SPIKED-IN MARKER SITE**

PANEL a: THE BASE X2S ARE LOCATED UPSTREAM AND DOWNSTREAM OF THE DEFINED BASE X1,
AND THE X2S ARE LOCATED IN THE EXON SEQUENCE AND THE INTRON SEQUENCE

NOTE 1:
F1: A REFERENCE DNA FRAGMENT CARRYING A MARKER ABLE TO BE SPIKED INTO A SAMPLE TO BE DETECTED AND A DEFINED
GENE MUTATION, COMPRISING (i) A DEFINED GENE MUTATION, WHEREIN, AS COMPARED TO A WILD TYPE OF THE GENE, AT
LEAST ONE DEFINED BASE X1 IN THE DEFINED GENE MUTATION UNDERGOES A MUTATION ASSOCIATED WITH THE ONSET,
DIAGNOSIS, AND/OR TREATMENT OF A DISEASE (SUCH AS A DRUG TARGET), WHEREIN THE MUTATION IS A SUBSTITUTION
MUTATION, A DELETION MUTATION, OR AN INSERTION MUTATION, AND (ii) AT LEAST ONE OTHER ARTIFICIALLY CHANGED
BASE X2 (LOCATED IN THE EXON SEQUENCE AND THE INTRON SEQUENCE), TO DISTINGUISH FROM THE REFERENCE DNA
FRAGMENT SPIKED INTO THE SAMPLE TO BE DETECTED AND THE MUTANT X1 FRAGMENT THAT IS CONTAINED IN THE SAMPLE
TO BE DETECTED AND DETERMINED TO BE ASSOCIATED WITH THE ONSET, DIAGNOSIS AND/OR TREATMENT OF A DISEASE.
F2: DNA FRAGMENT 2 COMPRISING THE BASE X2 (LOCATED IN THE EXON SEQUENCE AND THE INTRON SEQUENCE) AND BUT
NOT COMPRISING THE DEFINED BASE X1, WHEREIN THE DNA FRAGMENT 2 AND THE REFERENCE DNA FRAGMENT ARE MIXED
AND THEN SPIKED INTO A SAMPLE TO BE DETECTED, OR THE DNA FRAGMENT 2 AND THE REFERENCE DNA FRAGMENT ARE
SPIKED INTO A SAMPLE TO BE DETECTED, RESPECTIVELY.

NOTE 2:
$N_a$ REPRESENTS THE REFERENCE POSITION OF X2 IN THE DNA FRAGMENT SITE, WHICH IS SET AS 0; n IS A POSITIVE INTEGER.
$N_a$' IS LOCATED IN THE INTRON SEQUENCE UPSTREAM OF THE EXON SEQUENCE. $N_a$'=+1, $N_b$'=+2, $N_b$'=+3, ······ ; $N_1$' IS LOCATED
IN THE INTRON SEQUENCE DOWNSTREAM OF THE EXON SEQUENCE. $N_1$'=-1, $N_b$'=-2, $N_b$'=-3, ······ ; n IS A POSITIVE INTEGER.
REPRESENTS CODON.

Fig. 39

**LONG-FRAGMENT MUTATION SITE AND SPIKED-IN MARKER SITE**

PANEL b: THE BASE X2S ARE LOCATED UPSTREAM AND DOWNSTREAM OF THE DEFINED BASE X1, AND THE X2S ARE LOCATED IN THE INTRON SEQUENCE

NOTE 1:

F1: A REFERENCE DNA FRAGMENT CARRYING A MARKER ABLE TO BE SPIKED INTO A SAMPLE TO BE DETECTED AND A DEFINED GENE MUTATION, COMPRISING (i) A DEFINED GENE MUTATION, WHEREIN, AS COMPARED TO A WILD TYPE OF THE GENE, AT LEAST ONE DEFINED BASE X1 IN THE DEFINED GENE MUTATION UNDERGOES A MUTATION ASSOCIATED WITH THE ONSET, DIAGNOSIS, AND/OR TREATMENT OF A DISEASE (SUCH AS A DRUG TARGET), WHEREIN THE MUTATION IS A SUBSTITUTION MUTATION, A DELETION MUTATION, OR AN INSERTION MUTATION, AND (ii) AT LEAST ONE OTHER ARTIFICIALLY CHANGED BASE X2 (LOCATED IN THE INTRON SEQUENCE), TO DISTINGUISH FROM THE REFERENCE DNA FRAGMENT SPIKED INTO THE SAMPLE TO BE DETECTED AND THE MUTANT X1 FRAGMENT THAT IS CONTAINED IN THE SAMPLE TO BE DETECTED AND DETERMINED TO BE ASSOCIATED WITH THE ONSET, DIAGNOSIS AND/OR TREATMENT OF A DISEASE. F2: DNA FRAGMENT 2 COMPRISING THE BASE X2 (LOCATED IN THE INTRON SEQUENCE) AND BUT NOT COMPRISING THE DEFINED BASE X1, WHEREIN THE DNA FRAGMENT 2 AND THE REFERENCE DNA FRAGMENT ARE MIXED AND THEN SPIKED INTO A SAMPLE TO BE DETECTED, OR THE DNA FRAGMENT 2 AND THE REFERENCE DNA FRAGMENT ARE SPIKED INTO A SAMPLE TO BE DETECTED, RESPECTIVELY.

NOTE 2:

$N_a{}'$ IS LOCATED IN THE INTRON SEQUENCE UPSTREAM OF THE EXON SEQUENCE, $N_a{}'=+1$, $N_b{}'=+2$, $N_b{}'=+3$, ······· ; $N_i{}'$ IS LOCATED IN THE INTRON SEQUENCE DOWNSTREAM OF THE EXON SEQUENCE, $N_i{}'=-1$, $N_b{}'=-2$, $N_b{}'=-3$, ······· ; $n$ IS A POSITIVE INTEGER.

⌐_____⌐ REPRESENTS CODON.

Fig. 40

## INTERNATIONAL SEARCH REPORT

| International application No. |
|---|
| **PCT/CN2019/119684** |

| **A.** | **CLASSIFICATION OF SUBJECT MATTER** |
|---|---|

C12Q 1/68(2018.01)i; C12N 15/00(2006.01)i

According to International Patent Classification (IPC) or to both national classification and IPC

| **B.** | **FIELDS SEARCHED** |
|---|---|

Minimum documentation searched (classification system followed by classification symbols)

    C12Q; C12N

Documentation searched other than minimum documentation to the extent that such documents are included in the fields searched

Electronic data base consulted during the international search (name of data base and, where practicable, search terms used)

    CNABS, DWPI, WOTXT, USTXT, EPTXT, CNKI, 万方, WANFANG, pubmed, NGS, next-generation sequencing, quality control, donor, sgRNA, CRISPR, 二代测序, 质量控制, 质控, 参照

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| Y | JIA, Shiyu et al. "A Novel Cell Line Generated Using the CRISPR/Cas9 Techonology as Universal Quality Control Material for KRAS G12V Mutation Testing" *Journal of Clinical Laboratory Analysis*, Vol. 32, No. (5), 30 January 2018 (2018-01-30), Abstract, Methods and Materials, and Discussion | 1-45 |
| Y | CN 106381334 A (SHANGHAI 3DMED BIOMEDICAL TECHNOLOGY CO., LTD.) 08 February 2017 (2017-02-08) the claims | 1-45 |
| Y | CN 106636404 A (3D MEDICINES CORPORATION) 10 May 2017 (2017-05-10) the claims | 1-45 |
| Y | CN 106498079 A (SHANGHAI 3DMED BIOMEDICAL TECHNOLOGY CO., LTD.) 15 March 2017 (2017-03-15) the claims | 1-45 |
| A | CN 105861653 A (BEIJING HOSPITAL) 17 August 2016 (2016-08-17) entire document | 1-45 |
| A | WO 2015118513 A1 (VELA OPERATIONS PTE. LTD.) 13 August 2015 (2015-08-13) entire document | 1-45 |

☑ Further documents are listed in the continuation of Box C.      ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **22 January 2020** | **20 February 2020** |

| Name and mailing address of the ISA/CN | Authorized officer |
|---|---|
| **China National Intellectual Property Administration** **No. 6, Xitucheng Road, Jimenqiao Haidian District, Beijing 100088 China** | |
| Facsimile No. **(86-10)62019451** | Telephone No. |

Form PCT/ISA/210 (second sheet) (January 2015)

# EP 3 910 066 A1

**INTERNATIONAL SEARCH REPORT**

International application No.

**PCT/CN2019/119684**

**C. DOCUMENTS CONSIDERED TO BE RELEVANT**

| Category* | Citation of document, with indication, where appropriate, of the relevant passages | Relevant to claim No. |
|---|---|---|
| A | Tumor Marker Committee of Chinese Society of Clinical Oncology et al. "二代测序技术在肿瘤精准医学诊断中的应用专家共识 (Non-official translation: Expert Consensus on Application of Next-Generation Sequencing Technology in Precise Medical Diagnosis of Tumor)"<br>中华医学杂志 (National Medical Journal of China),<br>Vol. 98, No. (26), 10 July 2018 (2018-07-10),<br>pages 2057-2065 | 1-45 |

Form PCT/ISA/210 (second sheet) (January 2015)

73

## INTERNATIONAL SEARCH REPORT

| International application No. |
| --- |
| **PCT/CN2019/119684** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☐ forming part of the international application as filed:

        ☐ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☑ furnished subsequent to the international filing date for the purposes of international search only:

        ☑ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
Information on patent family members

International application No.

**PCT/CN2019/119684**

| Patent document cited in search report | | | Publication date (day/month/year) | Patent family member(s) | | | Publication date (day/month/year) |
|---|---|---|---|---|---|---|---|
| CN | 106381334 | A | 08 February 2017 | None | | | |
| CN | 106636404 | A | 10 May 2017 | None | | | |
| CN | 106498079 | A | 15 March 2017 | None | | | |
| CN | 105861653 | A | 17 August 2016 | CN | 105861653 | B | 01 November 2019 |
| WO | 2015118513 | A1 | 13 August 2015 | AU | 2015213570 | A1 | 16 June 2016 |
| | | | | TR | 201903795 | T4 | 21 June 2019 |
| | | | | ES | 2719412 | T3 | 10 July 2019 |
| | | | | US | 2018037949 | A1 | 08 February 2018 |
| | | | | DK | 3105324 | T3 | 08 April 2019 |
| | | | | EP | 3105324 | B1 | 19 December 2018 |
| | | | | CN | 106257989 | A | 28 December 2016 |
| | | | | JP | 2017505120 | A | 16 February 2017 |
| | | | | PT | 3105324 | T | 01 April 2019 |
| | | | | GB | 201402249 | D0 | 26 March 2014 |
| | | | | EP | 3105324 | A1 | 21 December 2016 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **IRA W. DEVESON et al.** *Nature Methods,* 2016, vol. 13 (9), 784-791